# EUROPEAN PATENT APPLICATION

(11) **EP 4 071 146 A1**
(43) Date of publication of application: **12.10.2022**
(21) Application number: 20895795.1
(22) Date of filing: 04.12.2020
(51) Int. Cl.: C07D 405/14, C07D 409/14, A61K 31/343, A61K 31/352, A61P 35/00

(54) **BIARYL COMPOUND AS PAN-RAF KINASE INHIBITOR**

(30) Priority: 06.12.2019 CN 201911242225; 30.04.2020 CN 202010367751; 08.07.2020 CN 202010652149
(71) Applicant: Medshine Discovery Inc., Nanjing, Jiangsu 210032 (CN)
(72) Inventor: DING, Charles Z., Shanghai 200131 (CN); LIU, Xile, Shanghai 200131 (CN); CHEN, Shuhui, Shanghai 200131 (CN); HU, Lihong, Shanghai 200131 (CN); ZHOU, Chengliang, Shanghai 200131 (CN)
(74) Representative: Dehns
(86) International application number: PCT/CN2020/133933
(87) International publication number: WO 2021/110141

(57) **Abstract**

Disclosed is a biarylamide compound as a Pan-RAF kinase inhibitor, and specifically disclosed are a compound as shown in formula (III) and a pharmaceutically acceptable salt thereof.

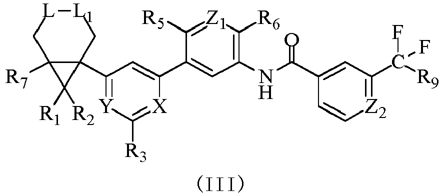

## Description

The present application claims the following right of priority:
CN 201911242225.7, date of filing: December 06, 2019;
CN 202010367751.2, date of filing: April 30, 2020;
CN 202010652149.3, date of filing: July 08, 2020.

### TECHNICAL FIELD

The present disclosure discloses a novel biaryl compound as a Pan-RAF kinase inhibitor, and specifically discloses a compound as shown in formula (III) or a pharmaceutically acceptable salt thereof, and the use thereof in the treatment of cancer-related diseases.

### BACKGROUND

The mitogen-activated protein kinase (MAPK) pathway is an important intracellular signaling transduction pathway, which transmits signals from outside a cell, through the specific cascade phosphorylation of RAS/RAF/MEK/ERK, into the cell nucleus, eventually leading to the activation of specific genes and causing proliferation, apoptosis or differentiation of the cell. Excessive activation of this pathway is closely related to the occurrence of various tumors. RAF, as a very important serine/threonine protein kinase in the RAS/RAF/MEK/ERK signaling pathway, is located downstream of RAS and can be activated by RAS. The RAF family includes three subtypes, ARAF, BRAF and CRAF (RAF-1), with high homology and similar domains. Wild-type RAF is capable of producing homo- or hetero-dimers of the three subtypes. ARAF and CRAF mutations occur less frequently, and BRAF mutation rates are relatively higher. About 5% to 10% of malignant tumor patients, including 66% of melanoma patients, have BRAF mutations. As a key signaling protein downstream of RAS, RAF kinase has important research significance in the treatment of RAS mutant tumors. Inhibiting RAF kinase and regulating MAPK signaling can lead to an effect on the proliferation of RAS mutant tumor cells. Therefore, RAF kinase has become an important target for clinical treatment of tumors.

The first-generation BRAF kinase inhibitors Vemurafenib and Dabrafenib have been approved by FDA for the treatment of cancers with B-Raf^{V600E} mutations. Although Vemurafenib and Dabrafenib have shown promising efficacy in the treatment of B-Raf^{V600E} mutant melanoma, there are still some limitations. In most patients receiving Vemurafenib and Dabrafenib, tumors shrink initially, but relapse within a year (acquired resistance); and the main mechanism of this resistance involves the reactivation of MAPK signaling pathway. The study has found that on the basis of BRAF with occurrence of V600E mutation, NRAS mutation can lead to the activation of MAPK pathway in the presence of an inhibitor, resulting in resistance. Mutated N-Ras promotes the formation of a homo- or hetero-dimer between B-Raf^{V600E} and C-Raf. The inhibitor binds to one monomer of the dimer, which can reduce the affinity of the medicaments for the other monomer, promote the phosphorylation of the monomer on which the inhibitor does not exert an effect and lead to the activation of MEK. At present, the main clinical method for overcoming resistance caused by reactivation of MAPK pathway involves blocking two key sites of the MAPK pathway by using a combination of Raf and MEK inhibitors, thereby delaying the development of resistance. In addition, the development of a new generation of pan-Raf inhibitors to overcome resistance and expand the scope of clinical applications is also in process. Pan-RAF inhibitors can reduce resistance by means of inhibiting dimer activity and blocking paradoxical activation of the pathway. The pan-Raf dimer inhibitors in clinical research mainly include HM95573, TAK-580, BGB-283, LXH254, LY3009120, etc. The development of these novel RAF inhibitors is expected to overcome the resistance of the first-generation inhibitors and further expand clinical applications.

### CONTENT OF THE PRESENT INVENTION

The present disclosure provides a compound as shown in formula (III) or a pharmaceutically acceptable salt thereof, 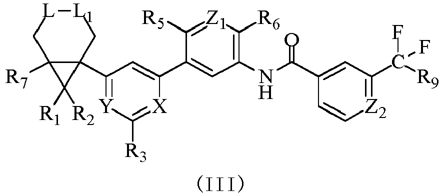
wherein,
X and Y are each independently selected from CH and N;
L is selected from -O-, -S-, -S(=O)- and -S(=O)₂-;
L₁ is selected from -CH₂- and a single bond;
Z₁ and Z₂ are each independently selected from CH and N;
R₁ and R₂ are each independently selected from H, F and C₁₋₃ alkyl, wherein the C₁₋₃ alkyl is optionally substituted with 1, 2 or 3 Rₐ;
R₃ is selected from 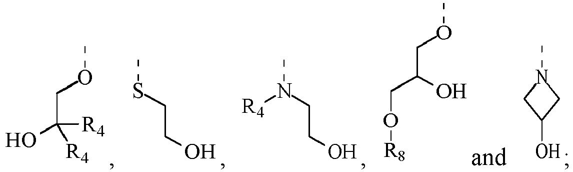
R₄ is selected from H and C₁₋₃ alkyl, wherein the C₁₋₃ alkyl is optionally substituted with 1, 2 or 3 R_{b};
R₅ is selected from H and C₁₋₃ alkyl, wherein the C₁₋₃ alkyl is optionally substituted with 1, 2 or 3 R_{c};
R₆ is selected from H and F;
R₇ is selected from H and CN;
R₈ is selected from H and CH₃;
R₉ is selected from H, F and CH₃;
each Rₐ is independently selected from F, Cl, Br and I;
each R_{b} is independently selected from F, Cl, Br, I and CH₃;
each R_{c} is independently selected from F, Cl, Br and I.

In some embodiments of the present disclosure, the above-mentioned compound is selected from formula (I'), 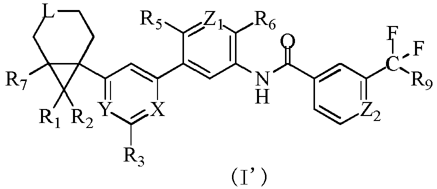
wherein,
X and Y are each independently selected from CH and N;
L is selected from -O-, -S-, -S(=O)- and -S(=O)₂-;
Z₁ and Z₂ are each independently selected from CH and N;
R₁ and R₂ are each independently selected from H, F and C₁₋₃ alkyl, wherein the C₁₋₃ alkyl is optionally substituted with 1, 2 or 3 Rₐ;
R₃ is selected from 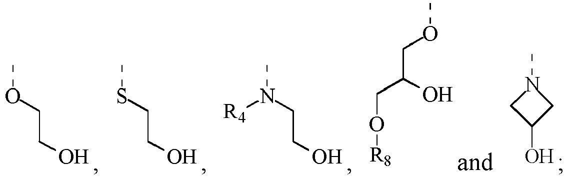
R₄ is selected from H and C₁₋₃ alkyl, wherein the C₁₋₃ alkyl is optionally substituted with 1, 2 or 3 R_{b};
R₅ is selected from H and C₁₋₃ alkyl, wherein the C₁₋₃ alkyl is optionally substituted with 1, 2 or 3 R_{c};
R₆ is selected from H and F;
R₇ is selected from H and CN;
R₈ is selected from H and CH₃;
R₉ is selected from H, F and CH₃;
each Rₐ is independently selected from F, Cl, Br and I;
each R_{b} is independently selected from F, Cl, Br, I and CH₃;
each R_{c} is independently selected from F, Cl, Br and I.

The present disclosure provides a compound as shown in formula (I) or a pharmaceutically acceptable salt thereof, 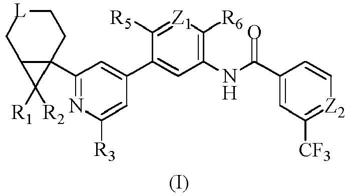
wherein,
L is selected from -O-, -S-, -S(=O)- and -S(=O)₂-;
Z₁ and Z₂ are each independently selected from CH and N;
R₁ and R₂ are each independently selected from H and C₁₋₃ alkyl, wherein the C₁₋₃ alkyl is optionally substituted with 1, 2 or 3 Rₐ;
R₃ is selected from 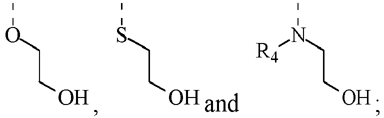
R₄ is selected from H and C₁₋₃ alkyl, wherein the C₁₋₃ alkyl is optionally substituted with 1, 2 or 3 R_{b};
R₅ is selected from H and C₁₋₃ alkyl, wherein the C₁₋₃ alkyl is optionally substituted with 1, 2 or 3 R_{c};
R₆ is selected from H and F;
each Rₐ is independently selected from F, Cl, Br and I;
each R_{b} is independently selected from F, Cl, Br, I and CH₃;
each R_{c} is independently selected from F, Cl, Br and I.

In some embodiments of the present disclosure, the above-mentioned R₁ and R₂ are each independently selected from H and F, and other variables are as defined in the present disclosure.

In some embodiments of the present disclosure, the above-mentioned R₁ and R₂ are selected from H, and other variables are as defined in the present disclosure.

In some embodiments of the present disclosure, the above-mentioned R₃ is selected from 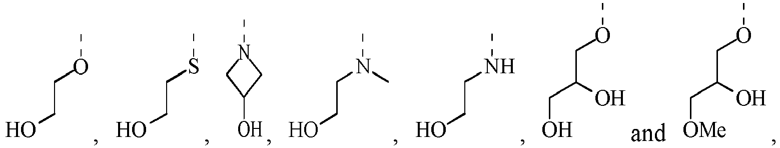 and other variables are as defined in the present disclosure.

In some embodiments of the present disclosure, the above-mentioned R₃ is selected from 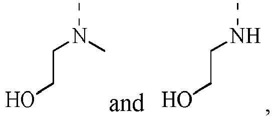 and other variables are as defined in the present disclosure.

In some embodiments of the present disclosure, R₄ is selected from H, CH₃ and CH₂CH₃, and other variables are as defined in the present disclosure.

In some embodiments of the present disclosure, R₅ is selected from CH₃, and other variables are as defined in the present disclosure.

In some embodiments of the present disclosure, the above-mentioned moiety 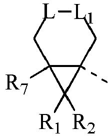 is selected from

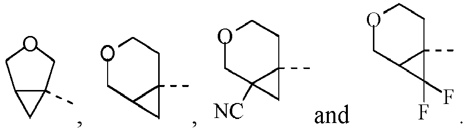

In some embodiments of the present disclosure, the above-mentioned moiety 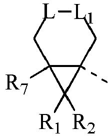 is selected from 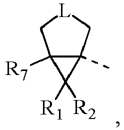 and other variables are as defined in the present disclosure.

In some embodiments of the present disclosure, the above-mentioned moiety 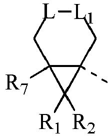 is selected from 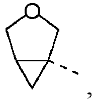 and other variables are as defined in the present disclosure.

In some embodiments of the present disclosure, the moiety 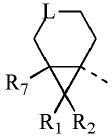 is selected from 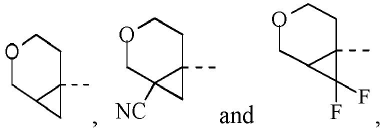 and other variables are as defined in the present disclosure.

In some embodiments of the present disclosure, the moiety 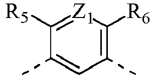 is selected from 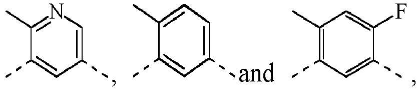 and other variables are as defined in the present disclosure.

In some embodiments of the present disclosure, the above-mentioned moiety 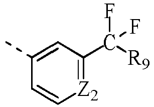 is selected from 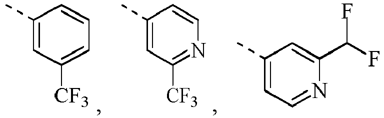 and 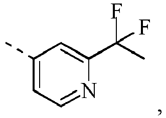 and other variables are as defined in the present disclosure.

In some embodiments of the present disclosure, the above-mentioned moiety 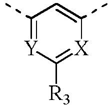 is selected from 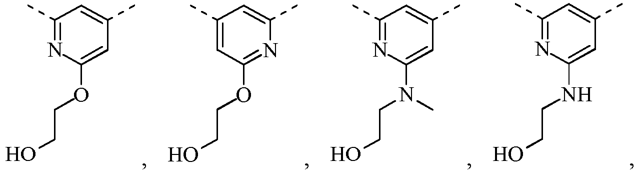 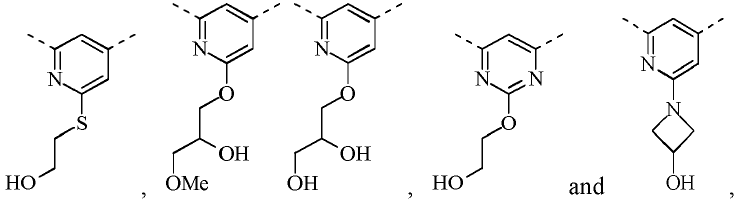 and other variables are as defined in the present disclosure.

In some embodiments of the present disclosure, the compound is selected from 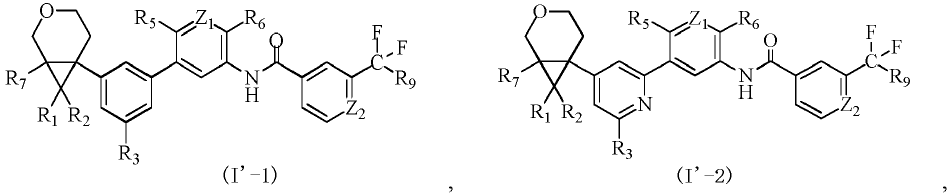 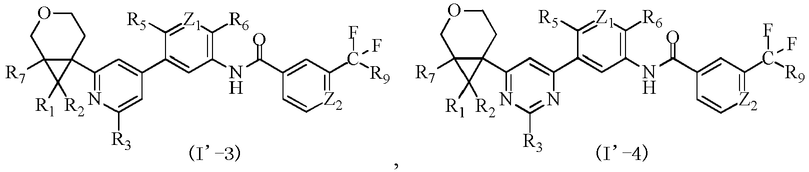 and 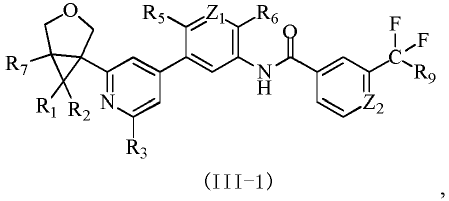 wherein, R₁, R₂, R₃, R₅, R₆, R₇, R₉, Z₁ and Z₂ are as defined in the present disclosure.

In some embodiments of the present disclosure, the compound is selected from 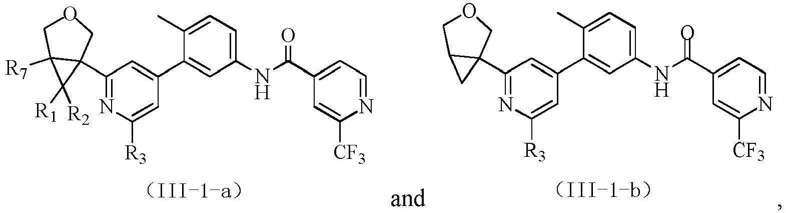 wherein, R₁, R₂, R₃ and R₇ are as defined in the present disclosure.

The present disclosure further provides a compound or a pharmaceutically acceptable salt thereof, wherein, the compound is selected from

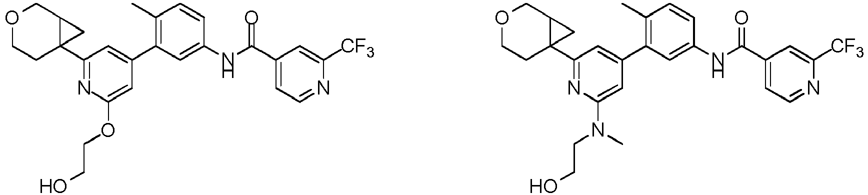

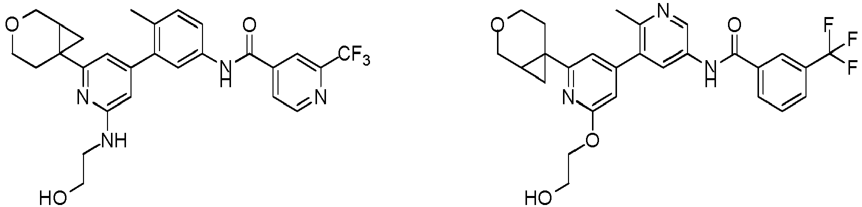

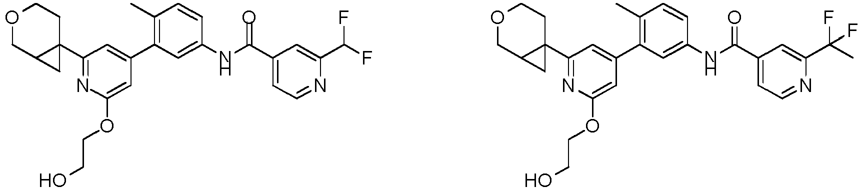

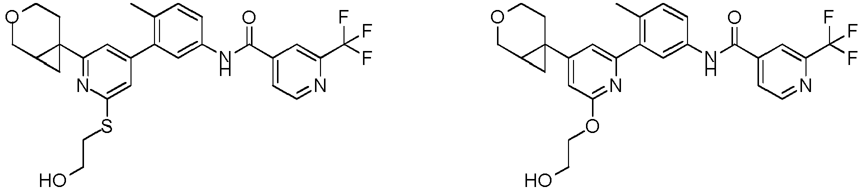

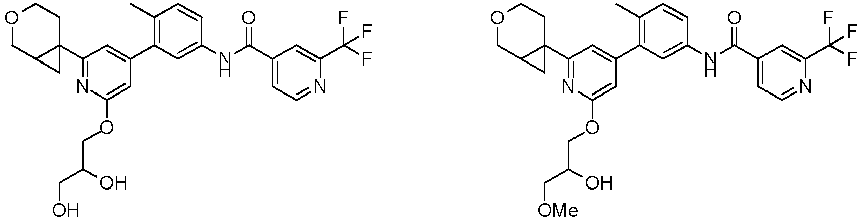

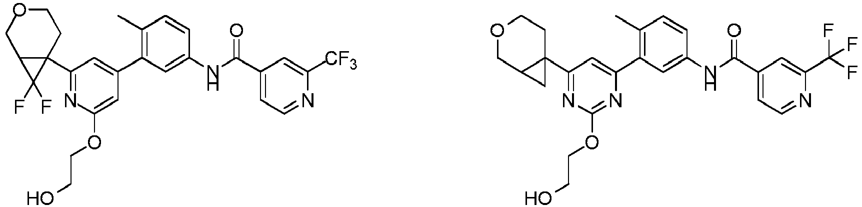

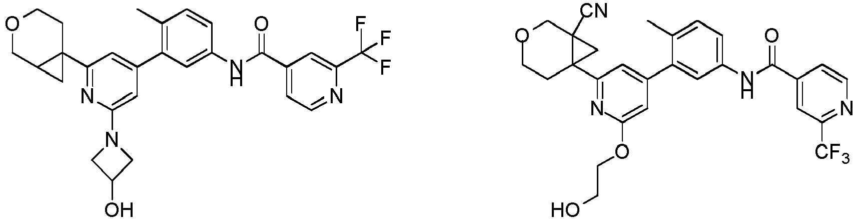

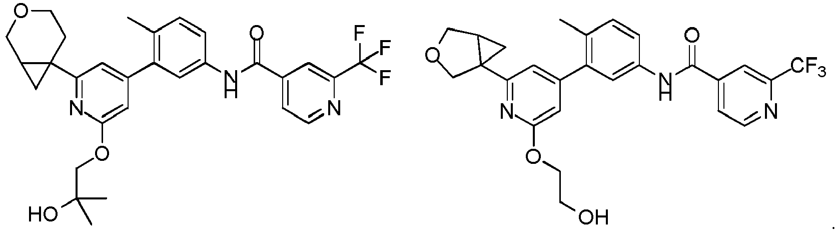

In some embodiments of the present disclosure, the compound is selected from

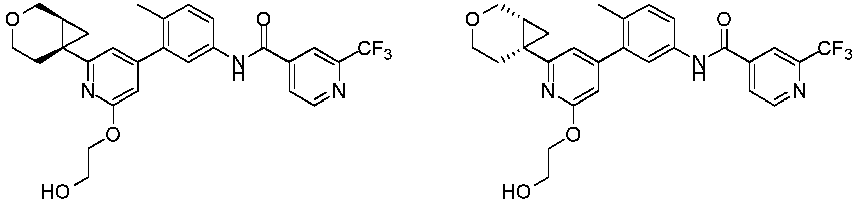

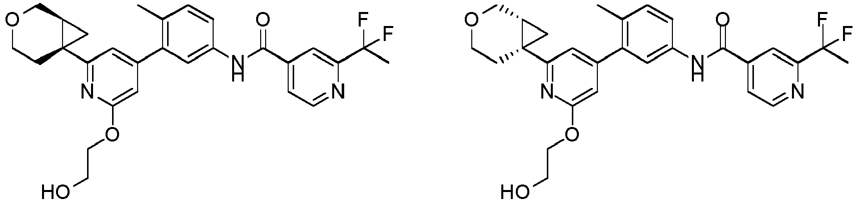

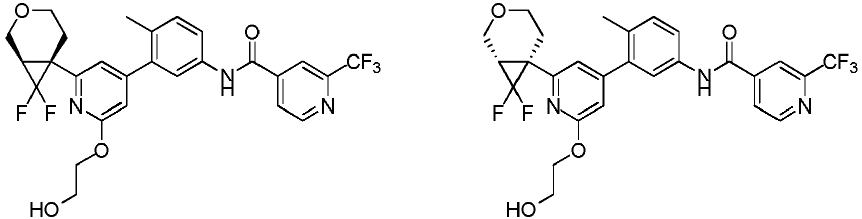

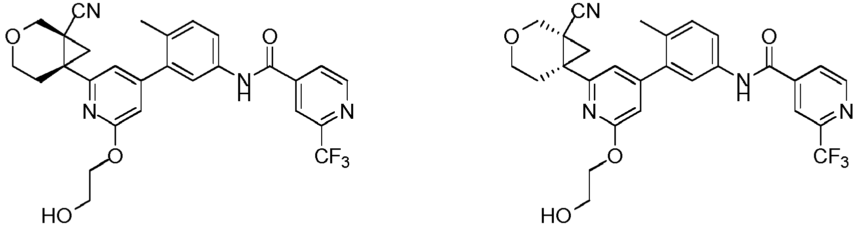

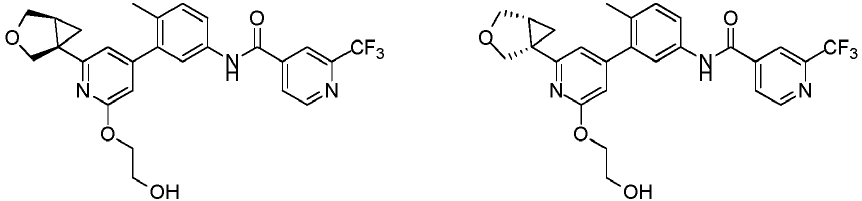

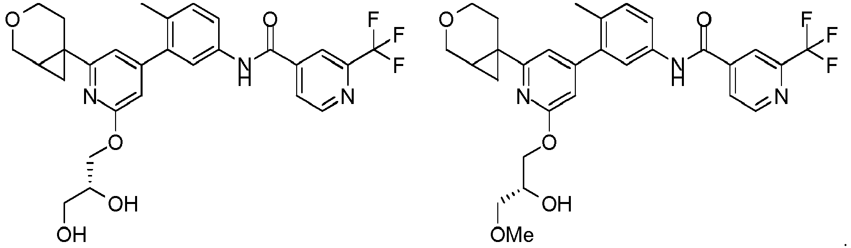

The present disclosure further provides a compound as shown in formula (IV-1), (IV-2), (IV-3) or (IV-4), or a pharmaceutically acceptable salt thereof, 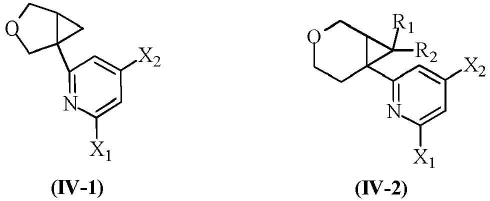 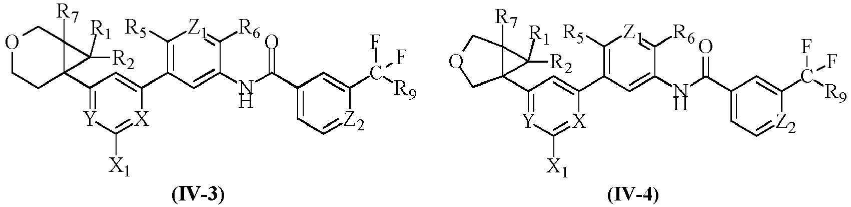
wherein,
X₁ is selected from halogen, -SO₂Me, -OMs, OTf, OTs, 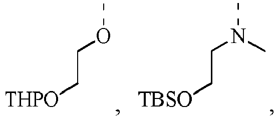 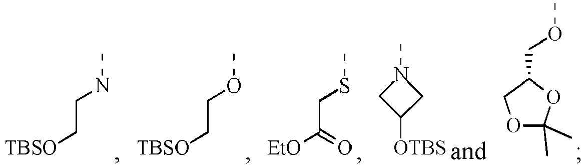
X₂ is selected from halogen, OH, -SO₂Me, -OMs, OTf, OTs and H;
R₁, R₂, R₅, R₆, R₇, R₉, X, Y, Z₁ and Z₂ are as defined in the present disclosure.

The present disclosure further provides a compound as shown in formula (V) or a pharmaceutically acceptable salt thereof, 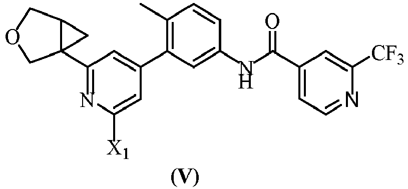 wherein, X₁ is selected from halogen, -SO₂Me, -OMs, OTf, OTs,

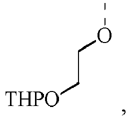

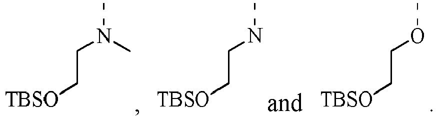

The present disclosure further provides use of the above-mentioned compound or pharmaceutically acceptable salt thereof in the preparation of an RAF kinase inhibitor.

The present disclosure further provides use of the above-mentioned compound or pharmaceutically acceptable salt thereof in the preparation of a medicament for treating cancers.

There are still some embodiments of the present disclosure derived from any combination of the above-mentioned variables.

### TECHNICAL EFFECTS

The compounds of the present disclosure have good drug-like properties, excellent RAF enzyme inhibitory activities, various cell anti-proliferation activities and good in vivo efficacy. It is expected to solve the resistance problem of current therapy for cancers with BRAF^{V600E} mutations and provide effective treatment for RAS mutant cancers.

### Definition and Description

Unless otherwise stated, the following terms and phrases used herein are intended to have the following meanings. A specific term or phrase should not be considered uncertain or unclear unless specifically defined, but should be understood in its ordinary meaning. When a trade name appears herein, it is intended to refer to the corresponding commodity or an active ingredient thereof.

The term "pharmaceutically acceptable" as used herein refers to those compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The term "pharmaceutically acceptable salt" refers to a salt of the compound of the present disclosure, which is prepared from the compound having specific substituents found in the present disclosure with relatively non-toxic acids or bases. When compounds of the present disclosure contain relatively acidic functional groups, base addition salts can be obtained by contacting such compounds with a sufficient amount of base, either in pure solution or a suitable inert solvent. Pharmaceutically acceptable base addition salts include sodium, potassium, calcium, ammonium, organic amine or magnesium salts or similar salts. When compounds of the present disclosure contain relatively basic functional groups, acid addition salts can be obtained by contacting such compounds with a sufficient amount of acid, either in pure solution or a suitable inert solvent. Examples of pharmaceutically acceptable acid addition salts include salts of inorganic acids, which include, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid and phosphorous acid; and salts of organic acids, which include, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and also include salts of amino acids (such as arginine), and salts of organic acids such as glucuronic acid. Certain specific compounds of the present disclosure contain basic and acidic functional groups and thus can be converted to any base or acid addition salt.

The pharmaceutically acceptable salts of the present disclosure can be synthesized from a parent compound containing acid radicals or base radicals by conventional chemical methods. In general, the method for preparing such salts comprises: in water or an organic solvent or a mixture of both, reacting these compounds in free acid or base forms with a stoichiometric amount of a suitable base or acid to prepare the salts.

The compounds of the present disclosure may exist in specific geometric or stereoisomeric forms. The present disclosure contemplates all such compounds, including cis and trans isomers, (-)- and (+)-enantiomers, (R)- and (*S*)-enantiomers, diastereomers, (*D*)-isomers, (*L*)-isomers, and racemic mixtures and other mixtures thereof, such as enantiomerically or diastereomerically enriched mixtures, all of which fall within the scope of the present disclosure. Additional asymmetric carbon atoms may be present in a substituent such as an alkyl group. All these isomers and mixtures thereof are included in the scope of the present disclosure.

Unless otherwise stated, the term "enantiomer" or "optical isomer" refers to stereoisomers that are mirror images of each other.

Unless otherwise stated, the term "cis-trans isomer" or "geometric isomer" is caused by the fact that double bonds or single bonds of ring-forming carbon atoms cannot rotate freely.

Unless otherwise stated, the term "diastereomer" refers to stereoisomers in which molecules have two or more chiral centers and are not mirror images of each other.

Unless otherwise stated, "(+)" represents right-handed, "(-)" represents left-handed, and "(±)" means racemic.

Unless otherwise stated, the wedge-shaped solid bond ( 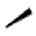 ) and the wedge-shaped dotted bond ( 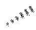 ) represent the absolute configuration of a stereoscopic center; the straight solid bond ( 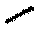 ) and the straight dotted bond ( 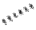 ) represent the relative configuration of a stereoscopic center; the wavy line ( 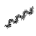 ) represents the wedge-shaped solid bond ( 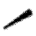 ) or the wedge-shaped dotted bond ( 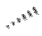 ); or the wavy line ( 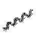 ) represents the straight solid bond ( 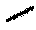 ) and the straight dotted bond ( 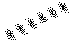 ).

Unless otherwise stated, the term "tautomer" or "tautomeric form" means that at room temperature, isomers with different functional groups are in dynamic equilibrium and can be quickly converted to each other. Where tautomerization is possible (such as in solution), a chemical equilibrium of tautomers can be achieved. For example, proton tautomers (also known as prototropic tautomers) include interconversion via migration of a proton, such as keto-enol isomerization and imine-enamine isomerization. Valence tautomers include some interconversions by recombination of some of bond-forming electrons. A specific example of keto-enol tautomerization is the interconversion between two tautomers, pentane-2,4-dione and 4-hydroxypent-3-en-2-one.

Unless otherwise stated, the term "rich in one isomer", "isomer enriched", "rich in one enantiomer" or "enantiomerically enriched" refers to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomeric excess (ee value) is 80%.

Optically active (*R*)- and (*S*)-isomers and *D* and *L* isomers can be prepared using chiral synthesis or chiral reagents or other conventional techniques. If a particular enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as an amino group) or an acidic functional group (such as a carboxyl group), diastereomeric salts can be formed with an appropriate optically active acid or base, followed by resolution of the diastereomers using conventional methods well known in the art, and subsequent recovery of the pure enantiomers. In addition, separation of enantiomers and diastereomers is frequently accomplished using chromatography, which uses chiral stationary phases, optionally in combination with chemical derivatization methods (e.g., formation of carbamates from amines).

"Optional" or "optionally" means that the subsequently described event or circumstance may, but not necessarily occur, and that the description includes instances where said event or circumstance occurs and instances where said event or circumstance does not occur.

The term "substituted" means that any one or more hydrogen atoms on the designated atom are substituted by a substituent, which may include heavy hydrogen and hydrogen variants, provided that the valence state of the designated atom is normal, and the substituted compound is stable. Where the substituent is oxygen (i.e., =O), it means that two hydrogen atoms are substituted. Oxygen substitution does not occur on aromatic groups. The term "optionally substituted" means that it may or may not be substituted. Unless otherwise specified, the type and number of substituents may be arbitrary on the basis that they can be achieved in chemistry.

Where any variable (such as R) appears more than once in the composition or structure of a compound, its definition in each case is independent. Thus, for example, if a group is substituted with 0-2 R, the group can optionally be substituted with up to two R, and R in each case has independent options. In addition, combinations of substituents and/or variants thereof are permissible only if such combinations result in stable compounds.

When the number of a linking group is 0, such as -(CRR)o-, it means that the linking group is a single bond. -Co alkyl-A means that the structure is actually -A.

When the number of a substituent is 0, it means that the substituent does not exist. For example, -A-(R)o means that the structure is actually -A.

When a substituent is vacant, it means that the substituent does not exist. For example, when X is vacant in A-X, it means that the structure is actually A.

When one of the variables is selected from a single bond, it means that the two groups to which it is connected are directly connected. For example, when L represents a single bond in A-L-Z, it means that the structure is actually A-Z.

When the substituents listed do not indicate through which atom they are connected to the substituted group, such substituents can be bonded through any of the atoms thereof, for example, pyridyl as a substituent can be attached to the substituted group via any carbon atom on the pyridine ring.

When the linking group listed does not indicate the linking direction thereof, the linking direction is arbitrary, for example, the linking group L is -M-W- in 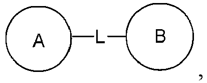 at this situation, -M-W- can connect ring A and ring B in the same direction as the reading order from left to right to form 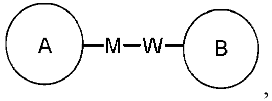 and can also connect ring A and ring B in the opposite direction as the reading order from left to right to form 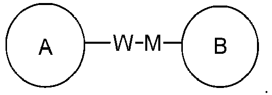 Combinations of the linking groups, substituents, and/or variants thereof are permissible only if such combinations result in stable compounds.

Unless otherwise specified, when a group has one or more connectable sites, any one or more sites of the group can be connected to other groups through chemical bonds. The chemical bonds between the sites and other groups can be represented by a straight solid bond 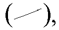 a straight dotted bond 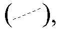 or a wavy line 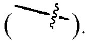 For example, the straight solid bond in -OCH₃ means that the group is connected to other groups through the oxygen atom in the group; the straight dotted bond in 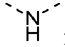 means that the group is connected to other groups through the two ends of the nitrogen atom in the group; the wavy line in 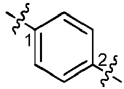 means that the group is connected to other groups through the 1 and 2 carbon atoms in the phenyl group.

Unless otherwise specified, the term "C₁₋₃ alkyl" is used to represent a linear or branched saturated hydrocarbon group consisting of 1 to 3 carbon atoms. The C₁₋₃ alkyl includes C₁₋₂ alkyl, C₂₋₃ alkyl, etc.; and it can be monovalent (such as methyl), divalent (such as methylene) or multivalent (such as methine). Examples of C₁₋₃ alkyl include, but are not limited to, methyl (Me), ethyl (Et), propyl (including *n*-propyl and isopropyl), etc.

Unless otherwise specified, Cn-n+m or Cn-Cn+m includes any specific case of n to n+m carbons, for example, C₁₋₁₂ includes C₁, C₂, C₃, C₄, C₅, C₆, C₇, C₈, C₉, C₁₀, C₁₁ and C₁₂, and also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂; Similarly, n-membered to n+m-membered means that the number of atoms in the ring is n to n+m, for example, a 3- to 12-membered ring includes a 3-membered ring, a 4-membered ring, a 5-membered ring, a 6-membered ring, a 7-membered ring, a 8-membered ring, a 9-membered ring, a 10-membered ring, a 11-membered ring, and a 12-membered ring, and also includes any range from n to n+m, for example, a 3- to 12-membered ring includes a 3- to 6-membered ring, a 3- to 9-membered ring, a 5- to 6-membered ring, a 5-to 7-membered ring, a 6- to 7-membered ring, a 6- to 8-membered ring, and a 6- to 10-membered ring.

The term "leaving group" refers to a functional group or atom that can be substituted by another functional group or atom through a substitution reaction (e.g., an affinity substitution reaction). For example, representative leaving groups include trifluoromethanesulfonate; chlorine, bromine and iodine; sulfonates, such as methanesulfonate, tosylate, p-bromobenzenesulfonate, and p-toluenesulfonate; and acyloxy, such as acetoxy and trifluoroacetoxy.

The term "protecting group" includes, but is not limited to, "amino protecting group", "hydroxy protecting group" or "mercapto protecting group". The term "amino protecting group" refers to a protecting group suitable for preventing side reactions occurring at the nitrogen atom of an amino group. Representative amino protecting groups include, but are not limited to: formyl; acyl, such as alkanoyl (e.g., acetyl, trichloroacetyl or trifluoroacetyl); alkoxycarbonyl, such as tert-butoxycarbonyl (Boc); aryl methoxycarbonyl, such as benzyloxycarbonyl (Cbz) and 9-fluorenylmethoxycarbonyl (Fmoc); aryl methyl, such as benzyl (Bn), triphenyl methyl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for preventing side reactions of a hydroxyl group. Representative hydroxyl protecting groups include, but are not limited to: alkyl, such as methyl, ethyl and tert-butyl; acyl, such as alkanoyl (e.g., acetyl); arylmethyl such as benzyl (Bn), p-methoxybenzyl (PMB), 9-fluorenylmethyl (Fm) and diphenylmethyl (benzhydryl, DPM); silyl such as trimethylsilyl (TMS) and tert-butyl dimethylsilyl (TBS).

The compounds of the present disclosure can be prepared by various synthetic methods well known to a person skilled in the art, including the specific embodiments listed below, the embodiments formed by the combination with other chemical synthesis methods, and equivalent alternative embodiments well known to a person skilled in the art, wherein the preferred embodiments include but are not limited to the examples of the present disclosure.

The structure of the compound of the present disclosure can be confirmed by conventional methods well known to a person skilled in the art. If the present disclosure relates to the absolute configuration of the compound, the absolute configuration can be confirmed by conventional technical means in the art. For example, single-crystal X-ray diffraction (SXRD) uses a Bruker D8 venture diffractometer to collect the diffraction intensity data of the cultivated single crystal, with a light source of CuKα radiation, and a scanning mode of ϕ/ω scanning. After the related data is collected, a direct method (Shelxs97) is further used to resolve the crystal structure, so that the absolute configuration can be confirmed. The absolute configuration of a compound can also be confirmed by the chiral structure of the starting material and the reaction mechanism of asymmetric synthesis.

The solvents used in the present disclosure are commercially available.

The present disclosure uses the following abbreviations: HATU represents 2-(7-azobenzotriazole)-N,N,N',N'-tetramethylurea hexafluorophosphate; HOBt represents 1-hydroxybenzo triazole; T₃P represents tri-n-propyl cyclic phosphoric anhydride; Pd₂dba₃ represents tris-diphenylacetone-dipalladium; Ruphos represents 2-dicyclohexylphosphino-2',6'-diisopropoxy-1,1'-biphenyl; Brettphos represents (2-dicyclohexylphosphino-3,6-dimethoxy-2',4',6'-triisopropyl-1,1'-biphenyl; TBAF represents tetrabutylammonium fluoride; [Ir(COD)OMe]₂ represents cyclooctadiene methoxy iridium dimer; tmphen represents 3,4,7,8-tetramethyl-1,10 phenanthroline; dtbpy represents 4,4'-ditertbutyl-2,2'-bipyridine. Pd(dppf)Cl₂ represents diphenylphosphine ferrocene palladium dichloride; Pd(dppf)Cl₂·DCM represents a complex of diphenylphosphine ferrocene palladium dichloride and dichloromethane; DIAD represents diisopropyl azodicarboxylate; DIPEA or DIEA represents diisopropylethylamine; PPh₃ represents triphenylphosphine; TFA represents trifluoroacetic acid; THP represents 2-tetrahydropyran; OTHP represents 2-oxotetrahydropyran; TBSCl represents tert-butyl dimethylsilyl chloride; TBS represents tert-butyldimethylsilyl; OTBS represents tert-butyldimethylsilyloxy; DMF represents N,N-dimethylformamide; DMSO represents dimethyl sulfoxide; EA represents ethyl acetate; PE represents petroleum ether; EtOH represents ethanol; MeOH represents methanol; DME represents ethylene glycol dimethyl ether; DCM represents dichloromethane; THF represents tetrahydrofuran; MeCN represents acetonitrile; NMP represents N-methylpyrrolidone; PE/EA represents a volume ratio of petroleum ether and ethyl acetate; DCM/MeOH represents a volume ratio of dichloromethane and methanol; HCOOH-MeCN-H₂O represents a separation system of formic acid-acetonitrile-water; IPA represents isopropanol; Me represents methyl; OMs represents methanesulfonyloxy.

### BRIEF DESCRIPTION OF THE DRAWINGS

Fig. 1 shows the tumor growth curves of tumor-bearing mice as human lung cancer Calu-6 subcutaneous xenograft tumor models after administration of compound 1.
Fig. 2 shows the body weight of tumor-bearing mice as human lung cancer Calu-6 subcutaneous xenograft tumor models in the process of administration of compound 1.
Fig. 3 shows the tumor growth curves of tumor-bearing mice as human lung cancer Calu-6 subcutaneous xenograft tumor models after administration of compound 16B.
Fig. 4 shows the body weight of tumor-bearing mice as human lung cancer Calu-6 subcutaneous xenograft tumor models in the process of administration of compound 16B.

### DETAILED DESCRIPTION OF THE PREFERRED EMBODIMENT

The present disclosure will be described in detail with the following examples, but not imply any adverse limitation to the present disclosure. The compounds of the present disclosure can be prepared by various synthetic methods well known to a person skilled in the art, including the specific embodiments listed below, the embodiments formed by the combination with other chemical synthesis methods, and equivalent alternative embodiments well known to a person skilled in the art, wherein the preferred embodiments include but are not limited to the examples of the present disclosure. For a person skilled in the art, without departing from the spirit and scope of the present disclosure, all the variations and improvements made to the specific embodiments of the present disclosure would have been obvious.

### Synthesis of intermediate 1A-5:

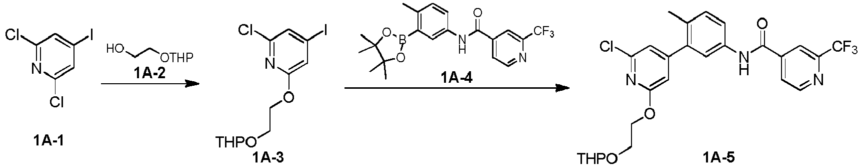

### Compound 1A-3

At 0 °C, sodium hydride (43.81 mg, 1.10 mmol, 60% purity) was added to a solution of 1A-2 (192.15 mg, 1.31 mmol) in THF (1 mL), and the mixture was warmed to 25 °C and stirred for 30 minutes. At 0 °C, 1A-1 (300 mg, 1.10 mmol) was added to the mixed solution; and the mixture was warmed to 25 °C and stirred for 2 hours. A saturated ammonium chloride solution (10 mL) was added to the reaction mixture, and the resulting mixture was extracted with ethyl acetate (10 mL × 3); the combined extract was concentrated; and the residue was separated by column chromatography (PE/EA= 20/1, V/V) to obtain 1A-3.

### Compound 1A-5

1A-3 (200 mg, 521.36 µmol), 1A-4 (232.96 mg, 573.50 µmol), Pd(dppf)Cl₂ (38.15 mg, 52.14 µmol) and sodium carbonate (110.52 mg, 1.04 mmol) were dissolved in a mixed solvent of dioxane (2 mL) and water (0.4 mL). Under nitrogen protection, the reaction mixture was heated to 100 °C and stirred for 2 hours. The reaction solution was diluted with water (10 mL) and extracted with dichloromethane (10 × 2 mL). The combined organic phase was dried over anhydrous sodium sulfate and then concentrated; and the residue was separated by preparative silica gel plate (PE/EA = 5/1, V/V) to obtain 1A-5. ¹H NMR (400MHz, DMSO-*d*₆) δ 9.00 (d, *J*=5.0 Hz, 1H), 8.37 (s, 1H), 8.20 (d, *J*=4.8 Hz, 1H), 7.77 (dd, *J*=2.2, 8.2 Hz, 1H), 7.70 (d, *J*=2.2 Hz, 1H), 7.37 (d, *J*=8.4 Hz, 1H), 7.12 (d, *J*=1.0 Hz, 1H), 6.86 (d, *J*=1.0 Hz, 1H), 4.68 - 4.64 (m, 1H), 4.52 - 4.40 (m, 2H), 3.82 - 3.71 (m, 2H), 3.48 - 3.41 (m, 2H), 2.26 (s, 3H), 1.75 - 1.58 (m, 2H), 1.53 - 1.40 (m, 4H).

### Synthesis of intermediate 1A-6:

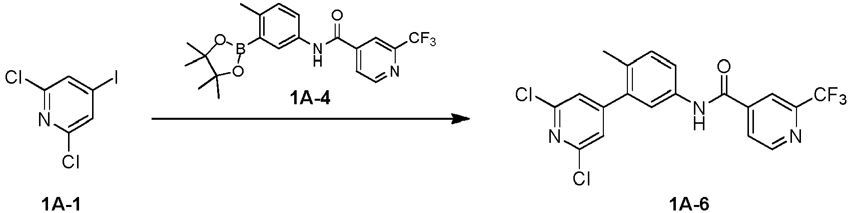

### Compound 1A-6

1A-1 (1.0 g, 3.65 mmol), 1A-4 (1.67 g, 4.11 mmol), Pd(dppf)Cl₂ (300 mg, 367.36 µmol) and sodium carbonate (774 mg, 7.30 mmol) were dissolved in a mixed solvent of dioxane (10 mL) and water (2.5 mL). Under nitrogen protection, the reaction mixture was heated to 70 °C and stirred for 4 hours. The reaction solution was filtered and extracted with ethyl acetate (10 × 3 mL). The combined organic phase was dried over anhydrous sodium sulfate and then concentrated; and the residue was separated by silica gel column chromatography (PE/EA = 30/1 to 3/1, V/V) to obtain 1A-6. ¹HNMR (400MHz, CDCl₃) δ 8.98-8.91 (d, *J*=5.0 Hz, 1H), 8.11 (s, 1H), 8.06 (s, 1H), 7.97-7.92 (d, *J*=5.0 Hz, 1H), 7.61 - 7.56 (m, 2H), 7.39-7.30 (d, *J*=9.0 Hz, 1H), 7.28 - 7.26 (m, 2H), 2.37 - 2.22 (m, 3H).

### Synthesis of intermediate 1A-8:

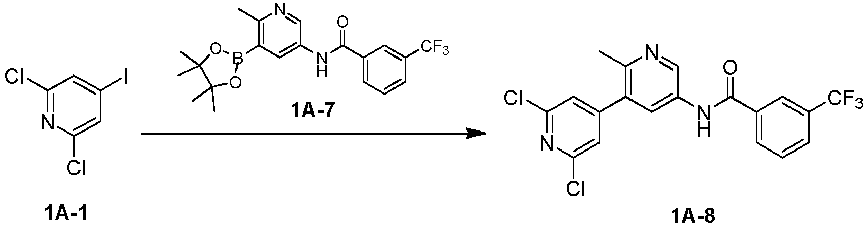

### Compound 1A-8

1A-1 (785 mg, 2.87 mmol), 1A-7 (1.11 g, 2.72 mmol), Pd(dppf)Cl₂ (210 mg, 287.0 µmol) and sodium carbonate (609 mg, 5.75 mmol) were dissolved in a mixed solvent of DME (10 mL) and water (2.5 mL). Under nitrogen protection, the reaction mixture was heated to 90 °C and stirred for 2 hours. Ethyl acetate (50 mL) was added to the reaction solution, and the mixture was then filtered and washed with water (50 × 2 mL) and saturated brine (80 ×1 mL). The combined organic phase was dried over anhydrous sodium sulfate and then concentrated; and the residue was separated by silica gel column chromatography (PE/EA = 20/1 to 3/1, V/V) to obtain 1A-8. ¹HNMR (400 MHz, CDCl₃) δ 8.65 (d, *J*=2.51 Hz, 1H), 8.26 (d, *J*=2.51 Hz, 1 H), 8.15 (s, 1 H), 8.10 (d, *J*=7.91 Hz, 1 H), 8.03 (s, 1 H), 7.87 (d, *J*=7.78 Hz, 1 H), 7.69 (t, *J*=7.78 Hz, 1 H), 7.31 (s, 2 H), 2.53 (s, 3 H).

### Synthesis of intermediate 2-1:

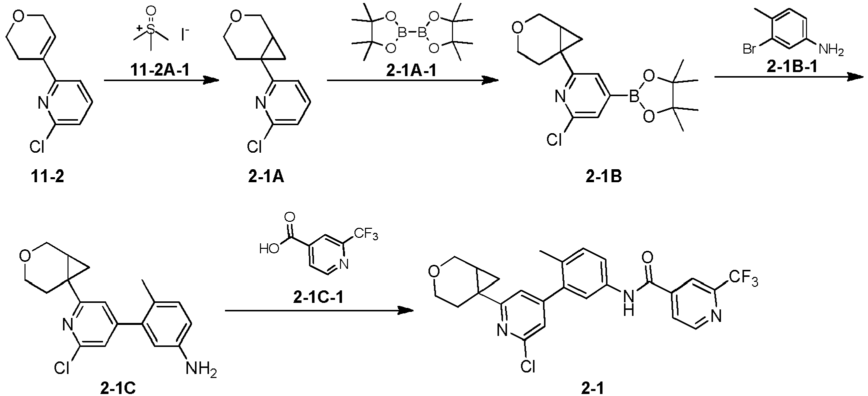

### Compound 2-1A

DMSO (4 mL) was added to a mixture of sodium hydride (163.18 mg, 4.08 mmol, 60%) and 11-2A-1 (897.89 mg, 4.08 mmol). Under nitrogen protection, the reaction mixture was stirred at 20 °C for 0.5 hours. A solution of 11-2 (200 mg, 1.02 mmol) in DMSO (4 mL) was added to the reaction solution; and the reaction mixture was heated to 80 °C and stirred for 2.5 hours. Water (2 mL) and ethyl acetate (40 mL) were added to the reaction mixture, and the resulting mixture was washed with water (20 × 2 mL); the organic phase was dried over anhydrous sodium sulfate and then concentrated; and the residue was separated by preparative silica gel plate (PE/EA= 5/1, V/V) to obtain compound 2-1A. ¹HNMR (400 MHz, CDCl₃) δ 7.65 - 7.47 (m, 1H), 7.21 - 7.02 (m, 2H), 4.11 - 3.91 (m, 2H), 3.74 - 3.57 (m, 1H), 3.56 - 3.37 (m, 1H), 2.55 - 2.42 (m, 1H), 2.20 - 2.05 (m, 1H), 1.85 - 1.71 (m, 1H), 1.39 - 1.28 (m, 1H), 1.16 - 1.04 (m, 1H).

### Compound 2-1B

2-1A-1 (395.45 mg, 1.56 mmol), [Ir(COD)OMe]₂ (23.71 mg, 35.77 µmol), tmphen (16.91 mg, 71.54 µmol) and potassium acetate (11.5 g, 117.18 mmol) were dissolved in MTBE (8 mL). Under nitrogen protection, the reaction mixture was heated to 80 °C and stirred for 5 minutes. A solution of 2-1A (300 mg, 1.43 mmol) in MTBE (2 mL) was then added, and the mixture was warmed to 80 °C and stirred for 16 hours. The reaction solution was filtered and concentrated to obtain compound 2-1B as a crude. MS (ESI): m/z 254.1 [M+H-82]⁺.

### Compound 2-1C

2-1B (480 mg, 1.43 mmol), 2-1B-1 (320 mg, 1.72 mmol), Pd(dppf)Cl₂ (117 mg, 143 µmol) and sodium carbonate (454.74 mg, 4.29 mmol) were dissolved in a mixed solvent of DME (10 mL) and water (2 mL). Under nitrogen protection, the reaction mixture was heated to 80 °C and stirred for 2 hours. Ethyl acetate (20 mL) was added to the reaction solution, and the mixture was then filtered; the filtrate was washed with water (20 × 3 mL); the organic phase was dried over anhydrous sodium sulfate and then concentrated; and the residue was separated by silica gel column chromatography (PE/EA = 20/1 to 10/1) to obtain compound 2-1C. ¹H NMR (400 MHz, CDCl₃) δ 7.09 - 7.06 (m, 2H), 7.05 (d, *J=* 1.0 Hz, 1H), 6.70 - 6.64 (m, 1H), 6.53 (d, *J=* 2.6 Hz, 1H), 4.07 - 4.00 (m, 1H), 3.99 - 3.93 (m, 1H), 3.70 - 3.59 (m, 3H), 3.52 - 3.42 (m, 1H), 2.56 - 2.47 (m, 1H), 2.16 - 2.14 (m, 3H), 2.14 (br s, 1H), 1.75 - 1.60 (m, 1H), 1.40 - 1.33 (m, 1H), 1.11 (dd, *J* = 4.2, 6.4 Hz, 1H).

### Compound 2-1

HATU (181.17 mg, 476.48 µmol) and DIPEA(123.16 mg, 952.96 µmol) were added to 2-1C (100 mg, 317.65 µmol) and 2-1C-1 (60.71 mg, 317.65 µmol) in DMF (3 mL), and the mixture was stirred at 20 °C for 2 hours. The reaction mixture was diluted with ethyl acetate (20 mL) and washed with water (10 × 3 mL); the organic phase was combined, dried over anhydrous sodium sulfate and concentrated; and the residue was purified by preparative silica gel plate (PE/EA= 2/1, V/V) to obtain compound 2-1. ¹HNMR (400 MHz, CDCl₃) δ 8.94 (d, *J=* 5.0 Hz, 1H), 8.16 - 8.06 (m, 1H), 8.00 - 7.85 (m, 2H), 7.61 - 7.56 (m, 1H), 7.55 - 7.51 (m, 1H), 7.36 - 7.31 (m, 1H), 7.11 - 7.06 (m, 2H), 4.07 - 4.01 (m, 1H), 3.99 - 3.93 (m, 1H), 3.68 - 3.59 (m, 1H), 3.53 - 3.43 (m, 1H), 2.57 - 2.47 (m, 1H), 2.30 - 2.25 (m, 3H), 2.18 - 2.08 (m, 1H), 1.88 - 1.79 (m, 1H), 1.41 - 1.35 (m, 1H), 1.20-1.10 (dd, *J=* 4.2, 6.4 Hz, 1H). MS (ESI) m/z: 488.1 [M+H]⁺.

### Synthesis of intermediate 14-2A:

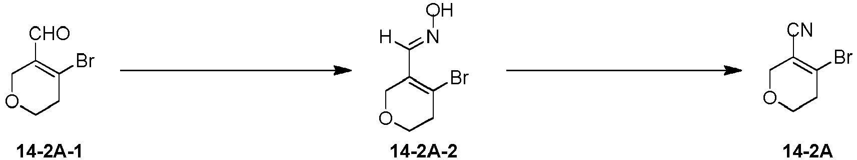

### Compound 14-2A-2

Hydroxylamine hydrochloride (4.77 g, 68.71 mmol) and triethylamine (16.69 g, 164.90 mmol) were added to a solution of 14-2A-1 (10.5 g, 54.97 mmol) in DMF (100 mL), and the mixture was stirred at 20 °C for 1 hour. T₃P (34.98 g, 54.97 mmol, 50% DMF solution) was added to the reaction solution, and the mixture was stirred at 20 °C for 4 hours. A saturated sodium bicarbonate solution (200 mL) was added to the reaction mixture; the resulting mixture was extracted with EtOAc (50 mL × 3); and the combined extract was dried over anhydrous sodium sulfate and then concentrated to obtain 14-2A-2 as a crude. ¹H NMR (400MHz, CDCl₃) δ 9.24 - 8.56 (m, 1H), 8.08 (s, 1H), 4.35-4.28 (t, *J*=2.2 Hz, 2H), 3.85-3.76 (t, *J*=5.6 Hz, 2H), 2.70 - 2.62 (m, 2H).

### Compound 14-2A

Thionyl chloride (15.67 g, 131.74 mmol) was added to a solution of 14-2A-2 (9.5 g, 46.11 mmol) in DCM (100 mL), and the mixture was stirred at 20 °C for 1 hour. At 0 °C, a saturated sodium bicarbonate solution (100 mL) was slowly added dropwise to the reaction mixture; the resulting mixture was extracted with DCM (100 mL × 2); and the combined extract was dried over anhydrous sodium sulfate and then concentrated to obtain 14-2A as a crude. ¹HNMR (400MHz, CDCl₃) δ 4.30-4.20 (t, *J*=2.6 Hz, 2H), 3.92-3.85 (t, *J*=5.4 Hz, 2H), 2.75-2.64 (tt, *J*=2.6, 5.4 Hz, 2H).

### Example 1

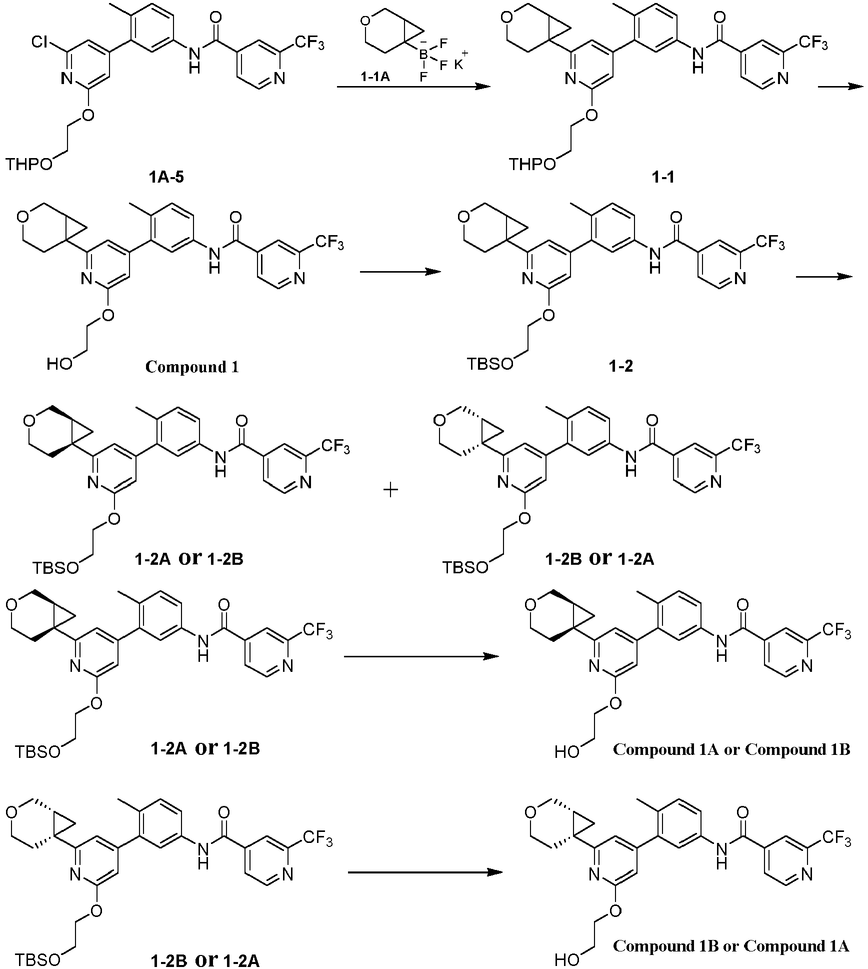

### Compound 1-1

1A-5 (100 mg, 186.59 µmol), 1-1A(77 mg, 377.38 µmol), Pd(dppf)Cl₂ (14 mg, 19.13 µmol) and sodium carbonate (40 mg, 377.40 µmol) were dissolved in a mixed solvent of DME (2 mL) and water (0.2 mL). Under nitrogen protection, the reaction mixture was heated to 100 °C and stirred for 16 hours. The reaction solution was filtered; the filtrate was concentrated; and the residue was separated by preparative silica gel plate (PE/EA= 5/1, V/V) to obtain a crude, which was then separated by chromatographic column (silica gel) (HCOOH-MeCN-H₂O) to obtain compound 1-1. ¹H NMR (400 MHz, CDCl₃) δ 8.99-8.90 (d, *J* = 5.0 Hz, 1H), 8.11 (s, 1H), 7.96 - 7.89 (m, 2H), 7.67-7.58 (dd, *J* = 2.0, 8.6 Hz, 1H), 7.49-7.40 (d, *J* = 1.8 Hz, 1H), 7.36-7.29 (d, *J* = 8.4 Hz, 1H), 6.76 (s, 1H), 6.55-6.49 (d, *J* = 0.8 Hz, 1H), 4.74-4.68 (t, *J* = 3.6 Hz, 1H), 4.60 - 4.45 (m, 2H), 4.09 - 4.02 (m, 2H), 3.98 - 3.88 (m, 2H), 3.89-3.76 (ddd, *J* = 4.0, 6.4, 11.2 Hz, 1H), 3.66 - 3.58 (m, 1H), 3.57 - 3.44 (m, 2H), 2.58-2.45 (td, *J* = 5.2, 13.8 Hz, 1H), 2.27 (s, 3H), 2.14 - 2.07 (m, 1H), 1.89 - 1.81 (m, 2H), 1.77 - 1.71 (m, 1H), 1.68 - 1.61 (m, 2H), 1.45-1.36 (dd, *J* = 4.0, 9.2 Hz, 1H), 1.10-1.02 (dd, *J* = 4.0, 6.4 Hz, 1H), 0.99 - 0.73 (m, 2H).

### Compound 1

Trifluoroacetic acid (616 mg, 5.4 mmol) was added to 1-1 (54 mg, 90.36 µmol) in dichloromethane (2 mL), and the reaction solution was stirred at 25 °C for 0.5 hours. The reaction solution was diluted with dichloromethane (20 mL) and adjusted to neutral with a saturated sodium bicarbonate aqueous solution. The organic phase was washed with saturated brine (15 × 1 mL), dried over anhydrous sodium sulfate and then concentrated; and the residue was separated by preparative silica gel plate (PE/EA = 1/1, V/V) to obtain compound 1. ¹H NMR (400 MHz, CDCl₃) δ 8.99-8.90 (d, *J* = 5.0 Hz, 1H), 8.11 (s, 1H), 7.96 - 7.85 (m, 2H), 7.67-7.59 (dd, *J* = 1.8, 8.0 Hz, 1H), 7.51 (s, 1H), 7.37-7.28 (d, *J* = 8.6 Hz, 1H), 6.84-6.78 (d, *J* = 1.0 Hz, 1H), 6.57-6.52 (d, *J* = 1.0 Hz, 1H), 4.53 - 4.46 (m, 2H), 4.08 - 4.03 (m, 1H), 4.00 - 3.94 (m, 3H), 3.69 - 3.58 (m, 1H), 3.54-3.36 (ddd, *J* = 5.2, 8.6, 11.6 Hz, 1H), 2.53-2.46 (td, *J* = 5.0, 14.0 Hz, 1H), 2.28 (s, 3H), 2.18-2.09 (ddd, *J* = 5.4, 8.4, 13.8 Hz, 1H), 1.79 - 1.72 (m, 1H), 1.39-1.28 (dd, *J* = 4.2, 8.8 Hz, 1H), 1.11 - 1.03 (m, 1H). MS (ESI) m/z: 514.2 [M+H]⁺.

### Compound 1-2

TBSCl (10 mg, 48.68 µmol) was added to a solution of compound 1 (25 mg, 48.68 µmol) and imidazole (8 mg, 117.51 µmol) in dichloromethane (3 mL). The reaction mixture was stirred at 25 °C for 12 hours. The reaction mixture was diluted with dichloromethane (20 mL) and washed with water (30 × 1 mL) and saturated brine (20 × 1 mL); the organic phase was dried over anhydrous sodium sulfate; the filtrate was concentrated; and the residue was separated by preparative silica gel plate (PE/EA = 2/1, V/V) to obtain compound 1-2. MS (ESI) m/z: 628.3 [M+H]⁺.

### Compound 1-2A and Compound 1-2B

Compound 1-2 was subjected to SFC chiral separation (chiral column: REGIS (s,s) WHELK-O1 (250 mm^{∗}50 mm, 10 µm), mobile phase A: isopropanol (containing 0.05% DIEA); mobile phase B: carbon dioxide) to obtain compound 1-2A (retention time: 3.846 min) and compound 1-2B (retention time: 3.966 min). Compound 1-2A: MS (ESI) m/z: 628.3 [M+H] ⁺, 99.4% (ee%); Compound 1-2B: MS (ESI) m/z: 628.3 [M+H]⁺ , 94.3% (ee%).

### Compound 1A

TBAF (1 M, 0.048 mL) was added to 1-2A(15 mg, 23.89 µmol) in THF (2 mL), and the reaction solution was stirred at 20 °C for 0.5 hours. The reaction solution was respectively diluted with ethyl acetate (30 mL) and washed with water (20 × 2 mL); the organic phase was dried over anhydrous sodium sulfate and then concentrated; and the residue was separated by preparative silica gel plate (PE/EA= 1/2, V/V) to obtain compound 1A. ¹H NMR (400 MHz, CDCl₃) δ 8.96-8.90 (d, *J=* 5.0 Hz, 1H), 8.15 - 8.11 (m, 1H), 8.08 (s, 1H), 7.98 - 7.94 (m, 1H), 7.65 - 7.58 (m, 1H), 7.55-7.48 (d, *J=* 2.0 Hz, 1H), 7.36-7.25 (d, *J=* 8.2 Hz, 1H), 6.80 (s, 1H), 6.58-6.50 (d, *J* = 1.0 Hz, 1H), 4.54 - 4.48 (m, 2H), 4.09 - 4.01 (m, 1H), 4.00 - 3.94 (m, 3H), 3.67 - 3.62 (m, 1H), 3.52-3.40(ddd, *J* = 5.0, 8.4, 11.6 Hz, 1H), 2.50-2.40 (td, *J* = 4.8, 14.2 Hz, 1H), 2.28 (s, 3H), 2.20-2.10 (ddd, *J=* 5.6, 8.4, 13.8 Hz, 1H), 1.79 - 1.70 (m, 1H), 1.37 - 1.34 (m, 1H), 1.10-1.02 (dd, *J* = 4.2, 6.0 Hz, 1H). MS (ESI) m/z:514.2 [M+H]⁺.

### Compound 1B

TBAF (1 M, 0.035 mL) was added to 1-2B (11 mg, 17.52 µmol) in THF (2 mL), and the reaction solution was stirred at 20 °C for 0.5 hours. The reaction solution was respectively diluted with ethyl acetate (30 mL) and washed with water (20 × 2 mL); the organic phase was dried over anhydrous sodium sulfate and then concentrated; and the residue was separated by preparative silica gel plate (PE/EA= 1/2, V/V) to obtain compound 1B. ¹H NMR (400 MHz, CDCl₃) δ 8.96-8.90 (d, *J* = 5.0 Hz, 1H), 8.15 - 8.11 (m, 1H), 7.97 - 7.89 (m, 2H), 7.64-7.55 (dd, *J* = 1.6, 7.0 Hz, 1H), 7.54-7.48 (br d, *J* = 0.8 Hz, 1H), 7.38-7.26(d, *J* = 8.6 Hz, 1H), 6.84-6.78 (d, *J* = 0.8 Hz, 1H), 6.58-6.50 (d, *J* = 1.2 Hz, 1H), 4.54 - 4.47 (m, 2H), 4.07 - 4.02 (m, 1H), 4.00 - 3.93 (m, 3H), 3.68 - 3.58 (m, 1H), 3.53 - 3.44 (m, 1H), 2.50-2.40 (td, *J=* 5.3, 13.6 Hz, 1H), 2.28 (s, 3H), 2.16-2.06 (ddd, *J=* 5.6, 8.7, 14.1 Hz, 1H), 1.78 - 1.71 (m, 1H), 1.37 - 1.34 (m, 1H), 1.12 - 1.05 (m, 1H). MS (ESI) m/z: 514.2 [M+H]⁺.

### Example 2

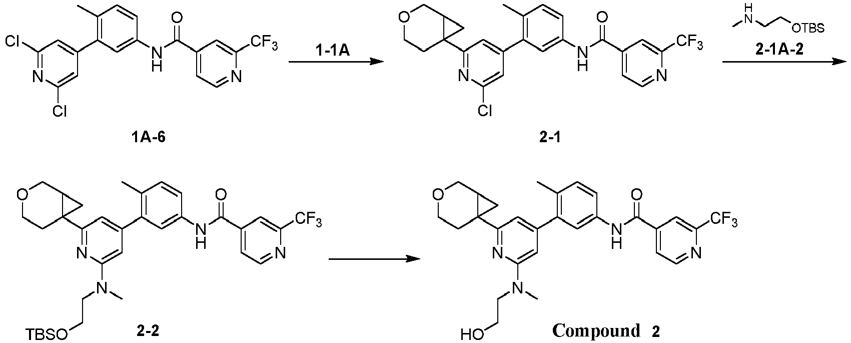

### Compound 2-1

1A-6 (1.0 g, 2.35 mmol), 1-1A (480 mg, 2.35 mmol), Pd(dppf)Cl₂ (250 mg, 341.67 µmol) and cesium carbonate (1.6 g, 4.90 mmol) were dissolved in a mixed solvent of dioxane (30 mL) and water (5 mL). Under nitrogen protection, the reaction mixture was heated to 110 °C and stirred for 16 hours. The reaction solution was diluted with ethyl acetate (30 mL) and then filtered; the filtrate was washed with water (20 × 2 mL); the organic phase was dried over anhydrous sodium sulfate and then concentrated; and the residue was separated by silica gel column chromatography (PE/EA = 20/1 to 5/1, V/V) to obtain a crude, which was then separated by chromatographic column (silica gel) (HCOOH-MeCN-H₂O) to obtain compound 2-1. ¹H NMR (400 MHz, CDCl₃) δ 8.99-8.92 (d, *J* = 5.0 Hz, 1H), 8.10 (s, 1H), 7.98-7.91 (d, *J* = 4.4 Hz, 1H), 7.89 (s, 1H), 7.60-7.52 (br d, *J* = 8.2 Hz, 1H), 7.54 (s, 1H), 7.38-7.30 (d, *J* = 8.2 Hz, 1H), 7.14-7.06 (d, *J* = 3.8 Hz, 2H), 4.09 - 4.01 (m, 1H), 3.99 - 3.92 (m, 1H), 3.69-3.58 (td, *J* = 5.4, 11.4 Hz, 1H), 3.52-3.45 (ddd, *J* = 5.4, 8.6, 11.4 Hz, 1H), 2.56-2.48 (td, *J* = 5.0, 13.8 Hz, 1H), 2.28 (s, 3H), 2.22-2.10 (ddd, *J* = 5.4, 8.6, 14.0 Hz, 1H), 1.88 - 1.79 (m, 1H), 1.45-1.36 (dd, *J* = 4.2, 9.2 Hz, 1H), 1.18-1.10 (dd, *J* = 4.2, 6.4 Hz, 1H), 0.87 - 0.82 (m, 1H).

### Compound 2-2

Pd₂dba₃ (37.54 mg, 40.99 µmol), Ruphos (38.26 mg, 81.98 µmol) and cesium carbonate (248.99 mg, 764.19 µmol) were added to a solution of 2-1 (200 mg, 409.92 µmol) and 2-1A-2 (200 mg, 1.06 mmol) in toluene (5 mL). Under nitrogen protection, the reaction mixture was heated to 120 °C and stirred for 4 hours. The reaction mixture was filtered; the filtrate was concentrated; and the residue was separated by preparative silica gel plate (PE/EA = 2/1, V/V) to obtain compound 2-2. MS (ESI) m/z: 641.3 [M+H] ⁺.

### Compound 2

TBAF (1 M, 0.25 mL) was added to 2-2 (80 mg, 124.84 µmol) in THF (5 mL), and the reaction solution was stirred at 20 °C for 0.5 hours. The reaction solution was filtered; the filtrate was washed with a citric acid solution (1 M, 20 × 2 mL) and saturated brine (15 × 1 mL) respectively; the organic phase was concentrated; and the residue was separated by preparative silica gel plate (PE/EA= 1/3, V/V) to obtain compound 2. ¹H NMR (400 MHz, DMSO*-*d₆) δ 10.67 (s, 1H), 9.07-9.96 (d, *J* = 5.0 Hz, 1H), 8.36 (s, 1H), 8.24-8.16 (d, *J* = 4.4 Hz, 1H), 7.78-7.70 (dd, *J* = 2.2, 8.4 Hz, 1H), 7.66-7.58 (d, *J* = 2.2 Hz, 1H), 7.36-7.25 (d, *J* = 8.4 Hz, 1H), 6.44 (s, 1H), 6.29 (s, 1H), 4.68 - 4.62 (m, 1H), 3.98-3.88 (dd, *J=* 4.8, 11.4 Hz, 1H), 3.85-3.74 (d, *J* = 11.4 Hz, 1H), 3.60 - 3.53 (m, 3H), 3.52 - 3.45 (m, 1H), 3.40 - 3.36 (m, 1H), 3.30 (s, 2H), 3.04 (s, 3H), 2.23 (s, 3H), 1.98 - 1.89 (m, 1H), 1.76 - 1.68 (m, 1H), 1.30-1.21 (dd, *J* = 3.2, 9.2 Hz, 1H), 0.99-0.90 (dd, *J* = 3.6, 5.8 Hz, 1H). MS (ESI) m/z: 527.2 [M+H] ⁺.

### Example 3

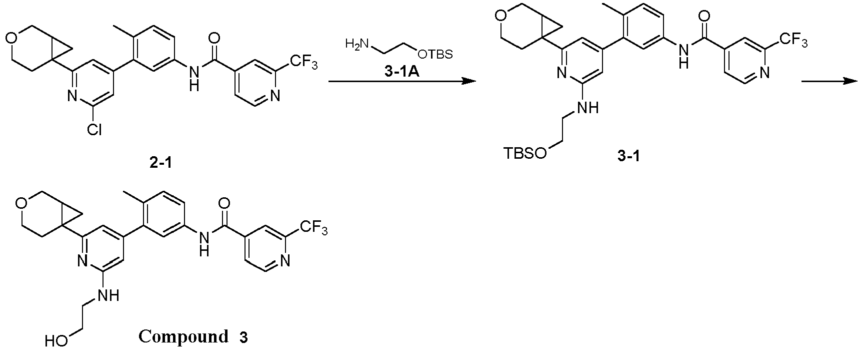

### Compound 3-1

Pd₂dba₃ (30.15 mg, 32.93 µmol), Ruphos (30.73 mg, 65.85 µmol) and cesium carbonate (200 mg, 613.84 µmol) were added to a solution of 2-1 (160 mg, 329.27 µmol) and 3-1A (150 mg, 855.46 µmol) in toluene (5 mL). Under nitrogen protection, the reaction mixture was heated to 120 °C and stirred for 4 hours. The reaction mixture was filtered; the filtrate was concentrated; and the residue was separated by preparative silica gel plate (PE/EA = 2/1, V/V) to obtain compound 3-1. MS (ESI) m/z: 627.2 [M+H] ⁺.

### Compound 3

TBAF (1 M, 0.16 mL) was added to 3-1 (50 mg, 79.77 µmol) in THF (5 mL), and the reaction solution was stirred at 20 °C for 0.5 hours. The reaction solution was filtered; the filtrate was washed with a citric acid solution (1 M, 15 × 2 mL) and saturated brine (15 × 1 mL) respectively; the organic phase was concentrated; and the residue was separated by preparative silica gel plate (PE/EA= 1/5, V/V) to obtain compound 3. ¹H NMR (400 MHz, DMSO*-*d₆) δ 10.66 (s, 1H), 9.05-9.95 (d, *J* = 5.0 Hz, 1H), 8.36 (s, 1H), 8.24-8.16 (d, *J* = 4.8 Hz, 1H), 7.80-7.68 (dd, *J* = 2.2, 8.4 Hz, 1H), 7.65-7.59 (d, *J* = 2.2 Hz, 1H), 7.36-7.27 (d, *J* = 8.4 Hz, 1H), 6.52-6.46 (t, *J* = 5.6 Hz, 1H), 6.38 (s, 1H), 6.25-6.18 (d, *J* = 0.6 Hz, 1H), 4.73-4.65 (t, *J* = 5.4 Hz, 1H), 3.98-3.90 (dd, *J* = 5.0, 11.4 Hz, 1H), 3.86-3.74 (d, *J* = 11.4 Hz, 1H), 3.58-3.50 (q, *J* = 5.8 Hz, 2H), 3.50 - 3.43 (m, 1H), 3.40 - 3.36 (m, 1H), 3.30 (s, 2H), 2.47 - 2.42 (m, 1H), 2.22 (s, 3H), 1.99-1.86 (ddd, *J* = 5.0, 8.2, 13.6 Hz, 1H), 1.74 - 1.67 (m, 1H), 1.30-1.21 (dd, *J* = 3.2, 9.2 Hz, 1H), 0.99-0.90 (dd, *J* = 3.6, 6.2 Hz, 1H). MS (ESI) m/z: 513.2 [M+H]⁺.

### Example 4

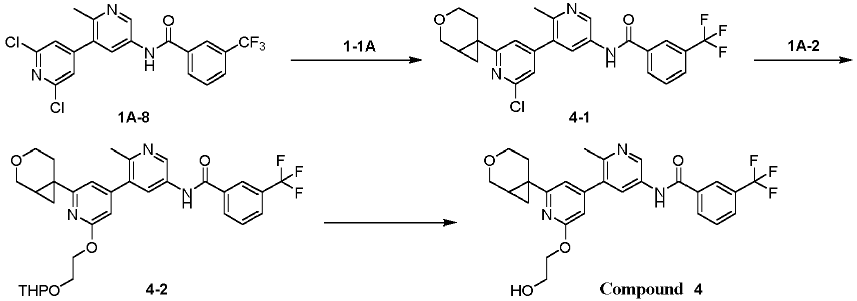

### Compound 4-1

1A-8 (460 mg, 1.08 mmol), 1-1A(405 mg, 1.19 mmol), Pd(dppf)Cl₂ (79 mg, 107.97 µmol) and cesium carbonate (706 mg, 2.17 mmol) were dissolved in a mixed solvent of DME (8 mL) and water (2 mL). Under nitrogen protection, the reaction mixture was heated to 100 °C and stirred for 24 hours. The reaction solution was diluted with ethyl acetate (20 mL) and then filtered; the filtrate was washed with water (20 × 2 mL); the organic phase was dried over anhydrous sodium sulfate and then concentrated; and the residue was separated by silica gel column chromatography (PE/EA= 5/1 to 2/1, V/V) to obtain compound 4-1. ¹HNMR (400 MHz, CDCl₃) δ 8.65 (d, *J*=2.6 Hz, 1 H), 8.20 (d, *J*=2.6 Hz, 1 H), 8.16 (s, 1 H), 8.10 (d, *J*=8.2 Hz, 1 H), 7.87 (d, *J*=7.6 Hz, 1 H), 7.94 (s, 1 H), 7.69 (s, 1 H), 7.12 (d, *J*=2.0 Hz, 2 H), 4.08-3.95 (m, 2 H), 3.69 - 3.62 (m, 1 H), 3.49 (ddd, *J*=11.6, 8. 8, 5.2 Hz, 1 H), 2.51 (s, 3 H), 2.21 - 2.12 (m, 1H), 1.90 - 1.82 (m, 1 H), 1.40 (dd, *J*=9.2, 4.0 Hz, 1 H), 1.15 (dd, *J*=4.4, 2.0 Hz, 1 H), 0.92 (dd, *J*=12.6, 5.6 Hz, 1 H).

### Compound 4-2

Pd₂dba₃ (53 mg, 57.88 µmol), Brettphos (31 mg, 57.75 µmol) and cesium carbonate (374 mg, 1.15 mmol) were added to a solution of 4-1 (280 mg, 573.89 µmol) and 1A-2 (126 mg, 861.93 µmol) in toluene (5 mL). The reaction solution was diluted with ethyl acetate (10 mL) and then filtered; the filtrate was washed with water (20 × 2 mL); the organic phase was dried over anhydrous sodium sulfate and then concentrated; and the residue was separated by silica gel column chromatography (PE/EA = 40/1 to 5/1, V/V) to obtain compound 4-2. ¹HNMR (400 MHz, CDCl₃) δ, 8.66 (d, *J*=2.6 Hz, 1 H), 8.16 (s, 2 H), 8.10 (d, *J*=2.6 Hz, 1 H), 7.91 (s, 1 H), 7.86 (br d, *J*=7.6 Hz, 1 H), 7.66 - 7.70 (m, 1 H), 6.79 (d, *J*=1.0 Hz, 1 H), 6.55 (d, *J*=1.0 Hz, 1 H), 4.72 (d, *J*=3.8 Hz, 1 H), 4.60- 4.54 (m, 2 H), 4.04 - 4.09 (m, 2 H), 3.93 - 3.97 (m, 2 H), 3.82 (s, 1 H), 3.76 (s, 1 H), 3.58 - 3.52 (m, 2 H), 2.80 (br d, *J*=6.4 Hz, 1 H), 2.51 (s, 3 H), 2.14- 2.08 (m, 1 H), 1.90 - 1.80 (m, 4 H), 1.80 - 1.74 (m, 2 H), 1.62 (br d, *J*=4.2 Hz, 1 H), 1.39 (d, *J*=3.8 Hz, 1 H), 1.07 (dd, *J*=6.2, 4.0 Hz, 1 H).

### Compound 4

HCl (2 M, 2.2 mL) was added to 4-2 (220 mg, 638.13 µmol) in DMF (2 mL), and the reaction solution was stirred at 15 °C for 0.5 hours. The reaction solution was adjusted to pH = 8 by adding a saturated NaHCO₃ solution, diluted with water (20 mL) and then extracted with ethyl acetate (20 × 2 mL); the organic phase was dried over anhydrous sodium sulfate and then concentrated; and the residue was separated by preparative silica gel plate (DCM/MeOH = 20/1, V/V) to obtain compound 4. ¹H NMR (400 MHz, MeOD) δ 8.88-8.80 (d, *J*=2.4 Hz, 1 H), 8.30 (s, 1 H), 8.26-8.19 (d, *J*=7.6 Hz, 1 H), 8.15-8.09 (d, *J*=2.4 Hz, 1 H), 7.99-7.91 (d, *J*=7.4 Hz, 1 H), 7.72 - 7.78 (m, 1 H), 6.96-6.89 (d, *J*=1.0 Hz, 1 H), 6.65-6.60 (d, *J*=1.0 Hz, 1 H), 4.40 - 4.44 (m, 2 H), 4.10-4.02 (dd, *J*=11.4, 4.6 Hz, 1 H), 3.97-3.90 (d, *J*=11.4 Hz, 1 H), 3.91 - 3.88 (m, 2 H), 3.68 - 3.60 (m, 1 H), 3.54 - 3.46 (m, 2 H), 2.60-2.51 (br d, *J*=14.0 Hz, 1 H), 2.47 (s, 3 H), 2.10 (s, 1 H), 1.88 - 1.80 (m, 1 H), 1.46-1.40 (dd, *J*=9.0, 4.2 Hz, 1 H), 1.34-1.28 (br s, 1 H), 1.10-1.01 (dd, *J*=6.4, 4.0 Hz, 1 H). MS (ESI) m/z: 514.2 [M+H]⁺.

### Example 5

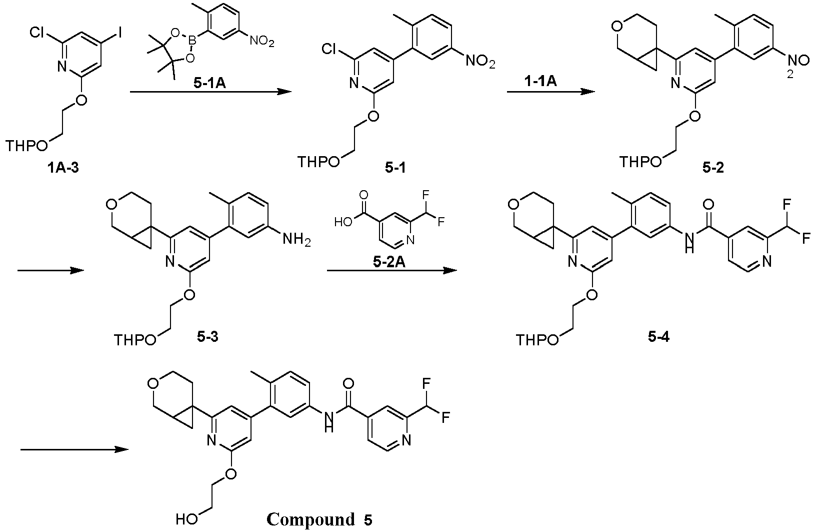

### Compound 5-1

1A-3 (2 g, 5.21 mmol), 5-1A (1.51 g, 5.74 mmol), Pd(dppf)Cl₂ (190.74 mg, 260.68 µmol) and sodium carbonate (1.38 g, 13.03 mmol) were dissolved in a mixed solvent of dioxane (20 mL) and water (4 mL). Under nitrogen protection, the reaction mixture was heated to 100 °C and stirred for 1.5 hours. The reaction solution was diluted with ethyl acetate (30 mL) and then filtered; the filtrate was washed with water (20 × 3 mL); the organic phase was dried over anhydrous sodium sulfate and then concentrated; and the residue was separated by silica gel column chromatography (PE/EA = 100/1 to 50/1, V/V) to obtain compound 5-1. ¹HNMR (400 MHz, CDCl₃) δ 8.17 (dd, *J=* 2.6 8.4 Hz, 1H), 8.07 (d, *J=* 2.4 Hz, 1H), 7.46 (d, *J* = 8.4 Hz, 1H), 6.88 (d, *J=* 1.2 Hz, 1H), 6.67 (d, *J=* 1.2 Hz, 1H), 4.74 - 4.69 (m, 1H), 4.57 (ddd, *J* = 3.4, 6.0, 9.2 Hz, 2H), 4.08 (ddd, *J=* 3.4, 5.8, 11.4 Hz, 1H), 3.96 - 3.78 (m, 2H), 3.60 - 3.50 (m, 1H), 2.38 (s, 3H), 1.94 - 1.80 (m, 1H), 1.79 - 1.70 (m, 1H), 1.69 - 1.53 (m, 4H).

### Compound 5-2

5-1 (200 mg, 509.12 µmol), 1-1A(190 mg, 558.72 µmol), Pd(dppf)Cl₂ (38 mg, 51.93 µmol) and cesium carbonate (333 mg, 1.02 mmol) were dissolved in a mixed solvent of DME (4 mL) and water (1 mL). Under nitrogen protection, the reaction mixture was heated to 100 °C and stirred for 24 hours. The reaction solution was diluted with ethyl acetate (10 mL) and then filtered; the filtrate was washed with water (10 × 2 mL); the organic phase was dried over anhydrous sodium sulfate and then concentrated; and the residue was separated by thin-layer silica gel plate (PE/EA= 3/1, V/V) to obtain compound 5-2. MS (ESI) m/z: 455.2 [M+H] ⁺.

### Compound 5-3

Pd/C (10%, 15 mg) was added to a solution of 5-2 (120 mg, 264.02 µmol) in methanol (4 mL); under hydrogen gas (15 psi), the mixture was stirred at 25 °C for 1 hour. The reaction solution was filtered and then concentrated to obtain compound 5-3 as a crude. MS (ESI): m/z 447.2 [M+Na+H]⁺.

### Compound 5-4

HATU (120 mg, 315.59 µmol) and DIPEA(92 mg, 711.83 µmol) were added to 5-2A (60 mg, 346.59 µmol) and 5-3 (120 mg, 282.66 µmol) in DCM (5 mL), and the mixture was stirred at 20 °C for 16 hours. The reaction mixture was diluted with water (20 mL) and extracted with DCM (20 × 2 mL); the organic phase was combined, then dried over anhydrous sodium sulfate and concentrated; the residue was purified by preparative silica gel plate (PE/EA = 2/1, V/V) to obtain compound 5-4. ¹HNMR (400 MHz, CDCl₃) δ, 8.89-8.80 (d, *J*=5.0 Hz, 1 H), 8.04 (s, 1 H), 7.96 (s, 1 H), 7.82-7.76 (br d, *J*=5.4 Hz, 1 H), 7.59 - 7.64 (m, 1 H), 7.50-7.42 (d, *J*=1.8 Hz, 1 H), 7.38-7.29 (d, *J*=8.2 Hz, 1 H), 6.58 - 6.88 (m, 2 H), 6.56-6.50 (d, *J*=0.8 Hz, 1 H), 4.71 (t, *J*=3.6 Hz, 1 H), 4.45 - 4.58 (m, 2 H), 4.01 - 4.15 (m, 2 H), 3.88 - 3.99 (m, 2 H), 3.82 (ddd, *J*=11.0, 6.8, 3.8 Hz, 1 H), 3.58 - 3.67 (m, 1 H), 3.43 - 3.56 (m, 2 H), 2.55 (dt, *J*=13.8, 5.2 Hz, 1 H), 2.03 - 2.14 (m, 1 H), 2.27 (s, 3 H), 1.79 - 1.90 (m, 2 H), 1.70 - 1.77 (m, 1 H), 1.60 - 1.69 (m, 2 H), 1.53 (br d, *J*=9.2 Hz, 2 H), 1.39 (dd, *J*=9.0, 3.6 Hz, 1 H), 1.05 (dd, *J*=6.2, 4.0 Hz, 1 H).

### Compound 5

HCl (4 M, 2 mL) was added to 5-4 (100 mg, 172.52 µmol) in DMF (2 mL), and the reaction solution was stirred at 15 °C for 0.5 hours. The reaction solution was adjusted to pH = 8 by adding a saturated NaHCO₃ solution, diluted with water (20 mL) and then extracted with ethyl acetate (20 × 2 mL); the organic phase was dried over anhydrous sodium sulfate and then concentrated to obtain compound 5. ¹HNMR (400 MHz, MeOD) δ 8.89-8.80 (d, *J*=5.0 Hz, 1 H), 8.18 (s, 1 H), 8.06-7.95 (d, *J*=4.6 Hz, 1 H), 7.69-7.60 (dd, *J*=8.4, 2.2 Hz, 1 H), 7.65-7.58 (d, *J*=2.2 Hz, 1 H), 7.38-7.30 (d, *J*=8.4 Hz, 1 H), 6.66 - 7.00 (m, 2 H), 6.58-6.50 (d, *J*=1.0 Hz, 1 H), 4.36 - 4.44 (m, 2 H), 4.08-4.01 (dd, *J*=11.4, 4.6 Hz, 1 H), 3.96-3.91 (d, *J*=1.2 Hz, 1 H), 3.86 - 3.91 (m, 2 H), 3.57 - 3.65 (m, 1 H), 3.52-3.46 (ddd, *J*=11.60, 8.4, 5.2 Hz, 2 H), 2.26 (s, 3 H), 2.51 - 2.62 (m, 1 H), 2.03 - 2.15 (m, 1 H), 1.76 - 1.90 (m, 1 H), 1.45-1.38 (dd, *J*=9.2, 3.8 Hz, 1 H), 1.10-1.01 (dd, *J*=6.2, 4.0 Hz, 1 H). MS (ESI) m/z: 496.1 [M+H]⁺.

### Example 6

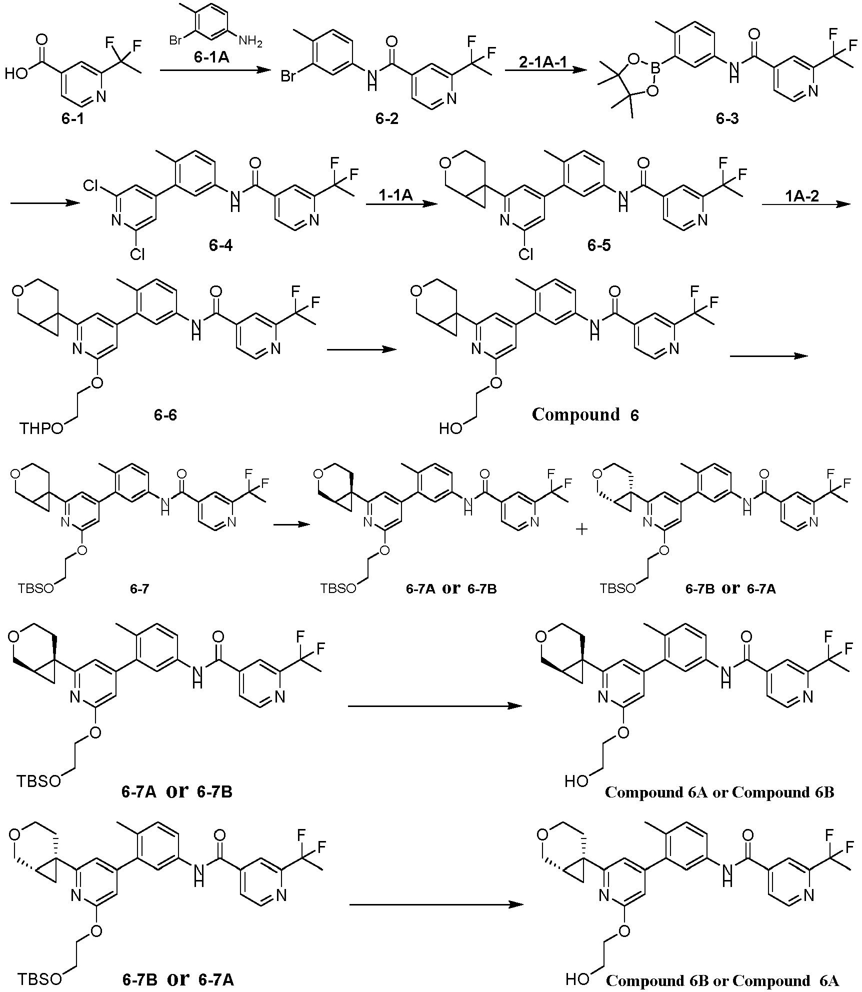

### Compound 6-2

HATU (610 mg, 1.60 mmol) and DIPEA(208 mg, 1.61 mmol) were added to 6-1 (250 mg, 1.34 mmol) and 6-1A(274 mg, 1.47 mmol) in DCM (5 mL); and the mixture was stirred at 20 °C for 2 hours. The reaction mixture was diluted with water (20 mL) and extracted with DCM (15 × 2 mL); the organic phase was combined, then dried over anhydrous sodium sulfate and concentrated; the residue was purified by preparative silica gel plate (PE/EA = 2/1, V/V) to obtain compound 6-2. ¹HNMR (400 MHz, CDCl₃) δ 8.83 (d, *J* = 5.0 Hz, 1H), 8.01 (d, *J* = 0.8 Hz, 1H), 7.91 (d, *J=* 2.0 Hz, 1H), 7.87 - 7.79 (m, 2H), 7.49 (dd, *J=* 2.0, 8.2 Hz, 1H), 7.25 (d, *J=* 8.2 Hz, 1H), 2.40 (s, 3H), 2.07 (t, *J* = 18.8 Hz, 3H).

### Compound 6-3

6-2 (370 mg, 1.04 mmol), 2-1A-1 (317 mg, 1.25 mmol), Pd(dppf)Cl₂ (39 mg, 53.30 µmol) and potassium acetate (205 mg, 2.09 mmol) were dissolved in DME (6 mL). Under nitrogen protection, the reaction mixture was heated to 90 °C and stirred for 12 hours. The reaction mixture was diluted with ethyl acetate (20 mL); the organic phase was washed with water (20 × 1 mL) and saturated brine (20 × 1 mL) and concentrated to obtain compound 6-3. MS (ESI): m/z 403.2 [M+H]⁺.

### Compound 6-4

6-3 (600 mg, 1.49 mmol), 1-1A (450 mg, 1.64 mmol), Pd(dppf)Cl₂ (55 mg, 75.17 µmol) and sodium carbonate (317 mg, 2.99 mmol) were dissolved in DME (8 mL) and H₂O (2 mL). Under nitrogen protection, the reaction mixture was heated to 85 °C and stirred for 2 hours. The reaction mixture was diluted with ethyl acetate (50 mL); the organic phase was washed with water (30 × 1 mL) and saturated brine (30 × 1 mL) and concentrated to obtain compound 6-4. ¹HNMR (400 MHz, CDCl₃) δ 8.86-8.81 (d, *J=* 5.0 Hz, 1H), 8.13 - 7.99 (m, 2H), 7.87-7.79 (d, *J=* 5.0 Hz, 1H), 7.65 - 7.42 (m, 3H), 7.41-7.38 (d, *J=* 8.2 Hz, 1H), 2.29 (s, 3H), 2.07 - 1.98 (m, 3H).

### Compound 6-5

6-4 (360 mg, 852.57 µmol), 1-1A(319 mg, 938.06 µmol), Pd(dppf)Cl₂ (63 mg, 86.10 µmol) and cesium carbonate (556 mg, 1.71 mmol) were dissolved in a mixed solvent of DME (6 mL) and water (1 mL). Under nitrogen protection, the reaction mixture was heated to 100 °C and stirred for 14 hours. The reaction solution was diluted with ethyl acetate (50 mL) and then filtered; the filtrate was washed with water (30 × 1 mL); the organic phase was dried over anhydrous sodium sulfate and then concentrated; and the residue was separated by thin-layer silica gel plate (PE/EA = 1/1, V/V) to obtain compound 6-5. ¹H NMR (400 MHz, CDCl₃) δ 8.84 (d, *J* = 5.0 Hz, 1H), 8.03 (s, 1H), 7.92 (s, 1H), 7.83 (d, *J* = 4.4 Hz, 1H), 7.62 - 7.53 (m, 2H), 7.33 (d, *J* = 8.2 Hz, 1H), 7.09 (d, *J* = 3.6 Hz, 2H), 4.08 - 4.00 (m, 1H), 4.00 - 3.89 (m, 1H), 3.70 - 3.61 (m, 1H), 3.48 (ddd, *J* = 5.2, 8.8, 11.8 Hz, 1H), 2.52 (td, *J* = 4.8, 13.8 Hz, 1H), 2.27 (s, 3H), 2.18 - 2.13 (m, 1H), 2.07 - 2.02 (m, 3H), 1.87 - 1.79 (m, 1H), 1.38 (dd, *J* = 4.2, 9.2 Hz, 1H), 1.13 (dd, *J* = 4.2, 6.4 Hz, 1H).

### Compound 6-6

Pd₂dba₃ (31 mg, 33.85 µmol), Brettphos (36 mg, 67.07 µmol) and cesium carbonate (216 mg, 662.94 µmol) were added to a solution of 6-5 (160 mg, 330.62 µmol) and 1A-2 (73 mg, 499.37 µmol) in toluene (4 mL). Under nitrogen protection, the reaction mixture was heated to 110 °C and stirred for 2 hours. The reaction solution was diluted with ethyl acetate (30 mL) and then filtered; the filtrate was washed with water (20 × 1 mL); the organic phase was dried over anhydrous sodium sulfate and then concentrated; and the residue was separated by thin-layer silica gel plate (PE/EA= 2/1, V/V) to obtain compound 6-6. ¹HNMR (400 MHz, CDCl₃) δ 8.83 (d, *J* = 4.6 Hz, 1H), 8.03 (s, 1H), 7.93 (s, 1H), 7.83 (d, *J* = 4.6 Hz, 1H), 7.62 (dd, *J* = 2.0, 8.2 Hz, 1H), 7.49 - 7.45 (m, 1H), 7.30 (d, *J* = 8.4 Hz, 1H), 6.76 (d, *J* = 1.0 Hz, 1H), 6.51 (d, *J* = 1.0 Hz, 1H), 4.75 - 4.68 (m, 1H), 4.59 - 4.47 (m, 2H), 4.09 - 4.01 (m, 2H), 3.99 - 3.88 (m, 2H), 3.82 (ddd, *J* = 4.0, 6.6, 11.0 Hz, 1H), 3.65 - 3.59 (m, 1H), 3.57 - 3.44 (m, 2H), 2.55 (td, *J* = 5.0, 14.2 Hz, 1H), 2.27 (s, 3H), 2.07 - 2.02 (m, 3H), 1.89 - 1.71 (m, 3H), 1.68 - 1.60 (m, 2H), 1.58 - 1.47 (m, 3H), 1.39 (dd, *J* = 4.0, 9.0 Hz, 1H), 1.05 (dd, *J* = 4.0, 6.2 Hz, 1H).

### Compound 6

HCl (4 M, 0.5 mL) was added to 6-6 (150 mg, 252.67 µmol) in DMF (2 mL); and the reaction solution was stirred at 20 °C for 2 hours. The reaction solution was adjusted to pH = 8 by adding a saturated NaHCO₃ solution and extracted with ethyl acetate (20 × 1 mL); the organic phase was concentrated; and the residue was separated by thin-layer silica gel plate (PE/EA = 1/2, V/V) to obtain compound 6. ¹HNMR (400 MHz, MeOD) δ 8.85 - 8.79 (d, *J* = 5.0 Hz, 1H), 8.17 (s, 1H), 7.98 - 7.90 (d, *J* = 4.6 Hz, 1H), 7.69 - 7.60 (dd, *J* = 2.4, 8.2 Hz, 1H), 7.60 - 7.54 (d, *J* = 2.2 Hz, 1H), 7.35 - 7.28 (d, *J* = 8.2 Hz, 1H), 6.89 - 6.84 (m, 1H), 6.58 - 6.51 (d, *J* = 1.0 Hz, 1H), 4.44 - 4.35 (m, 2H), 4.08 - 3.99 (dd, *J* = 4.6, 11.2 Hz, 1H), 3.95 - 3.85 (m, 3H), 3.65 - 3.57 (m, 1H), 3.54 - 3.44 (m, 1H), 2.61 - 2.51 (m, 1H), 2.25 (s, 3H), 2.09 - 1.97 (m, 4H), 1.84 - 1.76 (m, 1H), 1.40 - 1.32 (dd, *J* = 3.4, 9.2 Hz, 1H), 1.06-1.01 (dd, *J* = 3.8, 6.2 Hz, 1H). MS (ESI) m/z: 510.3 [M+H] ⁺.

### Compound 6-7

TBSCl (67 mg, 444.53 µmol) was added to a solution of compound 6 (150 mg, 294.38 µmol) and imidazole (41 mg, 602.23 µmol) in DMF (3 mL). The reaction mixture was stirred at 35 °C for 4 hours. The reaction mixture was diluted with ethyl acetate (20 mL) and washed with water (15 × 1 mL) and saturated brine (15 × 3 mL); the organic phase was concentrated; and the residue was separated by preparative silica gel plate (PE/EA = 3/1, V/V) to obtain compound 6-7. ¹HNMR (400 MHz, CDCl₃) δ 8.83 (d, *J* = 5.0 Hz, 1H), 8.03 (s, 1H), 7.90 (s, 1H),7.83 (d, *J* = 5.0 Hz, 1H), 7.62 (dd, *J* = 2.0, 8.0 Hz, 1H), 7.46 (d, *J* = 2.0 Hz, 1H), 7.31 (d, *J* = 8.0 Hz, 1H), 6.76 (s, 1H), 6.48 (s,1H), 4.41 (t, *J* = 5.4 Hz, 2H), 4.08 - 4.02 (m, 1H), 3.99 - 3.97 (m, 2H), 3.78 - 3.74 (m, 1H), 3.62 (td, *J* = 5.4, 11.2 Hz, 1H), 3.48(ddd, *J* = 5.2, 8.4, 11.6 Hz, 1H), 2.55 (td, *J* = 5.2, 14.0 Hz, 1H), 2.27 (s, 3H), 2.08 - 2.02 (m, 3H), 1.89 - 1.82 (m, 2H), 1.38 (dd, *J* = 3.8, 9.0 Hz, 1H), 1.05 (dd, *J* = 3.8, 6.0 Hz, 1H), 0.91 (s, 9H), 0.09 (s, 6H).

### Compound 6-7A and Compound 6-7B

Compound 6-7 was subjected to SFC chiral separation (chiral column: REGIS (R,R)WHELK-O1 (250 mm^{∗}25 mm, 10 µm), mobile phase A: isopropanol (containing 0.05% DIEA); mobile phase B: carbon dioxide) to obtain compound 6-7A (retention time: 2.263 min) and compound 6-7B (retention time: 2.325 min).

### Compound 6A

HCl (3 M, 0.78 mL) was added to 6-7A(55 mg, 88.17 µmol) in THF (2 mL); and the reaction solution was stirred at 25 °C for 1 hour. The reaction solution was neutralized with saturated NaHCO₃ and then extracted with ethyl acetate (15 × 2 mL); the organic phase was washed with saturated brine (30 × 1 mL) and concentrated; and the residue was separated by preparative silica gel plate (PE/EA = 1/1, V/V) to obtain compound 6A. ¹HNMR (400 MHz, MeOD) δ 8.85 - 8.79 (d, *J* = 5.0 Hz, 1H), 8.17 (s, 1H), 7.98 - 7.90 (d, *J* = 4.6 Hz, 1H), 7.69 - 7.60 (dd, *J* = 2.4, 8.2 Hz, 1H), 7.60 - 7.54 (d, *J* = 2.2 Hz, 1H), 7.35 - 7.28 (d, *J* = 8.2 Hz, 1H), 6.89 - 6.84 (m, 1H), 6.58 - 6.51 (d, *J* = 1.0 Hz, 1H), 4.44 - 4.35 (m, 2H), 4.08 - 3.99 (dd, *J* = 4.6, 11.2 Hz, 1H), 3.95 - 3.85 (m, 3H), 3.65 - 3.57 (m, 1H), 3.54 - 3.44 (m, 1H), 2.61 - 2.51 (m, 1H), 2.25 (s, 3H), 2.09 - 1.97 (m, 4H), 1.84 - 1.76 (m, 1H), 1.40 - 1.32 (dd, *J* = 3.4, 9.2 Hz, 1H), 1.06-1.01 (dd, *J* = 3.8, 6.2 Hz, 1H). MS (ESI) m/z: 510.3 [M+H]⁺, 100% (ee%).

### Compound 6B

HCl (3 M, 0.74 mL) was added to 6-7B (52 mg, 83.36 µmol) in THF (2 mL); and the reaction solution was stirred at 25 °C for 1 hour. The reaction solution was neutralized with saturated NaHCO3 and then extracted with ethyl acetate (10 × 2 mL); the organic phase was washed with saturated brine (20 × 1 mL) and concentrated; and the residue was separated by preparative silica gel plate (PE/EA= 1/1, V/V) to obtain compound 6B. ¹HNMR (400 MHz, MeOD) δ 8.85 - 8.79 (d, *J* = 5.0 Hz, 1H), 8.17 (s, 1H), 7.98 - 7.90 (d, *J* = 4.6 Hz, 1H), 7.69 - 7.60 (dd, *J* = 2.4, 8.2 Hz, 1H), 7.60 - 7.54 (d, *J* = 2.2 Hz, 1H), 7.35 - 7.28 (d, *J* = 8.2 Hz, 1H), 6.89 - 6.84 (m, 1H), 6.58 - 6.51 (d, *J* = 1.0 Hz, 1H), 4.44 - 4.35 (m, 2H), 4.08 - 3.99 (dd, *J* = 4.6, 11.2 Hz, 1H), 3.95 - 3.85 (m, 3H), 3.65 - 3.57 (m, 1H), 3.54 - 3.44 (m, 1H), 2.61 - 2.51 (m, 1H), 2.25 (s, 3H), 2.09 - 1.97 (m, 4H), 1.84 - 1.76 (m, 1H), 1.40 - 1.32 (dd, *J* = 3.4, 9.2 Hz, 1H), 1.06-1.01 (dd, *J* = 3.8, 6.2 Hz, 1H). MS (ESI) m/z: 510.3 [M+H]⁺, 100% (ee%).

### Example 7

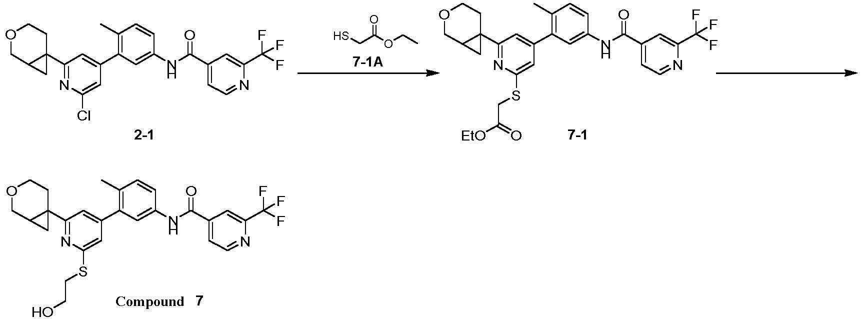

### Compound 7-1

Pd₂dba₃ (16 mg, 17.47 µmol), Brettphos (10 mg, 17.28 µmol) and DIPEA (44 mg, 340.45 µmol) were added to a solution of 2-1 (80 mg, 163.97 µmol) and 7-1A(25 mg, 208.04 µmol) in dioxane (2 mL). Under nitrogen protection, the reaction mixture was heated to 100 °C and stirred for 12 hours. The reaction solution was concentrated; and the residue was separated by thin-layer silica gel plate (PE/EA= 1/1, V/V) to obtain compound 7-1. MS (ESI) m/z: 572.3 [M+H] ⁺.

### Compound 7

Lithium aluminium hydride (9 mg, 237.13 µmol) was added to 7-1 (85 mg, 148.70 µmol) in THF (3 mL), and the reaction solution was stirred at 0 °C for 15 minutes. At 0 °C, water (10 mL) was added to the reaction solution, and the mixture was extracted with ethyl acetate (15 × 2 mL); the organic phase was dried over anhydrous sodium sulfate and then concentrated; and the residue was separated by column chromatography (HCOOH-MeCN-H₂O) to obtain compound 7. ¹HNMR (400 MHz, CDCl₃) δ 8.94 - 8.90 (br d, *J* = 4.8 Hz, 1H), 8.25 (br s, 1H), 8.13 (s, 1H), 7.98 -7.91 (br d, *J* = 4.0 Hz, 1H), 7.64 - 7.60 (br d, *J* = 7.8 Hz, 1H), 7.48 (br s, 1H), 7.34 - 7.27 (br d, *J=* 8.2 Hz, 1H), 7.04 (s, 1H), 6.91 (s, 1H), 4.58 - 4.28 (m, 1H), 4.11 - 3.88 (m, 4H), 3.71 - 3.57 (m, 1H), 3.55 - 3.30 (m, 3H), 2.44 - 2.34 (m, 1H), 2.26 (s, 3H), 2.19 - 2.06 (m, 1H), 1.74-1.69 (br d, *J* = 4.4 Hz, 1H), 1.37 - 1.24 (m, 1H), 1.10 -1.01 (br t, *J* = 5.0 Hz, 1H). MS (ESI) m/z: 530.0 [M+H] ⁺.

### Example 8

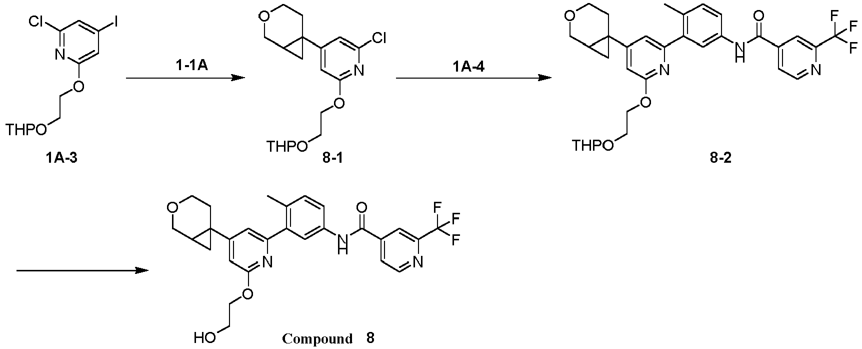

### Compound 8-1

1A-3 (500 mg, 1.30 mmol), 1-1A(450 mg, 1.32 mmol), Pd(dppf)Cl₂ (200 mg, 273.33 µmol) and cesium carbonate (860 mg, 2.64 mmol) were dissolved in DME (12 mL) and H₂O (2 mL). Under nitrogen protection, the reaction mixture was heated to 100 °C and stirred for 12 hours. The reaction mixture was diluted with ethyl acetate (10 mL) and water (15 × 1 mL), and extracted with ethyl acetate (20 × 3 mL); the organic phase was dried over anhydrous sodium sulfate and then concentrated; and the residue was separated by column chromatography (silica gel) (HCOOH-MeCN-H₂O) to obtain compound 8-1. ¹HNMR (400 MHz, CDCl₃) δ 6.68 (d, *J*=1.2 Hz, 1H), 6.47 (d, *J*=1.2 Hz, 1H), 4.62 (t, *J*=3.6 Hz, 1H), 4.40 (ddq, *J*=3.4, 6.2, 11.6 Hz, 2H), 3.99 - 3.78 (m, 5H), 3.71 (ddd, *J*=3.4, 6.2, 11.6 Hz, 1H), 3.54 (td, *J*=5.2, 11.6 Hz, 1H), 3.45 (td, *J*=5.2, 11.0 Hz, 1H), 3.33 (ddd, *J*=5.8, 8.4, 11.6 Hz, 1H), 2.05 - 1.97 (m, 2H), 1.82 - 1.61 (m, 2H), 1.60 - 1.50 (m, 2H), 1.45 (br d, *J*=5.4 Hz, 1H), 1.32 - 1.23 (m, 1H), 1.04 - 0.92 (m, 2H).

### Compound 8-2

8-1 (50 mg, 141.31 µmol), 1A-4 (80 mg, 196.94 µmol), Pd(dppf)Cl₂ (15 mg, 20.50 µmol) and sodium carbonate (30 mg, 283.05 µmol) were dissolved in a mixed solvent of dioxane (2 mL) and water (0.4 mL). Under nitrogen protection, the reaction mixture was heated to 100 °C and stirred for 4 hours. The reaction solution was diluted with ethyl acetate (10 mL) and water (15 mL) and then filtered; the filtrate was extracted with ethyl acetate (10 × 3 mL); the organic phase was dried over anhydrous sodium sulfate and then concentrated; and the residue was separated by thin-layer silica gel plate (PE/EA = 1/1, V/V) to obtain compound 8-2. MS (ESI) m/z: 598.4 [M+H]⁺.

### Compound 8

HCl (4 M, 1 mL) was added to 8-2 (50 mg, 83.66 µmol) in DMF (2 mL); and the reaction solution was stirred at 15 °C for 0.5 hours. The reaction solution was diluted with ethyl acetate (10 mL) and water (15 mL), then adjusted to pH = 8 with a saturated NaHCO₃ solution and extracted with ethyl acetate (10 × 3 mL); the organic phase was dried over anhydrous sodium sulfate and then concentrated; and the residue was separated by thin-layer silica gel plate (PE/EA = 1/1, V/V) to obtain compound 8. ¹HNMR (400 MHz, MeOD) δ 8.97-8.92 (d, *J*=5.0 Hz, 1H), 8.33 (s, 1H), 8.18-8.11 (d, *J*=4.0 Hz, 1H), 7.83 - 7.75 (m, 1H), 7.73-7.68 (dd, *J*=2.2, 8.2 Hz, 1H), 7.36-7.29 (d, *J*=8.2 Hz, 1H), 7.05-6.95 (d, *J*=1.4 Hz, 1H), 6.75-6.70 (d, *J*=1.4 Hz, 1H), 4.43 - 4.38 (m, 2H), 4.10-4.02 (dd, *J*=4.6, 11.4 Hz, 1H), 3.97-3.90 (d, *J*=11.4 Hz, 1H), 3.91 - 3.86 (m, 2H), 3.67 - 3.59 (m, 1H), 3.56-3.49 (ddd, *J*=5.6, 8.2, 11.6 Hz, 1H), 2.39 (s, 3H), 2.25 - 2.14 (m, 2H), 1.56-1.51 (td, *J*=4.6, 9.2 Hz, 1H), 1.22 - 1.15 (m, 1H), 1.10 - 1.05 (m, 1H), 1.10 - 1.05 (m, 1H). MS (ESI) m/z: 514.3 [M+H]⁺.

### Example 9

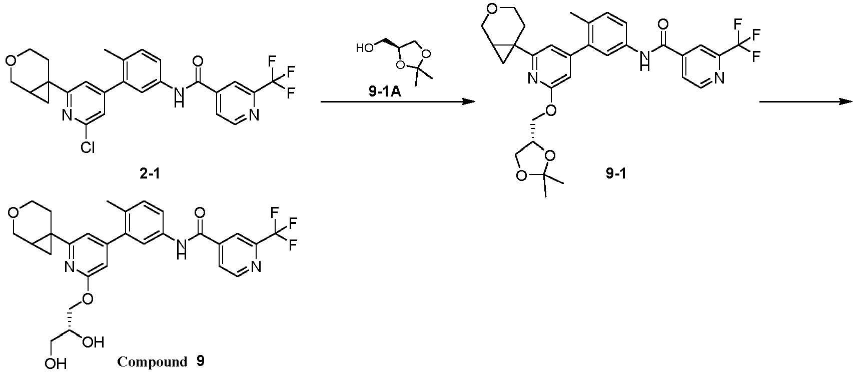

### Compound 9-1

Pd₂dba₃ (28 mg, 30.58 µmol), Brettphos (33 mg, 61.48 µmol) and cesium carbonate (201 mg, 616.91 µmol) were added to a solution of 2-1 (150 mg, 307.44 µmol) and 9-1A(82 mg, 620.47 µmol) in toluene (4 mL). Under nitrogen protection, the reaction mixture was heated to 110 °C and stirred for 4 hours. The reaction solution was diluted with ethyl acetate (20 mL) and washed with water (20 × 1 mL); the organic phase was dried over anhydrous sodium sulfate and then concentrated; and the residue was separated by thin-layer silica gel plate (PE/EA = 2/1, V/V) to obtain compound 9-1. ¹HNMR (400 MHz, CDCl₃) δ 8.93 (d, *J* = 5.0 Hz, 1H), 8.11 (s, 1H), 7.94 - 7.85 (m, 2H), 7.59 (dd, *J* = 2.0, 8.4 Hz, 1H), 7.47 (d, *J* = 1.8 Hz, 1H), 7.31 (d, *J* = 8.4 Hz, 1H), 6.78 (s, 1H), 6.52 (d, *J* = 1.0 Hz, 1H), 4.56 - 4.47 (m, 1H), 4.45 - 4.32 (m, 2H), 4.20 - 4.16 (m, 1H), 4.08 - 4.02 (m, 1H), 3.99 - 3.93 (m, 1H), 3.89 (ddd, *J* = 1.2, 6.2, 8.4 Hz, 1H), 3.62 (td, *J* = 5.4, 11.2 Hz, 1H), 3.48 (ddd, *J* = 5.2, 8.4, 11.6 Hz, 1H), 2.54 (td, *J=* 5.2, 12.4 Hz, 1H), 2.27 (s, 3H), 2.15 - 2.06 (m, 1H), 1.87 - 1.77 (m, 1H), 1.49 (s, 3H), 1.42 (s, 3H), 1.40 - 1.35 (m, 1H), 1.07 (dd, *J=* 3.8, 6.2 Hz, 1H).

### Compound 9

HCl (3 M, 0.2 mL) was added to 9-1 (140 mg, 239.89 µmol) in methanol (3 mL), and the reaction solution was stirred at 30 °C for 1 hour. The reaction solution was diluted with water (20 mL), then adjusted to pH=8 with a saturated NaHCO3 solution and extracted with ethyl acetate (20 × 1 mL); the organic phase was concentrated; and the residue was separated by thin-layer silica gel plate (PE/EA = 1/1, V/V) to obtain compound 9. ¹HNMR (400 MHz, MeOD) δ 8.96-8.89 (d, *J* = 5.0 Hz, 1H), 8.31 (s, 1H), 8.16-8.11 (dd, *J* = 1.4, 5.0 Hz, 1H), 7.71-7.65 (dd, *J* = 2.4, 8.2 Hz, 1H), 7.65-7.59 (d, *J* = 2.4 Hz, 1H), 7.36-7.30 (d, *J* = 8.2 Hz, 1H), 6.91-6.87 (d, *J* = 1.0 Hz, 1H), 6.60-6.54 (d, *J* = 1.0 Hz, 1H), 4.49 - 4.40 (m, 1H), 4.38 - 4.30 (m, 1H), 4.10 - 3.99 (m, 2H), 3.96-3.90 (dd, *J*= 1.2, 11.2 Hz, 1H), 3.69 - 3.56 (m, 3H), 3.54-3.38 (ddd, *J* = 5.2, 8.4, 11.6 Hz, 1H), 2.60-2.53 (td, *J* = 5.2, 14.0 Hz, 1H), 2.27 (s, 3H), 2.15 - 2.04 (m, 1H), 1.90 - 1.76 (m, 1H), 1.48 - 1.36 (m, 1H), 1.09-1.01 (dd, *J* = 4.0, 6.4 Hz, 1H). MS (ESI) m/z: 544.2 [M+H] ⁺.

### Example 10

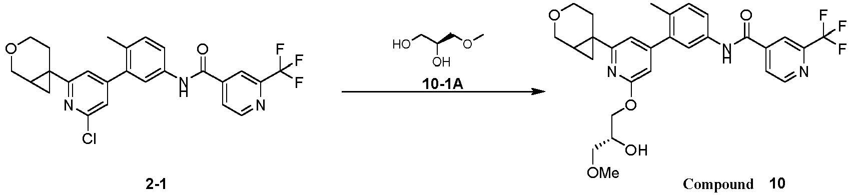

### Compound 10

Pd₂dba₃ (27 mg, 29.48 µmol), Brettphos (31 mg, 57.75 µmol) and cesium carbonate (187 mg, 573.94 µmol) were added to a solution of 2-1 (140 mg, 286.94 µmol) and 10-1A (46 mg, 433.47 µmol) in toluene (4 mL). Under nitrogen protection, the reaction mixture was heated to 110 °C and stirred for 4 hours. The reaction solution was diluted with ethyl acetate (20 mL) and then washed with water (20 × 1 mL); the organic phase was dried over anhydrous sodium sulfate and then concentrated; and the residue was separated by thin-layer silica gel plate (PE/EA= 2/1, V/V) to obtain compound 10. ¹HNMR (400 MHz, DMSO-d₆) δ 10.69 (s, 1H), 9.01-8.97 (d, *J=* 5.0 Hz, 1H), 8.36 (s, 1H), 8.23-8.18 (dd, *J=* 1.0, 5.0 Hz, 1H), 7.77-7.73 (dd, *J* = 2.4, 8.2 Hz, 1H), 7.69-7.65 (d, *J* = 2.2 Hz, 1H), 7.37-7.32 (d, *J* = 8.6 Hz, 1H), 6.89-6.85 (d, *J* = 1.0 Hz, 1H), 6.55-6.51 (d, *J* = 1.0 Hz, 1H), 5.08-5.01 (d, *J=* 5.4 Hz, 1H), 4.32 - 4.23 (m, 1H), 4.22 - 4.14 (m, 1H), 4.00 - 3.92 (m, 2H), 3.83-3.77 (dd, *J=* 1.2, 11.4 Hz, 1H), 3.54 - 3.46 (m, 1H), 3.45 - 3.35 (m, 3H), 3.28 (s, 3H), 2.55-2.51 (br d, *J=* 2.0 Hz, 1H), 2.24 (s, 3H), 2.06 - 1.93 (m, 1H), 1.84 - 1.73 (m, 1H), 1.36-1.30 (td, *J=* 3.0, 9.0 Hz, 1H), 1.05-0.98 (dd, *J=* 3.6, 6.2 Hz, 1H). MS (ESI) m/z: 558.0 [M+H] ⁺.

### Example 11

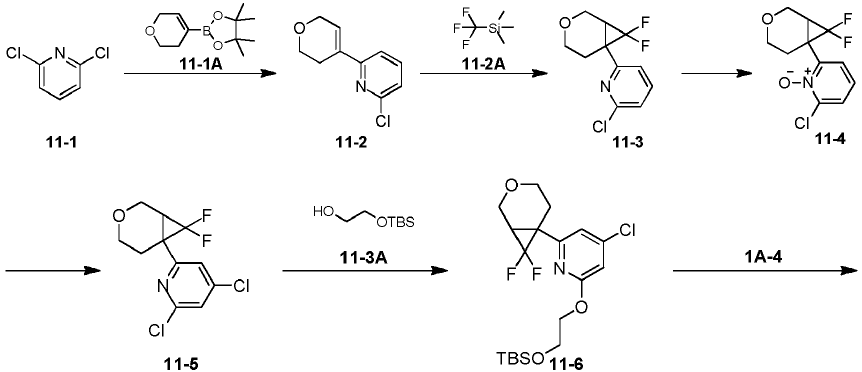

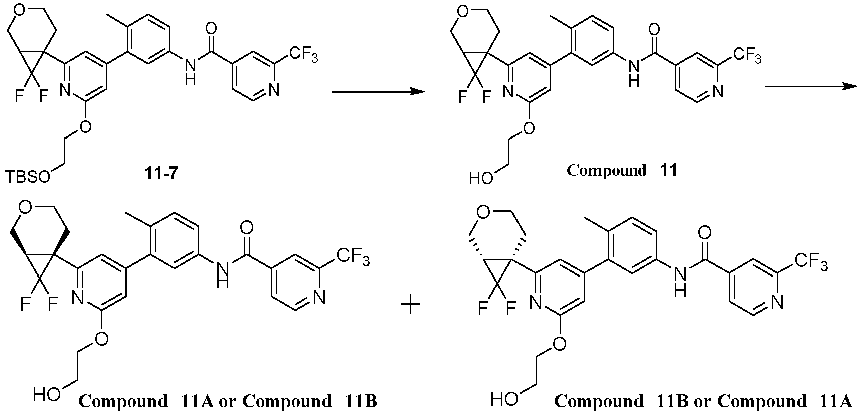

### Compound 11-2

11-1 (7.5 g, 50.68 mmol), 11-1A(10.1 g, 48.15 mmol), Pd(dppf)Cl₂ (4.1 g, 5.07 mmol) and sodium carbonate (10.7 g, 101.36 mmol) were dissolved in a mixed solvent of DME (225 mL) and water (50 mL). Under nitrogen protection, the reaction mixture was heated to 90°C and stirred for 12 hours. The reaction solution was concentrated; and the residue was separated by silica gel column chromatography (PE/EA= 1/0 to 0/1, V/V) to obtain compound 11-2. ¹HNMR (400 MHz, CDCl₃) δ 7.56-7.52 (m, 1H), 7.20-7.18 (m, 1H), 7.12-7.03 (m, 1H), 6.72-6.70 (m, 1H), 4.31 - 4.29 (m, 2H), 3.87 - 3.85 (m, 2H), 2.54 - 2.51 (m,2H).

### Compound 11-3

11-2 (1.8 g, 9.20 mmol), 11-2A(4.6 g, 32.11 mmol) and sodium iodide (450 mg, 3.00 mmol) were dissolved in THF (42 mL) solvent. Under nitrogen protection, the reaction mixture was heated to 80 °C and stirred for 12 hours. Dichloromethane (20 mL) and water (20 mL) were added to the reaction solution, and the mixture was subjected to liquid separation; the organic phase was dried over anhydrous sodium sulfate and then concentrated to obtain compound 11-3. MS (ESI) m/z:246.2[M+H]⁺.

### Compound 11-4

To 11-3 (2 g, 8.14 mmol) in dichloromethane (20 mL) solvent, m-chloroperoxybenzoic acid (4.1 g, 20.27 mmol, 85%) was added. The reaction mixture was heated to 65 °C and stirred for 12 hours. Sodium thiosulfate (20 g), water (200 mL) and dichloromethane (200 mL) were added to the reaction solution, and the mixture was stirred for 30 minutes. Sodium carbonate (15 g) was added, and the mixture was stirred for 20 minutes and subjected to liquid separation; the organic phase was dried over anhydrous sodium sulfate and then concentrated; and the residue was separated by silica gel column chromatography (DCM/MeOH = 1/0 to 1/1, V/V) to obtain compound 11-4. ¹HNMR (400MHz, CDCl₃) δ 7.51-7.49 (m, 1H), 7.35-7.33 (m, 1H), 7.20-7.16 (m, 1H), 4.23 - 4.21 (m, 1H), 4.13 - 4.08 (m, 1H), 3.82 - 3.80 (m, 2H), 2.30 - 2.25 (m, 1H), 1.77 - 1.67 (m, 2H).

### Compound 11-5

11-4 (100 mg, 382.19 µmol) was dissolved in phosphorus oxychloride (2 mL), and the mixture was heated to 90 °C and stirred for 12 hours. A saturated sodium bicarbonate solution (20 mL) was added to the reaction solution, and the mixture was extracted with ethyl acetate (20 × 1 mL); the organic phase was dried over anhydrous sodium sulfate and then concentrated to obtain compound 11-5. MS (ESI) m/z: 280.1 [M+H] ⁺.

### Compound 11-6

Potassium tert-butoxide (1.8 mL, 1 M) was added to 11-5 (100 mg, 357.02 µmol) and 11-3A (125.9 mg, 714.04 µmol) in THF (1 mL) solvent. The reaction mixture was stirred at 25 °C for 3 hours. Water (10 mL) and ethyl acetate (10 mL) were added to the reaction solution, and the mixture was subjected to liquid separation; the organic phase was dried over anhydrous sodium sulfate and then concentrated; and the residue was separated by thin-layer silica gel plate (PE/EA = 5/1, V/V) to obtain compound 11-6. ¹HNMR (400MHz, CDCl₃) δ 6.89 (d, *J*=1.4 Hz, 1H), 6.67 (d, *J*=1.4 Hz, 1H), 4.38 (t, *J*=5.2 Hz, 2H), 4.18 - 4.01 (m, 2H), 3.94 (t, *J*=5.2 Hz, 2H), 3.67 - 3.47 (m, 2H), 2.57 - 2.48 (m, 1H), 2.41 - 2.27 (m, 1H), 2.21 - 2.09 (m, 1H), 0.93 - 0.88 (m, 1H), 0.91 (s, 8H), 0.15 - 0.05 (m, 6H).

### Compound 11-7

11-6 (100 mg, 238.12 µmol), 1A-4 (116 mg, 285.74 µmol), Pd(dppf)Cl2 (38.9 mg, 47.62 µmol) and sodium carbonate (50.5 mg, 476.23 µmol) were dissolved in a mixed solvent of dioxane (1 mL) and water (0.2 mL). Under nitrogen protection, the reaction mixture was heated to 90 °C and stirred for 3 hours. The reaction solution was concentrated; and the residue was separated by thin-layer silica gel plate (PE/EA = 3/1, V/V) and then separated by chromatographic column (silica gel) (NH4HCO3-MeCN-H2O) to obtain compound 11-7. ¹HNMR (400MHz, CDCl₃) δ 8.94 (d, *J*=4.8 Hz, 1H), 8.11 (s, 1H), 7.93 (d, *J*=4.6 Hz, 1H), 7.83 (s, 1H), 7.63 (br d, *J*=8.6 Hz, 1H), 7.47 (s, 1H), 7.33 (d, *J*=8.4 Hz, 1H), 6.84 (s, 1H), 6.62 (d, *J*=1.0 Hz, 1H), 4.45 (t, *J*=5.2 Hz, 2H), 4.20 - 4.04 (m, 2H), 3.99 (t, *J*=5.2 Hz, 2H), 3.68 - 3.49 (m, 2H), 2.59 (ddd, *J*=3.2, 6.4, 9.8 Hz, 1H), 2.43 - 2.32 (m, 1H), 2.27 (s, 3H), 2.26 - 2.17 (m, 1H), 1.56 (s, 4H), 0.91 (s, 9H), 0.10 (s, 6H).

### Compound 11

HCl (12 M, 0.03 mL) was added to 11-7 (120 mg, 180.79 µmol) in THF (3 mL), and the reaction solution was stirred at 20 °C for 3 hours. The reaction solution was diluted with water (10 mL), adjusted to pH = 8 with a saturated NaHCO3 solution and extracted with ethyl acetate (10 × 1 mL); the organic phase was dried over anhydrous sodium sulfate and then concentrated to obtain compound 11. ¹HNMR (400MHz, CDCl₃) δ 8.97-8.92 (d, *J*=4.8 Hz, 1H), 8.11 (s, 1H), 7.95-7.90 (br d, *J*=4.8 Hz, 1H), 7.88 (s, 1H), 7.64-7.58(br d, *J*=8.0 Hz, 1H), 7.52 (s, 1H), 7.36-7.30 (d, *J*=8.4 Hz, 1H), 6.90 (s, 1H), 6.68 (s, 1H), 4.59 - 4.50 (m, 2H), 4.17 - 3.97 (m, 4H), 3.67 - 3.49 (m, 2H), 3.27-3.23 (t, *J*=5.8 Hz, 1H), 2.53 - 2.42 (m, 1H), 2.41 - 2.30 (m, 1H), 2.28 (s, 3H), 2.23 - 2.11 (m, 1H). MS (ESI) m/z: 550.3 [M+H]⁺.

### Compound 11A and Compound 11B

Compound 11 was subjected to SFC separation (chiral column: DAICEL CHIRALPAK AD-H (250 mm^{∗}30 mm, 5 µm), mobile phase A: ethanol (containing 0.05% DIEA); mobile phase B: carbon dioxide) to obtain compound 11A (retention time: 1.325 min) and compound 11B (retention time: 1.422 min). Compound 11A: ¹HNMR (400MHz, CDCl₃) δ 8.97-8.92 (d, *J*=4.8 Hz, 1H), 8.11 (s, 1H), 7.95-7.90 (br d, *J*=4.8 Hz, 1H), 7.88 (s, 1H), 7.64-7.58(br d, *J*=8.0 Hz, 1H), 7.52 (s, 1H), 7.36-7.30 (d, *J*=8.4 Hz, 1H), 6.90 (s, 1H), 6.68 (s, 1H), 4.59 - 4.50 (m, 2H), 4.17 - 3.97 (m, 4H), 3.67 - 3.49 (m, 2H), 3.27-3.23 (t, *J*=5.8 Hz, 1H), 2.53 - 2.42 (m, 1H), 2.41 - 2.30 (m, 1H), 2.28 (s, 3H), 2.23 - 2.11 (m, 1H). MS (ESI) m/z: 550.0 [M+H]⁺, 100% (ee%). Compound 11B: ¹HNMR (400MHz, CDCl₃) δ 8.97-8.92 (d, *J*=4.8 Hz, 1H), 8.11 (s, 1H), 7.95-7.90 (br d, *J*=4.8 Hz, 1H), 7.88 (s, 1H), 7.64-7.58(br d, *J*=8.0 Hz, 1H), 7.52 (s, 1H), 7.36-7.30 (d, *J*=8.4 Hz, 1H), 6.90 (s, 1H), 6.68 (s, 1H), 4.59 - 4.50 (m, 2H), 4.17 - 3.97 (m, 4H), 3.67 - 3.49 (m, 2H), 3.27-3.23 (t, *J*=5.8 Hz, 1H), 2.53 - 2.42 (m, 1H), 2.41 - 2.30 (m, 1H), 2.28 (s, 3H), 2.23 - 2.11 (m, 1H). MS (ESI) m/z: 550.0 [M+H]⁺, 97.5% (ee%).

### Example 12

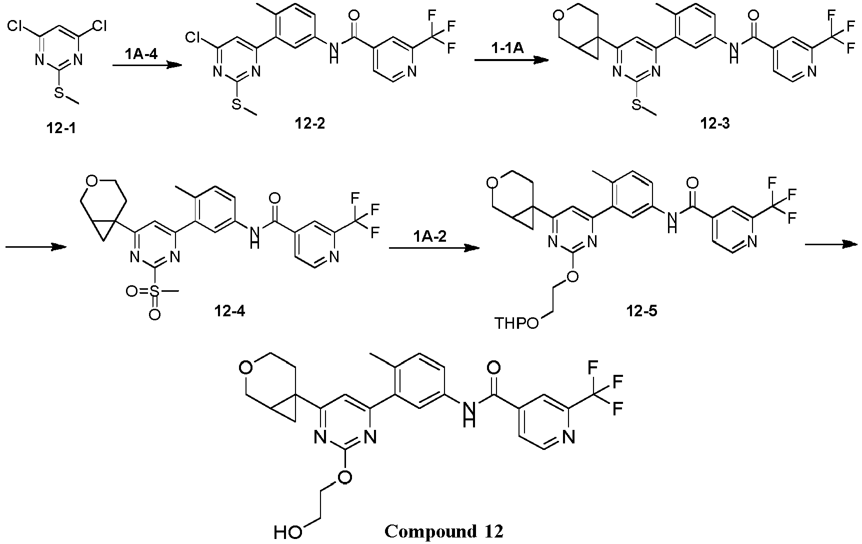

### Compound 12-2

12-1 (480 mg, 2.46 mmol), 1A-4 (1 g, 2.46 mmol), Pd(dppf)Cl₂ (400 mg, 489.81 µmol) and sodium carbonate (520 mg, 4.91 mmol) were dissolved in a mixed solvent of dioxane (20 mL) and water (4 mL). Under nitrogen protection, the reaction mixture was heated to 100 °C and stirred for 2 hours. The reaction solution was filtered; the filtrate was concentrated; and the residue was separated by silica gel column chromatography (PE/EA= 10/1 to 5/1, V/V) to obtain compound 12-2. ¹HNMR (400 MHz, CDCl₃) δ 8.92 (d, *J*=5.0 Hz, 1H), 8.11 (s, 2H), 7.93 (d, *J*=4.2 Hz, 1H), 7.76 (d, *J*=2.2 Hz, 1H), 7.66 (dd, *J*=2.2, 8.2 Hz, 1H), 7.32 (d, *J*=8.2 Hz, 1H), 7.14 (s, 1H), 2.61 (s, 3H), 2.44 (s, 3H).

### Compound 12-3

12-2 (300 mg, 683.60 µmol), 1-1A (156 mg, 764.56 µmol), Pd(dppf)Cl₂ (120 mg, 146.94 µmol) and cesium carbonate (450 mg, 1.38 mmol) were dissolved in DME (10 mL) and H₂O (2 mL). Under nitrogen protection, the reaction mixture was heated to 110 °C and stirred for 16 hours. The reaction solution was filtered; the filtrate was concentrated; and the residue was separated by silica gel column chromatography (PE/EA = 10/1 to 5/1, V/V) to obtain compound 12-2. MS (ESI) m/z: 501.2 [M+H] ⁺.

### Compound 12-4

To 12-3 (80 mg, 159.83 µmol) in dichloromethane (2 mL) solvent, m-chloroperoxybenzoic acid (65 mg, 320.17 µmol, 85%) was added. The reaction mixture was stirred at 15 °C for 1 hour. Water (2 mL) and dichloromethane (3 mL) were added to the reaction solution, and the mixture was subjected to liquid separation; the organic phase was washed with saturated sodium sulfite (1 mL); the organic phase was concentrated to obtain compound 12-4. MS (ESI) m/z: 533.3 [M+H] ⁺.

### Compound 12-5

Sodium hydride (16 mg, 400.04 µmol, 60%) was added to a solution of 1A-2 (16 mg, 109.45 µmol) in dioxane (2 mL). 12-4 (55 mg, 103.28 µmol) was added to the above-mentioned mixture, and the resulting mixture was stirred at 15 °C for 1 hour. A saturated ammonium chloride solution (3 mL) was added to the reaction solution, and the mixture was extracted with ethyl acetate (5 × 3 mL); the organic phase was dried over anhydrous sodium sulfate and then concentrated to obtain compound 12-5. MS (ESI) m/z: 599.4 [M+H]⁺.

### Compound 12

HCl (2 M, 0.15 mL) was added to 12-5 (50 mg, 83.53 µmol) in DMF (2 mL), and the reaction solution was stirred at 15 °C for 1 hour. A saturated NaHCO3 solution (5 mL) was added to the reaction solution, and the mixture was extracted with ethyl acetate (5×3 mL); the organic phase was dried over anhydrous sodium sulfate and then concentrated; and the residue was separated by chromatographic column (silica gel) (HCOOH-MeCN-H₂O) to obtain compound 12. ¹HNMR (400 MHz, DMSO-d₆) δ 10.80 (s, 1H), 9.02-8.97 (d, *J*=5.0 Hz, 1H), 8.39 (s, 1H), 8.24-8.20 (d, *J*=4.4 Hz, 1H), 7.89-7.86 (d, *J*=2.0 Hz, 1H), 7.87-7.83 (dd, *J*=2.2, 8.4 Hz, 1H), 7.39-7.34 (d, *J*=8.4 Hz, 1H), 7.17 (s, 1H), 4.91 (br s, 1H), 4.36-4.31 (t, *J*=5.2 Hz, 2H), 3.96 - 3.88 (m, 1H), 3.87 - 3.81 (m, 1H), 3.75-3.70 (br t, *J*=4.8 Hz, 2H), 3.60 - 3.52 (m, 1H), 2.64-2.58 (td, *J*=4.8, 14.0 Hz, 1H), 2.38 (s, 3H), 1.99-1.89 (ddd, *J*=5.8, 8.6, 14.2 Hz, 1H), 1.88 - 1.80 (m, 1H), 1.49-1.40 (dd, *J*=3.8, 9.2 Hz, 1H), 1.16-1.10 (dd, *J*=3.8, 6.4 Hz, 1H). MS (ESI) m/z: 515.1 [M+H]⁺.

### Example 13

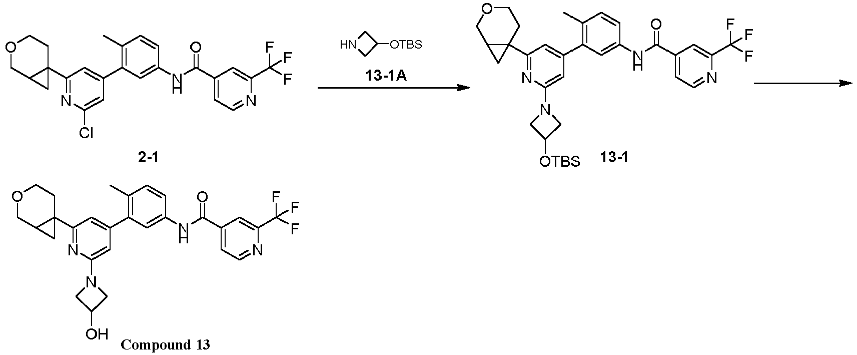

### Compound 13-1

Pd₂dba₃ (30 mg, 32.76 µmol), Ruphos (30 mg, 64.29 µmol) and cesium carbonate (150 mg, 460.38 µmol) were added to a solution of 2-1 (100 mg, 204.96 µmol) and 13-1A(100 mg, 533.75 µmol) in toluene (4 mL). Under nitrogen protection, the reaction mixture was heated to 120 °C and stirred for 4 hours. The reaction solution was filtered; the filtrate was concentrated; and the residue was separated by thin-layer silica gel plate (PE/EA = 3/1, V/V) to obtain compound 13-1. ¹HNMR (400 MHz, CDCl₃) δ 8.93 (d, *J*=5.0 Hz, 1H), 8.11 (s, 1H), 7.92 (br d, *J*=4.6 Hz, 1H), 7.85 (s, 1H), 7.62 (br d, *J*=8.4 Hz, 1H), 7.42 (s, 1H), 7.30 (d, *J*=8.4 Hz, 1H), 6.51 (s, 1H), 6.03 (s, 1H), 4.76 (quin, *J*=5.6 Hz, 1H), 4.22 (t, *J*=7.6 Hz, 2H), 4.06 - 3.99 (m, 1H), 3.98 - 3.93 (m, 1H), 3.84 - 3.77 (m, 2H), 3.62 (td, *J*=5.4, 11.2 Hz, 1H), 3.45 (ddd, *J*=5.4, 8.8, 11.2 Hz, 1H), 2.57 (td, *J*=4.8, 14.0 Hz, 1H), 2.10 - 2.03 (m, 1H), 1.81 (td, *J*=4.6, 9.2 Hz, 1H), 1.37 (dd, *J*=3.8, 9.2 Hz, 1H), 0.99 (dd, *J*=3.8, 6.4 Hz, 2H), 0.93 - 0.90 (m, 9H), 0.11 - 0.07 (m, 6H).

### Compound 13

TFA(1 mL) was added to 13-1 (80 mg, 125.24 µmol) in dichloromethane (4 mL), and the reaction solution was stirred at 15 °C for 13 hours. The reaction solution was diluted with dichloromethane (10 mL), then adjusted to pH = 8 with a saturated NaHCO3 solution and extracted with dichloromethane (10 × 3 mL); the organic phase was dried over anhydrous sodium sulfate and then concentrated; and the residue was separated by chromatographic column (silica gel) (HCOOH-MeCN-H₂O) to obtain compound 13. ¹HNMR (400 MHz, DMSO-d₆) δ 10.69 (s, 1H), 9.02-8.97 (d, *J*=5.0 Hz, 1H), 8.37 (s, 1H), 8.22-8.18 (d, *J*=5.0 Hz, 1H), 7.77-7.73 (dd, *J*=2.0, 8.4 Hz, 1H), 7.64-7.61 (d, *J*=2.0 Hz, 1H), 7.35-7.30 (d, *J*=8.4 Hz, 1H), 6.54 (s, 1H), 6.12 (br s, 1H), 5.77 - 5.36 (m, 1H), 4.64 - 4.50 (m, 1H), 4.18-4.14 (br t, *J*=7.2 Hz, 2H), 3.96-3.92 (dd, *J*=4.6, 11.2 Hz, 1H), 3.83-3.79 (d, *J*=10.2 Hz, 1H), 3.75 - 3.61 (m, 2H), 3.51-3.47 (td, *J*=5.6, 11.4 Hz, 1H), 3.42 - 3.37 (m, 2H), 2.23 (s, 3H), 1.98-1.94 (ddd, J=5.6, 8.2, 13.8 Hz, 1H), 1.77 - 1.70 (m, 1H), 1.29-1.27 (dd, *J*=3.6, 9.0 Hz, 1H), 0.95-0.90 (dd, J=3.6, 6.0 Hz, 1H). MS (ESI) m/z: 525.2 [M+H] ⁺.

### Example 14

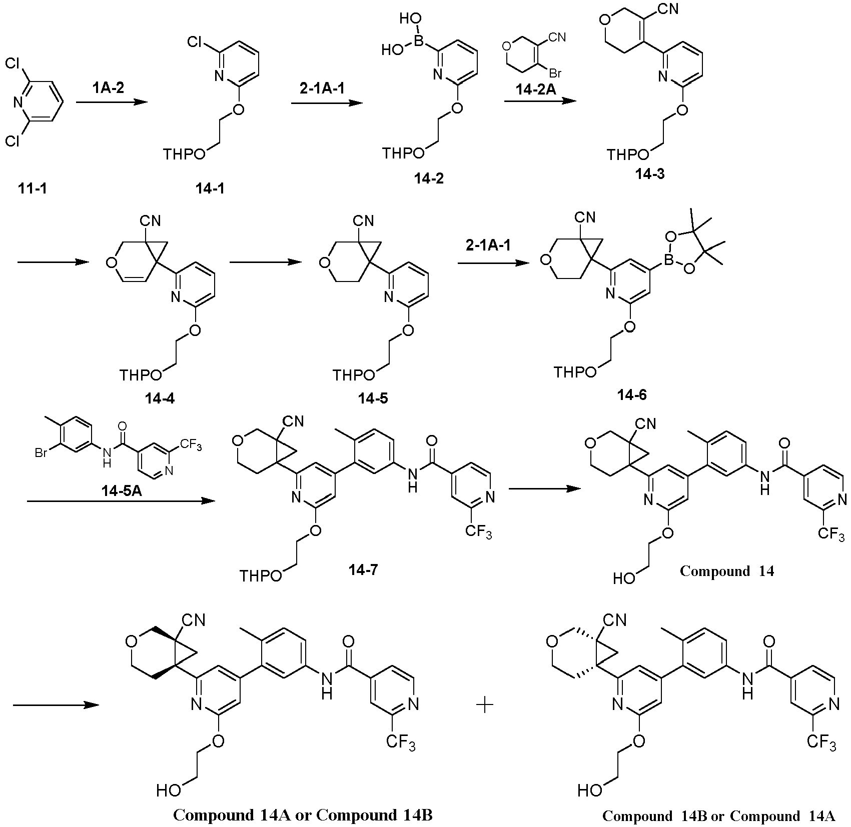

### Compound 14-1

At 0°C, sodium hydride (5.41 g, 135.14 mmol, 60% purity) was added to a solution of 1A-2 (11.8 g, 80.72 mmol) in THF (100 mL), and the mixture was warmed to 20 °C and stirred for 30 minutes. At 0°C, 11-1 (10 g, 67.57 mmol) was added to the mixed solution, and the mixture was warmed to 20 °C and stirred for 4 hours. A saturated ammonium chloride solution (30 mL) was added to the reaction mixture, and the resulting mixture was extracted with ethyl acetate (40 mL × 2); the combined extract was concentrated; and the residue was separated by column chromatography (PE/EA= 100/0 to 80/1, V/V) to obtain 14-1. ¹HNMR (400MHz, CDCl₃) δ 7.55 - 7.49 (m, 1H), 6.90 (d, *J*=7.4 Hz, 1H), 6.71 (d, *J*=8.2 Hz, 1H), 4.71 (t, *J*=3.6 Hz, 1H), 4.58 - 4.43 (m, 2H), 4.05 (ddd, *J*=3.6, 5.8, 11.6 Hz, 1H), 3.90 (ddd, *J*=3.2, 8.0, 11.4 Hz, 1H),3.81 (ddd, *J*=3.6, 6.4, 11.4 Hz, 1H), 3.58 - 3.49 (m, 1H), 1.91 - 1.80 (m, 1H), 1.79 - 1.70 (m, 1H), 1.68 - 1.50 (m, 4H).

### Compound 14-2

14-1 (10 g, 38.80 mmol), 2-1A-1 (20 g, 78.76 mmol), Pd(dppf)Cl₂ (2.75 g, 3.76 mmol) and potassium acetate (11.5 g, 117.18 mmol) were dissolved in DMF (200 mL). Under nitrogen protection, the reaction mixture was heated to 100 °C and stirred for 8 hours. The reaction solution was filtered to obtain compound 14-2 solution. MS (ESI): m/z 268.2 [M+H]⁺.

### Compound 14-3

14-2 (9.6 g, 35.94 mmol), 14-2A (6 g, 31.91 mmol), Pd(dppf)Cl₂ (2.4 g, 3.28 mmol) and sodium carbonate (7.2 g, 67.93 mmol) were dissolved in a mixed solvent of DMF (240 mL) and water (48 mL). Under nitrogen protection, the reaction mixture was heated to 100 °C and stirred for 3 hours. Water (300 mL) was added to the reaction solution, and the mixture was extracted with ethyl acetate (200 × 2 mL); the organic phase was washed with saturated brine (100 × 2 mL), dried over anhydrous sodium sulfate and then concentrated; and the residue was separated by silica gel column chromatography (PE/EA = 16/1 to 10/1, V/V) to obtain compound 14-3. ¹HNMR (400MHz, CDCl₃) δ 7.65 (dd, *J*=7.4, 8.2 Hz, 1H), 7.17 (d, *J*=7.4 Hz, 1H), 6.86 (d, *J*=8.2 Hz, 1H), 4.74 - 4.71 (m, 1H), 4.70 - 4.59 (m, 2H), 4.43 (t, *J*=2.6 Hz, 2H), 4.10 (ddd, *J*=3.2, 5.8, 11.6 Hz, 1H), 3.97 (t, *J*=5.4 Hz, 2H), 3.94 - 3.77 (m, 2H), 3.57 - 3.49 (m, 1H), 2.77 (tt, *J*=2.8, 5.6 Hz, 2H), 1.91 - 1.82 (m, 1H), 1.80 - 1.71 (m, 1H), 1.70 - 1.62 (m, 2H), 1.58 - 1.52 (m, 2H).

### Compound 14-4

At 0°C, a solution of tert-butyl alcohol lithium (1.45 g, 18.16 mmol) in NMP (20 mL) was added dropwise to a solution of 14-3 (2 g, 6.05 mmol) and chloroiodomethane (2 g, 18.16 mmol) in NMP (20 mL), and the mixture was warmed to 20 °C and stirred for 12 hours. A saturated ammonium chloride solution (50 mL) and water (50 mL) were added to the reaction mixture, and the resulting mixture was extracted with ethyl acetate (50 mL × 2); the combined extract was concentrated; and the residue was separated by column chromatography (PE/EA = 20/0 to 8/1) to obtain 14-4. ¹HNMR (400MHz, CDCl₃) δ 7.66 - 7.53 (m, 1H), 7.04 (d, *J=* 7.4 Hz, 1H), 6.71 (d, *J=* 8.4 Hz, 1H), 6.44 (d, *J=* 6.2 Hz, 1H), 5.57 (d, *J=* 6.2 Hz, 1H), 4.74 - 4.67 (m, 1H), 4.62 (ddd, *J* = 3.8, 6.2, 11.6 Hz, 1H), 4.55 (t, *J* = 5.0 Hz, 1H), 4.47 (ddd, *J* = 3.8, 6.4, 11.7 Hz, 1H), 4.29 (d, *J* = 10.8 Hz, 1H), 4.11 - 4.02 (m, 1H), 3.98 (d, *J* = 10.8 Hz, 1H), 3.91 (ddd, *J* = 3.2, 8.0, 11.2 Hz, 1H), 3.87 - 3.77 (m, 1H), 3.57 - 3.48 (m, 1H), 2.78 (ddd, *J* = 0.8, 4.6, 7.2 Hz, 1H), 1.89 - 1.81 (m, 1H), 1.77 (d, *J* = 4.0 Hz, 1H), 1.76 - 1.70 (m, 1H), 1.68 - 1.57 (m, 2H), 1.57 - 1.49 (m, 2H).

### Compound 14-5

Pd/C (10%, 400 mg) was added to a solution of 14-4 (1.5 g, 4.38 mmol) in methanol (20 mL); under hydrogen gas (15 psi), the mixture was stirred at 20 °C for 1 hour. The reaction solution was filtered and then concentrated to obtain compound 14-5 as a crude. ¹HNMR (400MHz, CDCl₃) δ 7.62 - 7.56 (m, 1H), 6.93 (dd, *J*=4.2, 7.2 Hz, 1H), 6.71 (d, *J*=8.4 Hz, 1H), 4.73 (t, *J*=3.6 Hz, 1H), 4.65 - 4.49 (m, 2H), 4.19 - 4.13 (m, 1H), 4.09 (dddd, *J*=1.6, 3.8, 5.6, 11.2 Hz, 1H), 4.05 - 4.01 (m, 1H), 3.93 (ddd, *J*=3.2, 8.0, 11.2 Hz, 1H), 3.89 - 3.79 (m, 2H), 3.58 - 3.48 (m, 2H), 2.59 - 2.49 (m, 1H), 2.28 (dd, *J*=5.0, 12.4 Hz, 1H), 2.12 - 2.00 (m, 1H), 1.94 - 1.83 (m, 1H), 1.82 - 1.72 (m, 1H), 1.71 - 1.64 (m, 1H), 1.62 (s, 1H), 1.59 - 1.54 (m, 2H), 1.49 (d, *J*=5.0 Hz, 1H).

### Compound 14-6

2-1A-1 (884.79 mg, 3.48 mmol), [Ir(COD)OMe]₂ (200 mg, 301.72 µmol) and dtbpy (162.10 mg, 603.94 µmol) were dissolved in tetrahydrofuran (10 mL). Under nitrogen protection, the reaction mixture was heated to 80 °C and stirred for 5 minutes. A solution of 14-5 (800 mg, 2.32 mmol) in tetrahydrofuran (10 mL) was then added, and the mixture was stirred at 80 °C for 4 hours. The reaction solution was filtered and then concentrated to obtain compound 14-6 as a crude. MS (ESI): m/z 305.1 [M+H-THP]⁺.

### Compound 14-7

14-6 (500 mg, 1.39 mmol), 14-5A (600 mg, 1.28 mmol), Pd(dppf)Cl₂ (93.66 mg, 128.00 µmol) and sodium carbonate (271.33 mg, 2.56 mmol) were dissolved in dioxane (10 mL) and water (2 mL). Under nitrogen protection, the reaction mixture was heated to 80 °C and stirred for 2 hours. The reaction mixture was filtered; the filtrate was concentrated; ethyl acetate (30 mL) was then added; the mixture was washed with saturated brine (20 mL × 2) and then concentrated; and the residue was separated by column chromatography (PE/EA= 20/1 to 5/1) to obtain 14-7. MS (ESI): m/z 623.3 [M+H]⁺.

### Compound 14

TFA(0.5 mL) was added to 14-7 (50 mg, 80.30 µmol) in dichloromethane (1 mL), and the reaction solution was stirred at 20 °C for 0.5 hours. The reaction solution was adjusted to pH = 8 with a saturated sodium bicarbonate solution and extracted with dichloromethane (20 × 3 mL); the organic phase was dried over anhydrous sodium sulfate and then concentrated; and the residue was separated by thin-layer silica gel plate (PE/EA = 1/2, V/V) to obtain compound 14. ¹HNMR (400 MHz, DMSO-d₆) δ 10.71 (s, 1H), 9.02-8.95 (d, *J* = 4.8 Hz, 1H), 8.37 (s, 1H), 8.21-8.18 (d, *J=* 4.8 Hz, 1H), 7.82-7.74 (dd, *J=* 2.2, 8.4 Hz, 1H), 7.68-7.60 (d, *J* = 2.2 Hz, 1H), 7.39-7.30 (d, *J=* 8.4 Hz, 1H), 7.14 (s, 1H), 6.69 (s, 1H), 4.89-4.83 (t, *J=* 5.6 Hz, 1H), 4.39-4.32 (t, *J=* 5.0 Hz, 2H), 4.10-4.05 (d, *J=* 11.2 Hz, 1H), 3.98-4.86 (d, *J=* 11.4 Hz, 1H), 3.82 - 3.68 (m, 3H), 3.55 - 3.49 (m, 1H), 2.61 - 2.55 (m, 1H), 2.46-2.38 (d, *J=* 5.4 Hz, 1H), 2.23 (s, 3H), 2.02 - 1.93 (m, 1H), 1.54-1.47 (d, *J* = 5.4 Hz, 1H). MS (ESI): m/z 539.2 [M+H]⁺.

### Compound 14A and 14B

Compound 14 was subjected to SFC chiral separation (chiral column: DAICEL CHIRALPAK AD (250 mm^{∗}30 mm, 10 µm), mobile phase A: isopropanol (containing 0.05% DIEA); mobile phase B: carbon dioxide) to obtain compound 14A (retention time: 1.702 min) and compound 14B (retention time: 1.815 min). Compound 14A: ¹HNMR (400 MHz, DMSO-d₆) δ 10.71 (s, 1H), 9.02-8.95 (d, *J=* 4.8 Hz, 1H), 8.37 (s, 1H), 8.21-8.18 (d, *J=* 4.8 Hz, 1H), 7.82-7.74 (dd, *J=* 2.2, 8.4 Hz, 1H), 7.68-7.60 (d, *J=* 2.2 Hz, 1H), 7.39-7.30 (d, *J* = 8.4 Hz, 1H), 7.14 (s, 1H), 6.69 (s, 1H), 4.89-4.83 (t, *J* = 5.6 Hz, 1H), 4.39-4.32 (t, *J* = 5.0 Hz, 2H), 4.10-4.05 (d, *J* = 11.2 Hz, 1H), 3.98-4.86 (d, *J* = 11.4 Hz, 1H), 3.82 - 3.68 (m, 3H), 3.55 - 3.49 (m, 1H), 2.61 - 2.55 (m, 1H), 2.46-2.38 *(d, J=* 5.4 Hz, 1H), 2.23 (s, 3H), 2.02 - 1.93 (m, 1H), 1.54-1.47 (d, *J* = 5.4 Hz, 1H). MS (ESI): m/z 539.2 [M+H]⁺, 100% (ee%). Compound 14B: ¹HNMR (400 MHz, DMSO-d₆) δ 10.71 (s, 1H), 9.02-8.95 (d, *J=* 4.8 Hz, 1H), 8.37 (s, 1H), 8.21-8.18 (d, *J* = 4.8 Hz, 1H), 7.82-7.74 (dd, *J* = 2.2, 8.4 Hz, 1H), 7.68-7.60 (d, *J* = 2.2 Hz, 1H), 7.39-7.30 (d, *J* = 8.4 Hz, 1H), 7.14 (s, 1H), 6.69 (s, 1H), 4.89-4.83 (t, *J* = 5.6 Hz, 1H), 4.39-4.32 (t, *J* = 5.0 Hz, 2H), 4.10-4.05 (d, *J* = 11.2 Hz, 1H), 3.98-4.86 (d, *J* = 11.4 Hz, 1H), 3.82 - 3.68 (m, 3H), 3.55 - 3.49 (m, 1H), 2.61 - 2.55 (m, 1H), 2.46-2.38 (d, *J* = 5.4 Hz, 1H), 2.23 (s, 3H), 2.02- 1.93 (m, 1H), 1.54-1.47 (d,*J* = 5.4 Hz, 1H). MS (ESI): m/z 539.2 [M+H]⁺, 100% (ee%).

### Example 15

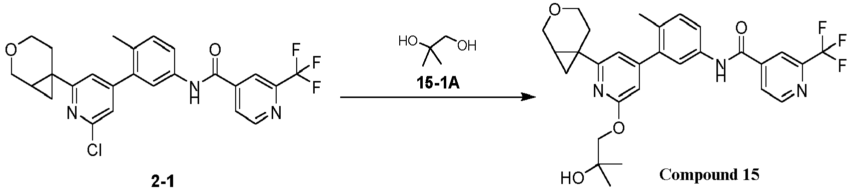

### Compound 15

Pd₂dba₃ (37.54 mg, 40.99 µmol), Brettphos (44.01 mg, 81.98 µmol) and cesium carbonate (267.12 mg, 819.84 µmol) were added to a solution of 2-1 (200 mg, 409.92 µmol) and 15-1A (84.97 mg, 942.81 µmol) in toluene (5 mL). Under nitrogen protection, the reaction mixture was heated to 100 °C and stirred for 4 hours. The reaction solution was diluted with ethyl acetate (10 mL) and then filtered; the filtrate was concentrated; and the residue was separated by chromatographic column (silica gel) (HCOOH-MeCN-H₂O) to obtain compound 15. ¹HNMR (400 MHz, CD₃OD) δ 8.94-8.92 (d, *J* = 5.0 Hz, 1H), 8.32 (s, 1H), 8.16-8.12 (dd, *J* = 1.0, 5.0 Hz, 1H), 7.72-7.66 (dd, *J* = 2.4, 8.2 Hz, 1H), 7.66-7.63 (d, *J* = 2.2 Hz, 1H), 7.37-7.32 (d, *J* = 8.6 Hz, 1H), 6.90-6.87 (d, *J* = 1.0 Hz, 1H), 6.60-6.58 (d, *J* = 1.0 Hz, 1H), 4.60 (s, 2H), 4.20 (s, 2H), 4.10 - 4.05 (m, 1H), 3.98-3.92 (m, 1H), 3.64 - 3.61 (m, 1H), 3.54 - 3.46 (m, 1H), 2.60-2.58 (m, 1H), 2.29 (s, 3H), 2.06 - 1.98 (m, 1H), 1.85 - 1.76 (m, 1H), 1.43-1.38 (m, 1H), 1.33 (s, 6H), 1.06-1.04 (m, 1H). MS (ESI) m/z: 542.1 [M+H]⁺.

### Example 16

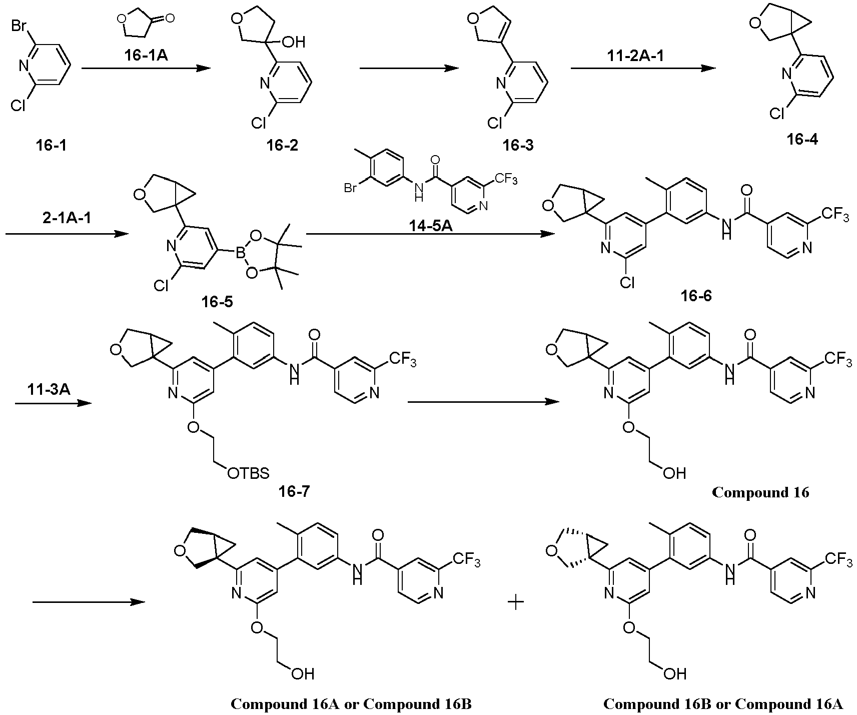

### Compound 16-2

At -78 °C, n-butyllithium (23 mL, 2.5 M) was added to a solution of 16-1 (10 g, 51.96 mmol) in dichloromethane (100 mL), and the mixture was stirred for 30 minutes. At -78 °C, 16-1A (4.92 g, 57.16 mmol) was added to the mixed solution, and the mixture was warmed to 25°C and stirred for 0.5 hours. A saturated ammonium chloride solution (40 mL) was added to the reaction mixture, and the resulting mixture was extracted with dichloromethane (40 mL × 2); the combined extract was dried over anhydrous sodium sulfate and then concentrated; and the residue was separated by column chromatography (PE/EA= 10/1 to 5/1, V/V) to obtain compound 16-2. ¹HNMR (400MHz, CDCl₃) δ 7.72 (t, *J*=7.8 Hz, 1H), 7.47 (d, *J*=7.2 Hz, 1H), 7.28 (d, *J*=7.2 Hz, 1H), 4.35 (s, 1H), 4.24 - 4.14 (m, 2H), 4.05 - 4.00 (m, 1H), 3.96 - 3.92 (m, 1H), 2.45 (td, *J*=8.8, 13.0 Hz, 1H), 2.32 - 2.22 (m, 1H).

### Compound 16-3

To a solution of 16-2 (6 g, 30.06 mmol) in toluene (80 mL), p-toluenesulfonic acid (11.5 g, 60.46 mmol) was added; and the mixture was warmed to 110 °C and stirred for 16 hours. A saturated sodium bicarbonate solution (40 mL) and ethyl acetate (100 mL) were added to the reaction mixture, and the resulting mixture was subjected to liquid separation; the organic phase was washed with a saturated sodium bicarbonate solution (50 mL × 2), dried over anhydrous sodium sulfate and then concentrated; and the residue was separated by silica gel column chromatography (PE/EA= 50/1 to 20/1, V/V) to obtain compound 16-3. ¹HNMR (400MHz, CDCl₃) δ 7.64 (t, *J*=7.8 Hz, 1H), 7.23 (dd, *J*=5.2, 7.8 Hz, 2H), 6.68 (quin, *J*=2.0 Hz, 1H), 5.08 (dt, *J*=2.0, 5.0 Hz, 2H), 4.91 (dt, *J*=2.0, 5.0 Hz, 2H).

### Compound 16-4

DMSO (50 mL) was added to a mixture of potassium tert-butoxide (3.71 g, 33.04 mmol) and 11-2A-1 (7.3 g, 33.17 mmol). Under nitrogen protection, the reaction mixture was stirred at 25 °C for 1 hour. A solution of 16-3 (1 g, 5.51 mmol) in DMSO (5 mL) was added to the reaction solution, and the reaction mixture was heated to 70 °C and stirred for 6 hours. Water (180 mL) was added to the reaction mixture, and the resulting mixture was extracted with ethyl acetate (30 × 3 mL); the organic phase was dried over anhydrous sodium sulfate and then concentrated; and the residue was separated by silica gel column chromatography (PE/EA = 20/1, V/V) to obtain compound 16-4. ¹HNMR (400 MHz, CDCl₃) δ 7.46 (t, *J*=7.8 Hz, 1H), 7.05 (d, *J*=7.8 Hz, 1H), 6.88 (d, *J*=7.6 Hz, 1H), 4.11 - 4.04 (m, 2H), 3.89 - 3.78 (m, 2H), 2.08 (ddd, *J*=2.8, 5.2, 8.0 Hz, 1H), 1.32 (dd, *J*=4.4, 8.0 Hz, 1H), 1.07 (t, *J*=4.6 Hz, 1H).

### Compound 16-5

2-1A-1 (1.08 g, 4.25 mmol), [Ir(COD)OMe]₂ (70 mg, 105.60 µmol) and tmphen (50 mg, 211.59 µmol) were dissolved in methyl tertiary butyl ether (20 mL). Under nitrogen protection, 16-4 (770 mg, 3.94 mmol) was added to the reaction mixture, and the resulting mixture was heated to 80 °C and stirred for 3 hours. The reaction solution was filtered, and the filtrate was concentrated to obtain compound 16-5 as a crude. MS (ESI): m/z 240.3 [M-84+2H]⁺.

### Compound 16-6

16-5 (1.2 g, 3.73 mmol), 14-5A (1.4 g, 3.90 mmol), Pd(dppf)Cl₂·DCM (300 mg, 367.36 µmol) and sodium carbonate (800 mg, 7.55 mmol) were dissolved in dioxane (50 mL) and water (10 mL). Under nitrogen protection, the reaction mixture was heated to 100 °C and stirred for 3 hours. The reaction mixture was filtered; the filtrate was concentrated; ethyl acetate (20 mL) and water (10 mL) were then added; the mixture was subjected to liquid separation; the aqueous phase was extracted with ethyl acetate (10 mL × 3); the combined organic phase was dried over anhydrous sodium sulfate and then concentrated; and the residue was separated by silica gel column chromatography (PE/EA = 10/1 to 5/1) to obtain 16-6. ¹HNMR (400 MHz, CDCl₃) δ 8.92 (d, *J*=5.0 Hz, 1H), 8.20 (s, 1H), 8.11 (s, 1H), 7.94 (d, *J*=5.0 Hz, 1H), 7.60 - 7.51 (m, 2H), 7.32 (d, *J*=7.8 Hz, 1H), 7.10 (d, *J*=1.2 Hz, 1H), 6.90 (d, *J*=1.2 Hz, 1H), 4.19 - 4.14 (m, 2H), 3.95 - 3.87 (m, 2H), 2.27 (s, 3H), 2.24 - 2.17 (m, 1H), 1.48 - 1.41 (m, 1H), 1.20 - 1.15 (m, 1H).

### Compound 16-7

Pd₂dba₃ (180 mg, 196.57 µmol), Brettphos (200 mg, 372.60 µmol) and cesium carbonate (1.26 g, 3.87 mmol) were added to a solution of 16-6 (900 mg, 1.90 mmol) and 11-3A(400 mg, 2.27 mmol) in toluene (30 mL). Under nitrogen protection, the reaction mixture was heated to 110 °C and stirred for 4 hours. The reaction solution was filtered; the filtrate was concentrated; and the residue was separated by silica gel column chromatography (PE/EA = 20/1 to 6/1) to obtain compound 16-7. ¹HNMR (400 MHz, DMSO-d₆) δ 10.65 (s, 1H), 8.95 (d, *J*=5.0 Hz, 1H), 8.31 (s, 1H), 8.14 (d, *J*=4.6 Hz, 1H), 7.70 (dd, *J*=2.0, 8.2 Hz, 1H), 7.59 (d, J=2.2 Hz, 1H), 7.29 (d, *J*=8.4 Hz, 1H), 6.68 (s, 1H), 6.49 (s, 1H), 4.31 (br d, *J*=2.8 Hz, 1H), 4.07 - 3.97 (m, 2H), 3.90 - 3.86 (m, 2H), 3.79 - 3.69 (m, 2H), 2.17 (s, 3H), 2.15 - 2.09 (m, 1H), 1.33 (dd, *J*=3.8, 7.8 Hz, 1H), 1.13 (t, *J*=7.2 Hz, 1H), 0.96 - 0.91 (m, 1H), 0.80 (s, 9H), 0.00 (s, 6H).

### Compound 16

Hydrochloric acid (1 mL, 4 M) was added to 16-7 (300 mg, 488.81 µmol) in tetrahydrofuran (10 mL), and the reaction solution was stirred at 25 °C for 1 hour. The reaction solution was diluted with ethyl acetate (20 mL), then adjusted to pH = 8 with a saturated NaHCO3 solution and extracted with ethyl acetate (10 × 3 mL); the organic phase was dried over anhydrous sodium sulfate and then concentrated; and the residue was separated by thin-layer silica gel plate (PE/EA= 1/1, V/V) to obtain compound 16. ¹HNMR (400 MHz, DMSO-d₆) δ 10.70 (s, 1H), 9.06-8.95 (d, *J*=5.0 Hz, 1H), 8.37 (s, 1H), 8.23-8.18 (d, *J*=4.2 Hz, 1H), 7.77-7.71 (dd, *J*=2.2, 8.4 Hz, 1H), 7.68-7.64 (d, *J*=2.2 Hz, 1H), 7.39-7.31 (d, *J*=8.4 Hz, 1H), 6.73 (s, 1H), 6.59-6.54 (d, *J*=1.0 Hz, 1H), 4.86-4.81 (t, *J*=5.2 Hz, 1H), 4.35-4.30 (t, *J*=5.2 Hz, 2H), 4.13 - 4.03 (m, 2H), 3.83 - 3.70 (m, 4H), 2.24 (s, 3H), 2.22-2.08 (ddd, *J*=2.4, 5.0, 7.8 Hz, 1H), 1.40-1.32 (dd, *J*=3.6, 7.8 Hz, 1H), 1.02-0.96 (t, *J*=4.4 Hz, 1H). MS (ESI) m/z: 500.4 [M+H] ⁺.

### Compound 16A and 16B

Compound 16 was subjected to SFC chiral separation (chiral column: DAICEL CHIRALCEL OJ-H (250 mm^{∗}30 mm, 5 µm), mobile phase A: ethanol (containing 0.05% DIEA); mobile phase B: carbon dioxide) to obtain compound 16A (retention time: 1.487 min) and compound 16B (retention time: 1.590 min).

Compound 16A: ¹HNMR (400 MHz, DMSO-d₆) δ 10.70 (s, 1H), 9.06-8.95 (d, *J*=5.0 Hz, 1H), 8.37 (s, 1H), 8.23-8.18 (d, *J*=4.2 Hz, 1H), 7.77-7.71 (dd, *J*=2.2, 8.4 Hz, 1H), 7.68-7.64 (d, *J*=2.2 Hz, 1H), 7.39-7.31 (d, *J*=8.4 Hz, 1H), 6.73 (s, 1H), 6.59-6.54 (d, *J*=1.0 Hz, 1H), 4.86-4.81 (t, *J*=5.2 Hz, 1H), 4.35-4.30 (t, *J*=5.2 Hz, 2H), 4.13 - 4.03 (m, 2H), 3.83 - 3.70 (m, 4H), 2.24 (s, 3H), 2.22-2.08 (ddd, *J*=2.4, 5.0, 7.8 Hz, 1H), 1.40-1.32 (dd, *J*=3.6, 7.8 Hz, 1H), 1.02-0.96 (t, *J*=4.4 Hz, 1H). MS (ESI) m/z: 500.4 [M+H] ⁺, 100% (ee%).

Compound 16B: ¹HNMR (400 MHz, DMSO-d₆) δ 10.70 (s, 1H), 9.06-8.95 (d, *J=5.0* Hz, 1H), 8.37 (s, 1H), 8.23-8.18 (d, *J*=4.2 Hz, 1H), 7.77-7.71 (dd, *J*=2.2, 8.4 Hz, 1H), 7.68-7.64 (d, *J*=2.2 Hz, 1H), 7.39-7.31 (d, *J*=8.4 Hz, 1H), 6.73 (s, 1H), 6.59-6.54 (d, *J*=1.0 Hz, 1H), 4.86-4.81 (t, *J*=5.2 Hz, 1H), 4.35-4.30 (t, *J*=5.2 Hz, 2H), 4.13 - 4.03 (m, 2H), 3.83 - 3.70 (m, 4H), 2.24 (s, 3H), 2.22-2.08 (ddd, *J*=2.4, 5.0, 7.8 Hz, 1H), 1.40-1.32 (dd, *J*=3.6, 7.8 Hz, 1H), 1.02-0.96 (t, *J*=4.4 Hz, 1H). MS (ESI) m/z: 500.4 [M+H]⁺, 99.7% (ee%).

### Compound 16B

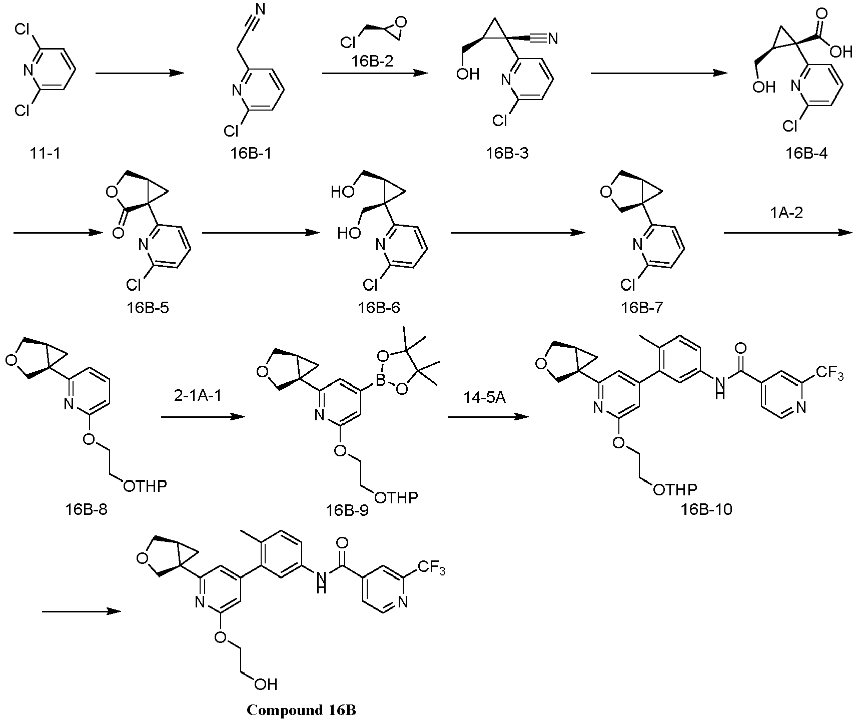

### Compound 16B-1

At -70 °C, n-butyllithium (500 mL, 2.5 M) was added to a solution of acetonitrile (54.60 g, 1.33 mol) in tetrahydrofuran (500 mL), and the reaction mixture was stirred for 1 hour. At -70 °C, a solution of 11-1 (65 g, 439.22 mmol) in tetrahydrofuran (200 mL) was added to the mixed solution, and the mixture was stirred for 1 hour and then warmed to 25 °C and stirred for another 1 hour. Water (200 mL) was added to the reaction mixture, and the mixture was extracted with ethyl acetate (100 mL × 2); the combined extract was dried over anhydrous sodium sulfate and then concentrated to obtain compound 16B-1 as a crude. ¹HNMR (400MHz, CDCl₃) δ 7.74 (t, *J*=7.8 Hz, 1H), 7.43 (d, *J*=7.4 Hz, 1H), 7.34 (d, *J*=7.8 Hz, 1H), 3.94 (s, 2H).

### Compound 16B-3

At -30 °C, LiHMDS (940.79 mL, 1 M) was added dropwise to a solution of 16B-1 (130 g, 852.01 mmol) and 16B-2 (80.5 g, 870.04 mmol) in methyl tertiary butyl ether (1.2 L), and the mixture was warmed to -10 °C-0 °C and stirred for 2 hours. At -30 °C, NaHMDS (855.26 mL, 1 M) was added dropwise to the mixed solution, and the mixture was warmed to 25 °C and stirred for 16 hours. Water (40 mL) was added to the reaction mixture, and the reaction solution was concentrated to obtain compound 16B-3 as a crude.

### Compound 16B-4

Potassium hydroxide (900 mL, 2 M) was added dropwise to a solution of 16B-3 (180 g, 862.71 mmol) in ethanol (1 L), and the mixture was warmed to 80 °C and stirred for 4 hours to obtain a mixed solution of compound 16B-4.

### Compound 16B-5

At 50 °C, hydrochloric acid (740 mL, 6 M) was added dropwise to a mixed solution 16B-4, and the mixture was warmed to 60 °C and stirred for 1 hour. The reaction mixture was concentrated to remove ethanol; the aqueous phase was extracted with ethyl acetate (800 mL × 3); the combined organic phase was washed with saturated brine (500 mL × 1), dried over anhydrous sodium sulfate and then concentrated; and the residue was separated by silica gel column chromatography (PE/EA = 20/1 to 10/1) to obtain compound 16B-5. ¹HNMR (400 MHz, CDCl₃) δ 8.06 (dd, *J*=0.8, 7.8 Hz, 1H), 7.63 (t, *J*=7.8 Hz, 1H), 7.16 (dd, *J*=0.8, 7.8 Hz, 1H), 4.41 (dd, *J*=4.6, 9.2 Hz, 1H), 4.29 (d, *J*=9.2 Hz, 1H), 3.02 - 2.84 (m, 1H), 2.12 (dd, *J*=4.2, 7.8 Hz, 1H), 1.54 - 1.38 (m, 1H).

### Compound 16B-6

At -5 °C, lithium borohydride (100 mL, 4 M) was added to a solution of 16B-5 (44 g, 209.89 mmol) in tetrahydrofuran (400 mL), and the mixture was warmed 20 °C and stirred for 2 hours. A saturated ammonium chloride aqueous solution (100 mL) was slowly added to the reaction mixture, and the mixture was filtered; the filtrate was washed with water (60 mL × 2); the organic phase was dried over anhydrous sodium sulfate and then concentrated to obtain a crude, which was slurried with PE/EA (10 : 1, 80 mL) to obtain compound 16B-6. ¹HNMR (400MHz, CDCl₃) δ 7.50 (t, *J*=7.8 Hz, 1H), 7.07 (dd, *J*=0.6, 7.8 Hz, 1H), 6.92 (d, *J*=7.8 Hz, 1H), 4.66 - 4.53 (m, 1H), 4.32 (dd, *J*=4.6, 8.3 Hz, 1H), 4.10 (ddd, *J*=5.2, 9.2, 12.4 Hz, 1H), 3.54 - 3.46 (m, 1H), 3.43 - 3.34 (m, 1H), 3.43 - 3.34 (m, 1H), 1.76 - 1.59 (m, 1H), 1.41 (dd, *J*=5.6, 8.8 Hz, 1H), 0.85 (t, *J*=5.6 Hz, 1H).

### Compound 16B-7

At 0 °C, DEAD (45.03 g, 258.53 mmol) was added dropwise to a solution of triphenylphosphine (58 g, 221.13 mmol) in tetrahydrofuran (500 mL), and the mixture was stirred at 0 °C for 30 minutes. At 0 °C, 16B-6 (40 g, 187.21 mmol) in tetrahydrofuran (200 mL) was added dropwise to the mixed solution, and the mixture was warmed to 25 °C and stirred for 1 hour. Water (100 mL) and ethyl acetate (300 mL) were added to the reaction mixture, and the resulting mixture was subjected to liquid separation; the organic phase was washed with water (100 mL × 2), dried over anhydrous sodium sulfate and then concentrated; and the residue was separated by column chromatography (PE/EA = 100/1 to 20/1, V/V) to obtain 16B-7. ¹HNMR (400MHz, CDCl₃) δ 7.54 (t, *J*=7.8 Hz, 1H), 7.12 (dd, *J*=0.6, 7.8 Hz, 1H), 6.95 (dd, *J*=0.6, 7.8 Hz, 1H), 4.18 - 4.13 (m, 2H), 3.95 - 3.85 (m, 2H), 2.16 (ddd, *J*=2.6, 5.2, 8.0 Hz, 1H), 1.40 (dd, *J*=4.4, 8.0 Hz, 1H), 1.15 (t, *J*=4.6 Hz, 1H).

### Compound 16B-8

At 110 °C, 16B-7 (26.7 g, 136.47 mmol) was added to a solution of potassium tert-butoxide (30.6 g, 272.70 mmol) and 1A-2 (24 g, 164.18 mmol) in dioxane (200 mL), and the mixture was stirred at 110 °C for 1 hour. The reaction solution was filtered; the filtrate was concentrated; and then water (100 mL) and ethyl acetate (200 mL) were added. The mixture was subjected to liquid separation; the organic phase was washed with water (100 mL × 2), dried over anhydrous sodium sulfate and then concentrated; and the residue was separated by column chromatography (PE/EA = 50/1 to 20/1, V/V) to obtain 16B-8. ¹HNMR (400 MHz, CDCl₃) δ 7.50 - 7.42 (m, 1H), 6.59 (t, *J*=8.0 Hz, 2H), 4.70 (t, *J*=3.6 Hz, 1H), 4.53 - 4.39 (m, 2H), 4.20 - 4.16 (m, 1H), 4.13 - 4.09 (m, 1H), 4.04 (ddd, *J*=4.0, 5.8, 11.2 Hz, 1H), 3.92 - 3.84 (m, 3H), 3.80 (ddd, *J*=4.0, 6.8, 11.2 Hz, 1H), 3.57 - 3.48 (m, 1H), 2.12 - 2.07 (m, 1H), 1.89 - 1.81 (m, 1H), 1.79 - 1.71 (m, 1H), 1.68 - 1.60 (m, 2H), 1.58 - 1.48 (m, 2H), 1.39 (dd, *J*=4.2, 8.0 Hz, 1H), 1.06 (t, *J*=4.4 Hz, 1H).

### Compound 16B-9

2-1A-1 (36 g, 141.77 mmol), [Ir(COD)OMe]₂ (2 g, 3.02 mmol) and tmphen (1.6 g, 6.77 mmol) were dissolved in methyl tertiary butyl ether (400 mL), and 16B-8 (40 g, 130.99 mmol) was added. Under nitrogen protection, the reaction mixture was heated to 80 °C and stirred for 4 hours. The reaction solution was concentrated to obtain compound 16B-9 as a crude. MS (ESI): m/z 350.4 [M-84+2H]⁺.

### Compound 16B-10

16B-9 (52 g, 120.56 mmol), 14-5A (48.53 g, 135.14 mmol), Pd(dppf)Cl₂·DCM (10.4 g, 12.74 mmol) and sodium carbonate (26 g, 245.31 mmol) were dissolved in dioxane (500 mL) and water (100 mL). Under nitrogen protection, the reaction mixture was heated to 100 °C and stirred for 3 hours. The reaction mixture was filtered; the filtrate was concentrated; ethyl acetate (400 mL) and water (300 mL) were then added; the mixture was subjected to liquid separation; the aqueous phase was extracted with ethyl acetate (100 mL × 3); the combined organic phase was dried over anhydrous sodium sulfate and then concentrated; and the residue was separated by silica gel column chromatography (PE/EA = 7/1 to 3/1) to obtain compound 16B-10. ¹HNMR (400MHz, CDCl₃) δ 8.83 (d, *J*=5.0 Hz, 1H), 8.11 (br s, 1H), 8.05 (s, 1H), 7.86 (br d, *J*=4.6 Hz, 1H), 7.53 (br d, *J*=8.2 Hz, 1H), 7.40 - 7.32 (m, 1H), 7.19 (s, 1H), 6.46 (s, 1H), 6.43 (s, 1H), 4.62 (t, *J*=3.6 Hz, 1H), 4.51 - 4.35 (m, 2H), 4.13 - 4.06 (m, 1H), 4.05 - 3.97 (m, 2H), 3.86 - 3.71 (m, 4H), 3.51 - 3.40 (m, 1H), 2.18 (s, 3H), 2.05 (ddd, *J*=2.6, 5.0, 7.8 Hz, 1H), 1.82 - 1.63 (m, 2H), 1.57 - 1.39 (m, 4H), 1.37 - 1.30 (m, 1H), 1.01 (t, *J*=4.4 Hz, 1H).

### Compound 16B

Hydrochloric acid (128.51 mL, 4 M) was added to 16B-10 (60 g, 102.81 mmol) in DMF (60 mL), and the reaction solution was stirred at 40 °C for 2 hours. Water (2.5 L) was added to the reaction solution, and the resulting mixed solution was filtered; the filter cake was dried and then dissolved in dichloromethane; the mixture was dried over anhydrous sodium sulfate and separated by silica gel column chromatography (PE/EA = 3/1 to 0/1) to obtain compound 16B as a crude, which was dissolved in a mixed solution of PE/EA (4 : 1, 150 mL), stirred and filtered to obtain compound 16B. ¹HNMR (400 MHz, DMSO-d₆) δ 10.70 (s, 1H), 9.06-8.95 (d, *J*=5.0 Hz, 1H), 8.37 (s, 1H), 8.23-8.18 (d, *J*=4.2 Hz, 1H), 7.77-7.71 (dd, *J*=2.2, 8.4 Hz, 1H), 7.68-7.64 (d, *J*=2.2 Hz, 1H), 7.39-7.31 (d, *J*=8.4 Hz, 1H), 6.73 (s, 1H), 6.59-6.54 (d, *J*=1.0 Hz, 1H), 4.86-4.81 (t, *J*=5.2 Hz, 1H), 4.35-4.30 (t, *J*=5.2 Hz, 2H), 4.13 - 4.03 (m, 2H), 3.83 - 3.70 (m, 4H), 2.24 (s, 3H), 2.22-2.08 (ddd, *J*=2.4, 5.0, 7.8 Hz, 1H), 1.40-1.32 (dd, *J*=3.6, 7.8 Hz, 1H), 1.02-0.96 (t, *J*=4.4 Hz, 1H). MS (ESI) m/z: 500.4 [M+H] ⁺, 100% ee (chiral column: DAICEL CHIRALCEL OJ-H (250 mm^{∗}30 mm, 5 µm), mobile phase A: ethanol (containing 0.05% DIEA); mobile phase B: carbon dioxide) retention time: 1.592 min).

### Experimental example 1: Experiments for c-RAF enzyme inhibitory activity

### Experimental materials:

| **Experimental material** | **Brand Catalog No** |
|---|---|
| cRAF protein | Creative BioMart-RAF1-416H |
| MEK1 protein | Invitrogen-PR3 984A |
| ADP-Glo kinase detection kit | Promega-V9102 |
| Tris-HCl, pH 7.4 | Sigma-T2663-1L |
| MgCl₂ | Sigma-63020-1L |
| NaCl | Sigma-S5150 |
| DTT | Invitrogen- P23 25 |
| Triton X-100 | Sigma-X100 |
| H₂O | Gibco-15230-162 |
| 384 intermediate plate | Greiner-781280 |
| 384 experimental plate | PerkinElmer -6007299 |

### Experimental steps:

**(1) compound preparation:**
   the compounds to be tested and reference compounds were diluted to 100 µM with DMSO, and the compounds were further subjected to a 3-fold gradient dilution using Echo to obtain target plates with 11 concentration gradients.
**(2) experimental process:**
   1) buffer formulation: 50 mM Tris-HCl (pH 7.4), 3.5 mM MgCl₂, 150 mM NaCl, 1 mM DTT, 0.02% Triton X-100, H₂O;
   2) a mixed solution of MEK1 and ATP was formulated with a buffer, and 5 µL of the substrate mixed solution was added to the 384 intermediate plate;
   3) the cRAF enzyme was diluted with a buffer, and 5 µL was added to the 384 intermediate plate;
   4) 5 µL of the mixed reaction solution was transferred to the 384 experimental plate using Bravo, and the plate was centrifuged for 15 seconds and then incubated in a 23 °C incubator;
   5) after 1 hour, 5 µL of ADP-Glo was added to the 384 experimental plate, and the plate was shaken, centrifuged for 15 seconds and then incubated in a 23 °C incubator;
   6) after 40 minutes, 10 µL of the kinase detection reagent was added to the 384 experimental plate, and the plate was shaken, centrifuged for 15 seconds and then incubated in a 23 °C incubator;
   7) after 1 hour, the plate was read on Envision.

### Experimental results:

| **Compound** | **c-RAF IC₅₀ (µM)** | **Compound** | **c-RAF IC₅₀ (µM)** |
|---|---|---|---|
| Compound 1A | 0.0025 | Compound 1B | 0.0020 |
| Compound 2 | 0.0027 | Compound 3 | 0.0017 |
| Compound 5 | 0.0010 | Compound 6 | 0.0009 |
| Compound 7 | 0.0025 | Compound 8 | 0.0011 |
| Compound 9 | 0.0012 | Compound 10 | 0.0025 |
| Compound 11 | 0.0028 | Compound 16 | 0.0008 |
| Compound 16B | 0.0006 | / | / |

**Experimental conclusion:** the compounds of the present disclosure have a better c-RAF enzyme inhibitory activity.

### Experimental example 2: Experiments for Calu-6 (Kras^{Q61K}) antiproliferative activity

### Experimental materials:

1) Experimental reagents and consumables

| **Name** | **Brand Catalog No** |
|---|---|
| EMEM medium | Vicente-320-005-CL |
| Fetal bovine serum | Biosera-FB-1058/500 |
| 0.25% trypsin | Basalmedia-S310KJ |
| Double antibodies (penicillin and streptomycin) | Procell- PB180120 |
| CellTiter Glo | Promega-G7573 |
| Cell plate | Corning-3610 |

2) Experimental instruments

| **Name** | **Brand Catalog No** |
|---|---|
| Cell counting plate | Qukin |
| Victor Nivo | PerkinElmer |

### Experimental steps:

### Cell inoculation:

(1) cell medium: 89% EMEM, 10% fetal bovine serum and 1% penicillin-streptomycin;
(2) the original medium in the cell culture flask was removed; the cells were digested with trypsin and then counted; and the cell suspension was diluted with the medium to a cell density of 3.75 × 10⁴ cells per milliliter required for plating;
(3) 100 µL of the medium was added to the outermost circle of wells of the cell plate; 80 µL of the cell suspension was added to other wells; and the plate was cultured overnight in a 37 °C incubator with 5% CO₂.

### Compound supplementation:

the compounds were subjected to a gradient dilution and then supplemented using Echo; and the cell plate was put back into the incubator for three days.

### Plate read and data analysis:

adding CTG and reading the plate involve adding 20 µL of CellTiterGlo to each well of the cell plate, shaking the plate for 10 min in the dark, and reading the plate on Victor Nivo.

### Experimental results:

| **Compound** | **Calu-6 antiproliferative activity IC₅₀ (µM)** | **Compound** | **Calu-6 antiproliferative activity IC₅₀ (µM)** |
|---|---|---|---|
| Compound 1A | 1.2 | Compound 1B | 0.96 |
| Compound 2 | 1.1 | Compound 3 | 1.4 |
| Compound 5 | 4.6 | Compound 6 | 1.0 |
| Compound 6A | 1.2 | Compound 6B | 1.3 |
| Compound 8 | 1.0 | Compound 9 | 6.4 |
| Compound 10 | 7.1 | Compound 11 | 1.1 |
| Compound 11A | 3.1 | Compound 11B | 1.1 |
| Compound 13 | 0.2 | Compound 14 | 3.5 |
| Compound 14A | 3.7 | Compound 14B | 4.6 |
| Compound 15 | 4.7 | Compound 16 | 1.3 |
| Compound 16A | 1.3 | Compound 16B | 0.6 |

**Experimental conclusion:** the compounds of the present disclosure have a Calu-6 antiproliferative activity.

### Experimental example 3: Experiments for HCT-116(Kras^{G13D}) antiproliferative activity

### Experimental materials:

1) Experimental reagents and consumables

| **Name** | **Brand Catalog No** |
|---|---|
| Mc'Coy 5A medium | BI- 01-075-1ACS |
| Fetal bovine serum | Biosera-FB-1058/500 |
| 0.25% trypsin | Basalmedia-S310KJ |
| Double antibodies (penicillin and streptomycin) | Procell- PB180120 |
| CellTiter Glo | Promega-G7573 |
| Cell plate | Corning-3610 |

2) Experimental instruments

| **Name** | **Brand Catalog No** |
|---|---|
| Cell counting plate | Qukin |
| Victor Nivo | PerkinElmer |

### Experimental steps:

### Cell inoculation:

(1) cell medium: 89% Mc'Coy 5A, 10% fetal bovine serum and 1% penicillin-streptomycin;
(2) the original medium in the cell culture flask was removed; the cells were digested with trypsin and then counted; and the cell suspension was diluted with the medium to a cell density of 2.5 × 10⁴ cells per milliliter required for plating;
(3) 100 µL of the medium was added to the outermost circle of wells of the cell plate; 80 µL of the cell suspension was added to other wells; and the plate was cultured overnight in a 37 °C incubator with 5% CO₂.

### Compound supplementation:

the compounds were subjected to a gradient dilution and then supplemented; and the cell plate was put back into the incubator for three days.

### Plate read and data analysis:

adding CTG and reading the plate involve adding 20 µL of CellTiterGlo to each well of the cell plate, shaking the plate for 10 min in the dark, and reading the plate on Victor Nivo.

### Experimental results:

| **Compound** | **HCT-116 antiproliferative activity IC₅₀ (µM)** | **Compound** | **HCT-116 antiproliferative activity IC₅₀ (µM)** |
|---|---|---|---|
| Compound 1A | 0.6 | Compound 1B | 0.6 |
| Compound 2 | 2.3 | Compound 3 | 2.3 |
| Compound 5 | 2.6 | Compound 6 | 0.6 |
| Compound 6A | 1.4 | Compound 6B | 1.5 |
| Compound 8 | 1.1 | Compound 9 | 0.8 |
| Compound 10 | 5.3 | Compound 11 | 0.8 |
| Compound 11A | 3.6 | Compound 11B | 1.4 |
| Compound 13 | 0.2 | Compound 14 | 0.4 |
| Compound 14A | 2.1 | Compound 14B | 2.3 |
| Compound 15 | 0.9 | Compound 16 | 2.2 |
| Compound 16A | 2.3 | Compound 16B | 1.1 |

**Experimental conclusion:** the compounds of the present disclosure have an HCT-116(Kras^{G13D}) antiproliferative activity.

### Experimental example 4: Experiments for HCT116(Kras^{G13D}) ERK phosphorylation inhibition

### Experimental materials:

1. Reagents and consumables

| Reagent | Brand Catalog No |
|---|---|
| Highly sensitive detection kit for human ERK phosphorylated protein | Cisbio-64AERPEH |
| RPMI1640 medium | Gibco-22400089 |
| Fetal bovine serum | Hyclone-SV30087.03 |
| 96 HTRF microwell plate | Cisbio-66PL96025 |
| 96 microwell plate | COSTAR-3599 |
| DMSO | Sigma-D2650-100 mL |
| 0.05% Trypsin-EDTA | Gibco-25300-062 |

2. Main instruments

| Instrument | Manufacturer | Model number |
|---|---|---|
| Biosafety cabinet | AIRTECH | BSC-1304IIA2 |
| Carbon dioxide incubator | Thermo | 311 |
| Cell counter | BECKMAN | Vi-cellXR |
| Microplate reader | PerkinElmer | Envision |
| Centrifuge | Eppendorf | Centrifuge 5810R |

3. Cell information

**Table 1 Cell information**

| **Cell name** | **Source** | **Catalog No.** |
|---|---|---|
| HCT116 | ATCC | ATCC- HTB-132 |

### Experimental steps and methods:

1) cells were resuscitated, cultured to a logarithmic growth phase, then digested with trypsin and seeded in a 96-well plate, and the plate was incubated overnight in an incubator;
2) serial gradients of compounds dissolved in DMSO were added to the 96-well plate, which was then put back into the incubator for 1 hour;
3) the cell plate was taken out; the supernatant was removed; and the cells were incubated and lysed by adding cell lysates (containing 1% blocking peptide) for 30 minutes at room temperature;
4) 16 µL of cell lysates per well was transferred to the HTRF plate, and then 4 µL of the mixed antibody solution formulated thereby was added;
5) after overnight incubation, the plate was read on Envision; the fitted curves were obtained according to ratio (the ratio of Ex665/Ex615 fluorescence intensity); and EC₅₀ was calculated with the four-parameter fitting formula, i.e., Y = Bottom + (Top-Bottom)/(1+10^((LogEC50-X)^{∗}HillSlope)) on Graphpad.

### Experimental results:

| **Compound** | **HCT-116 ERK phosphorylation inhibitory activity IC₅₀ (µM)** | **Compound** | **HCT-116 ERK phosphorylation inhibitory activity IC₅₀ (µM)** |
|---|---|---|---|
| Compound 1 | 0.12 | Compound 1A | 0.14 |
| Compound 1B | 0.22 | Compound 2 | 0.21 |
| Compound 3 | 0.19 | Compound 6A | 0.16 |
| Compound 6B | 0.19 | Compound 11A | 0.38 |
| Compound 11B | 0.31 | Compound 16 | 0.16 |
| Compound 16A | 0.23 | Compound 16B | 0.18 |

**Experimental conclusion:** the present disclosure compounds have an HCT-116 ERK phosphorylation inhibitory activity.

### Experimental example 5: Experiments for Calu-6(Kras^{Q61K}) ERK phosphorylation inhibition

### Experimental materials:

Reagents and consumables

| Reagent | Brand Catalog No |
|---|---|
| Highly sensitive detection kit for human ERK phosphorylated protein | Cisbio-64AERPEH |
| RPMI1640 medium | Gibco-22400089 |
| Fetal bovine serum | Hyclone-SV30087.03 |
| 96 HTRF microwell plate | Cisbio-66PL96025 |
| 96 microwell plate | COSTAR-3599 |
| DMSO | Sigma-D2650-100 mL |
| 0.05% Trypsin-EDTA | Gibco-25300-062 |

Main instruments

| Instrument | Manufacturer | Model number |
|---|---|---|
| Biosafety cabinet | AIRTECH | BSC-1304IIA2 |
| Carbon dioxide incubator | Thermo | 311 |
| Cell counter | BECKMAN | Vi-cellXR |
| Microplate reader | PerkinElmer | Envision |
| Centrifuge | Eppendorf | Centrifuge 5810R |

Cell information:

| **Cell name** | **Source** | **Catalog No.** |
|---|---|---|
| Calu6 | ATCC | ATCC-HTB-56 |

### Experimental steps and methods:

1) cells were resuscitated, cultured to a logarithmic growth phase, then digested with trypsin and seeded in a 96-well plate, and the plate was incubated overnight in an incubator;
2) serial gradients of compounds dissolved in DMSO were added to the 96-well plate, which was then put back into the incubator for 1 hour;
3) the cell plate was taken out; and the cells were incubated and lysed by adding cell lysates (containing 1% blocking peptide) for 30 minutes at room temperature;
4) 16 µL of cell lysates per well was transferred to the HTRF plate, and then 4 µL of the mixed antibody solution formulated thereby was added;
5) after overnight incubation, the plate was read on Envision; the fitted curves were obtained according to ratio (the ratio of Ex665/Ex615 fluorescence intensity); and EC₅₀ was calculated with the four-parameter fitting formula, i.e., Y = Bottom + (Top-Bottom)/(1+10^((LogEC50-X)^{∗}HillSlope)) on Graphpad.

### Experimental results:

| **Compound** | **Calu-6 ERK phosphorylation inhibitory activity IC₅₀ (µM)** | **Compound** | **Calu-6 ERK phosphorylation inhibitory activity IC₅₀ (µM)** |
|---|---|---|---|
| Compound 1A | 0.22 | Compound 1B | 0.25 |
| Compound 5 | 0.17 | Compound 6 | 0.22 |
| Compound 8 | 0.35 | Compound 9 | 0.18 |
| Compound 11 | 0.43 | Compound 13 | 0.39 |
| Compound 14A | 0.35 | Compound 14B | 0.25 |
| Compound 14 | 0.33 | Compound 15 | 0.33 |
| Compound 16A | 0.63 | Compound 16B | 0.49 |

**Experimental conclusion:** the present disclosure compounds have a Calu-6 ERK phosphorylation inhibitory activity.

### Experimental example 6: Experiments for in vivo pharmacodynamics of human lung cancer Calu-6 subcutaneous xenograft tumor BALB/c nude mouse model

### Experimental materials:

### 1.1 Experimental animals and feeding environment

### 1.1.1 Experimental animals

Specie: Mice
Strain BALB/c nude mice
Week old upon arrival: 6-8 weeks old
Gender: female

### 1.1.2 feeding environment

Mice are fed in SPF-level animal rooms in IVC (individual ventilated cages; constant temperature and humidity) (3-5 per cage)
Temperature: 20 °C-26 °C
Humidity: 40%-70%

### 1.2 Compound information

| Name | Molecular weight | Purity (%) | Content (mg) | Storage condition |
|---|---|---|---|---|
| Compound 1 | 518.49 | 99.21 | 380.0 | RT |
| Compound 16B | 499.48 | 99.44 | 655.0 | RT |

### 1.3 Tumor tissue or cell information

Cells: human lung cancer Calu-6 cells, which were cultured in vitro in EMEM medium containing 0.2 units/mL bovine insulin and 10% fetal bovine serum in a 37 °C incubator with 5% CO₂. Conventional digestion treatment with trypsin-EDTA for passage was carried out twice a week. When the cell saturation was 80% to 90%, and the number reached the requirement, the cells were collected, counted and inoculated.

### 1.4 Additional reagent information

| Name | Manufacturer | Catalog No. | Storage condition |
|---|---|---|---|
| Fetal bovine serum | Hyclone | SV30087.03 | -20 °C |
| Trypsin | Gibco | 25200-072 | -20 °C |
| EMEM medium | ATCC | ATCC30-2003 | 2-8 °C |

### 1.5 Instrument information

| Name | Manufacturer | Model number |
|---|---|---|
| Carbon dioxide incubator | Thermo Fisher | Heracell240i |
| Cryogenic high-speed centrifuge | Eppendorf | 5810R |
| Analytical balance | Sartorius | SECURA225D-1CN |
| General balance | Changzhou Tianzhiping Instrument Equipment Co., Ltd. | EL-2KJ |
| Digital vernier caliper | Mitutoyo | 0-150 mm |

### Experimental methods and steps:

### 2.1 Tumor cell inoculation

Cell inoculation: 0.2 mL of Calu-6 cells (at 1 1 ratio with Matrigel) were subcutaneously inoculated on the right back of each mouse, and when the average tumor volume reached 173 mm³, mice were grouped and administrated.

### 2.2 Grouping

**Table 2 Grouping and administrating schedule for experimental animals**

| Groups | N¹ | Compound treatment | Dose (mg/kg) | Administration volume parameters (µL/g)² | Route of administration | Frequency of administration |
|---|---|---|---|---|---|---|
| 1 | 6 | Solvent control group³ | - | 10 | PO | QD × 21 days |
| 2 | 6 | Compound 1 | 100 | 10 | PO | QD × 21 days |
| 3 | 6 | Compound 16B | 100 | 10 | PO | QD × 21 days |

| | | | | | | |
|---|---|---|---|---|---|---|
| [0300] Note: 1: Number of mice per group 2: Administration volume parameters based on mouse body weight (10 µL/g). If the body weight decreases by more than 15%, the administration is stopped until the body weight returns to within 10% of the original body weight; 3: 0.5% MC (methyl cellulose). | | | | | | |

### 2.3 Formulation of test substances

**Table 3 Formulation method of test substances**

| Compound | Package | Formulation method¹ | Concentration (mg/mL) | Storage condition |
|---|---|---|---|---|
| Solvent control group | - | 80% (5% solutol aqueous solution) + 20% PEG 400 | -- | 4°C |
| Compound 1 | 255.0 mg/bottle | 18.06 mg of compound 1 was weighed, and 1.44 ml of 5% solutol was added; vortex or sonication is performed until same is fully dissolved; and then 0.36 ml of 20% PEG-400 was added and fully dissolve to obtain a 10 mg/mL solution. | 10 | 4°C |
| Compound 16B | 580 mg/bottle | 18.10 mg of compound 16B was weighed, and 1.44 ml of 5% solutol was added; vortex or sonication is performed until same is fully dissolved; and then 0.36 ml of 20% PEG-400 was added and fully dissolve to obtain a 10 mg/mL solution. | 10 | 4°C |

| | | | | |
|---|---|---|---|---|
| [0302] Note: 1. before administration to mice, the compounds to be administrated needs to be mixed gently and thoroughly, wherein solutol is polyethylene glycol-15-hydroxystearate. | | | | |

### 2.4 Tumor measurement and experimental index

Tumor diameter was measured twice a week with a vernier caliper. The calculation formula of tumor volume was V = 0.5*a* × *b*², wherein *a* and *b* represent the long and short diameters of the tumor, respectively.

The anti-tumor efficacy of the compound was evaluated by TGI (%) or relative tumor proliferation rate T/C (%). Relative tumor proliferation rate T/C (%) = T_{RTV}/C_{RTV} × 100% (T_{RTV}: mean RTV of the treatment group; C_{RTV}: mean RTV of the negative control group). The relative tumor volume (RTV) was calculated according to the results of the tumor measurement. The calculation formula was RTV = Vt/Vo, wherein Vo was the tumor volume measured at the beginning of the grouping and administration (i.e., D0), and Vₜ was the tumor volume in a certain measurement. T_{RTV} and C_{RTV} were obtained from the data on the same day.

TGI (%) reflected the tumor growth inhibition rate. TGI (%) = [1-(average tumor volume at the end of administration in a certain treatment group - average tumor volume at the beginning of administration in the treatment group)/(average tumor volume at the end of administration in the solvent control group - average tumor volume at the beginning of administration in the solvent control group)] × 100%.

### 2.5 Statistical analysis

statistical analysis was performed using SPSS software on the basis of RTV data at the end of the experiment. The comparison between groups was analyzed by one-way ANOVA. If there was heterogeneity of variance (F value was significantly different), the Games-Howell test was applied. *p* < 0.05 was considered significantly different.

### 3. Experimental results

### 3.1 Inhibitory effect of test substances on the growth of subcutaneously transplanted tumors in human lung cancer-bearing nude mice

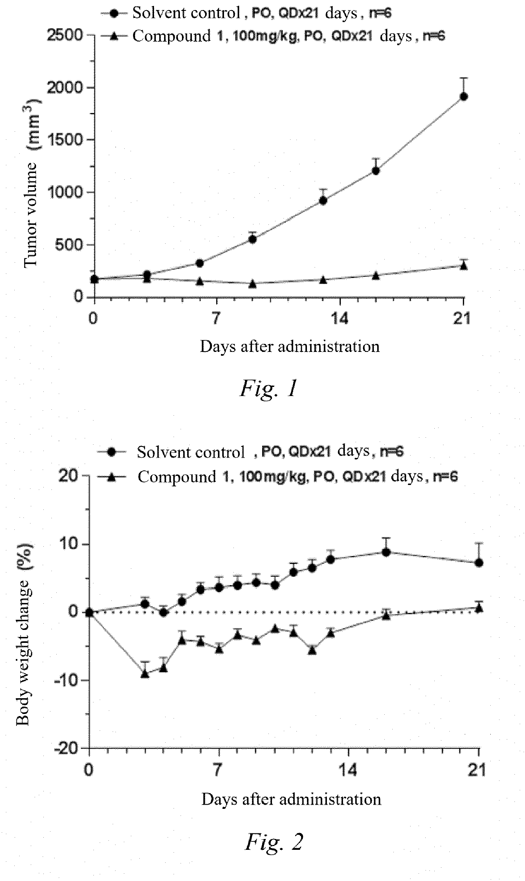

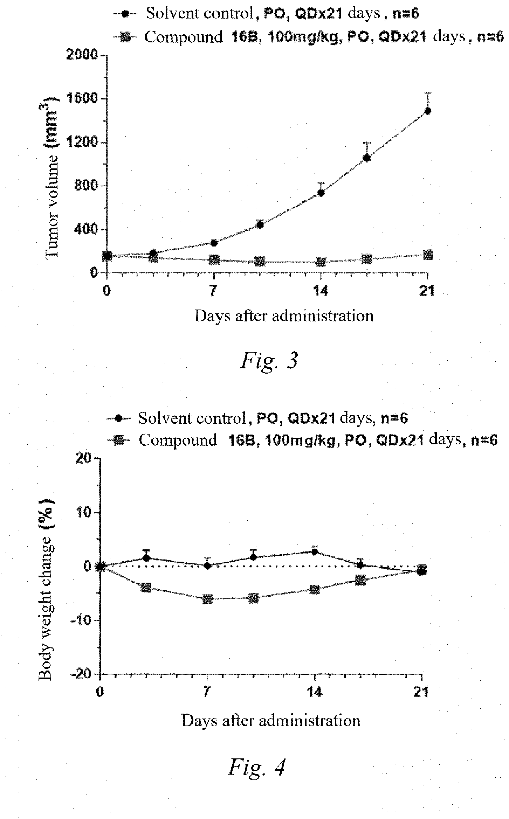

In this experiment, the efficacy of the test substances in human lung cancer xenograft tumor models was evaluated, and the solvent control group was used as a reference. The tumor volumes for each group at different time points were as shown in Figs. 1 and 3. The group with administration of compound 1 (100 mg/kg) had a T/C of 18.5% and a TGI of 100.9%, indicating a significant tumor inhibitory effect (P < 0.01). The tumor growth curves of tumor-bearing mice as human lung cancer Calu-6 subcutaneous xenograft tumor models after administration of compounds were shown in Fig. 1. The group with administration of compound 16B (100 mg/kg) had a T/C of 11.4%, and a TGI of 99.1%, indicating a significant tumor inhibitory effect (P < 0.01). The tumor growth curves of tumor-bearing mice as human lung cancer Calu-6 subcutaneous xenograft tumor models after administration of the compounds were shown in Fig. 3.

### 3.2 Body weight changes

No abnormality was observed in the weight and state of mice. The effects of test substances on the body weight of mice are shown in Figs 2 and 4.

### Experimental conclusion:

The compounds of the present disclosure have a significant inhibitory effect on the tumor growth of tumor-bearing mice as human lung cancer Calu-6 subcutaneous xenograft tumor models.

### Experimental example 7: Experiments for in vivo pharmacokinetics in mice

### Experimental objective

In vivo pharmacokinetic parameters of the compounds of the present disclosure in mice were detected.

### Experimental scheme

1) Experimental drug: Compound 16B;
2) Experimental animals: 4 female CD-1 mice, which were divided into 2 groups, 2 mice in each group;
3) Drug formulation: An appropriate amount of the drug was weighed and dissolved in an aqueous solution of solutol (5% by volume), DMSO (5% by volume) and PEG-300 (25% by volume) for injection administration. An appropriate amount of the drug was weighed and dispersed in a 5% solutol aqueous solution (80% by volume), and then PEG-400 (20% by volume) was added; the mixture was mixed well and used for intragastric administration. Solutol is polyethylene glycol-15-hydroxystearate.

### Experimental operations

The animals in the first group were administered the compound at a dose of 2 mg/kg at a concentration of 1 mg/mL by intravenous administration. Plasma samples were collected from the animals at 0.117, 0.333, 1, 2, 4, 7 and 24 hours after administration. The animals in the second group were administered the compound at a dose of 100 mg/kg at a concentration of 10 mg/mL by intragastric administration. Plasma samples were collected from the animals at 0.0833, 0.25, 0.5, 1, 2, 4, 6, 8 and 24 hours after administration. The drug concentration at each point was determined by the LC-MS/MS method, and kinetic parameters of the tested drug were as follows:

| | | | | | | |
|---|---|---|---|---|---|---|
| Compo und 16B | Intraven ous administ ration group | Clearance rate | Initial concentratio n | Distributi on volume | Half-life | Curve area |
| | | Cl (mL/min/kg) | Co (nM) | Vd (L/kg) | T_{1/2} (h) | AUC (nM·h) |
| | | 25.8 | 2011 | 2.75 | 1.43 | 2614 |
| | Intragast ric administ ration group | Maximum concentratio n | Maximum concentratio n time | Curve area | Bioavailabi lity | -- |
| | | Cₘₐₓ (nM) | Tₘₐₓ (h) | AUC (nM·h) | F (%) | -- |
| | | 25000 | 1 | 177513 | 131 | -- |

| | | | | | | |
|---|---|---|---|---|---|---|
| Note: -- means absence | | | | | | |

### Experimental conclusion

The compounds of the present disclosure have good in vivo pharmacokinetic properties in mice.

## Claims

1. A compound as shown in formula (III) or a pharmaceutically acceptable salt thereof, 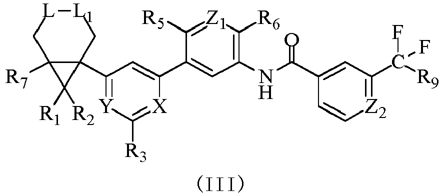 wherein,
X and Y are each independently selected from CH and N;
L is selected from -O-, -S-, -S(=O)- and -S(=O)₂-;
L₁ is selected from -CH₂- and a single bond;
Z₁ and Z₂ are each independently selected from CH and N;
R₁ and R₂ are each independently selected from H, F and C₁₋₃ alkyl, wherein the C₁₋₃ alkyl is optionally substituted with 1, 2 or 3 Rₐ;
R₃ is selected from 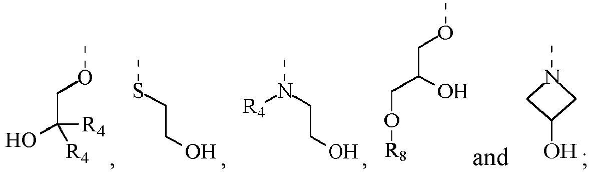
R₄ is selected from H and C₁₋₃ alkyl, wherein the C₁₋₃ alkyl is optionally substituted with 1, 2 or 3 R_{b};
R₅ is selected from H and C₁₋₃ alkyl, wherein the C₁₋₃ alkyl is optionally substituted with 1, 2 or 3 R_{c};
R₆ is selected from H and F;
R₇ is selected from H and CN;
R₈ is selected from H and CH₃;
R₉ is selected from H, F and CH₃;
each Rₐ is independently selected from F, Cl, Br and I;
each R_{b} is independently selected from F, Cl, Br, I and CH₃;
each R_{c} is independently selected from F, Cl, Br and I.

2. The compound or the pharmaceutically acceptable salt thereof according to claim 1, wherein, the compound is selected from formula (I'), 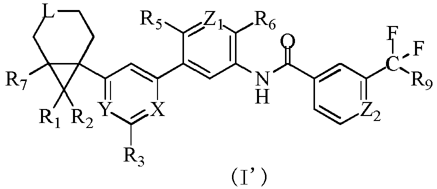 wherein,
X and Y are each independently selected from CH and N;
L is selected from -O-, -S-, -S(=O)- and -S(=O)₂-;
Z₁ and Z₂ are each independently selected from CH and N;
R₁ and R₂ are each independently selected from H, F and C₁₋₃ alkyl, wherein the C₁₋₃ alkyl is optionally substituted with 1, 2 or 3 Rₐ;
R₃ is selected from 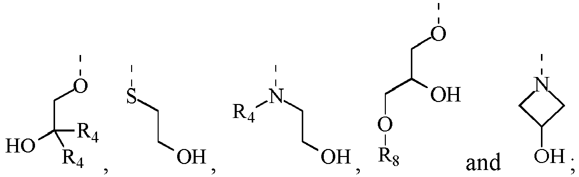
R₄ is selected from H and C₁₋₃ alkyl, wherein the C₁₋₃ alkyl is optionally substituted with 1, 2 or 3 R_{b};
R₅ is selected from H and C₁₋₃ alkyl, wherein the C₁₋₃ alkyl is optionally substituted with 1, 2 or 3 R_{c};
R₆ is selected from H and F;
R₇ is selected from H and CN;
R₈ is selected from H and CH₃;
R₉ is selected from H, F and CH₃;
each Rₐ is independently selected from F, Cl, Br and I;
each R_{b} is independently selected from F, Cl, Br, I and CH₃;
each R_{c} is independently selected from F, Cl, Br and I.

3. The compound or the pharmaceutically acceptable salt thereof according to claim 1 or 2, wherein, R₁ and R₂ are each independently selected from H and F.

4. The compound or the pharmaceutically acceptable salt thereof according to claim 1 or 2, wherein, R₃ is selected from

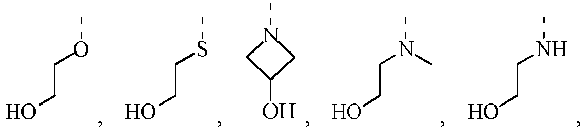

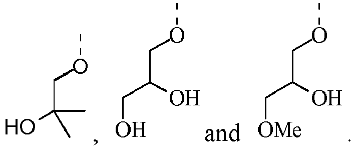

5. The compound or the pharmaceutically acceptable salt thereof according to claim 1 or 2, wherein, R₄ is selected from H, CH₃ and CH₂CH₃.

6. The compound or the pharmaceutically acceptable salt thereof according to claim 1 or 2, wherein, R₅ is selected from CH₃.

7. The compound or the pharmaceutically acceptable salt thereof according to claim 1, wherein, the moiety 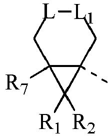 is selected from 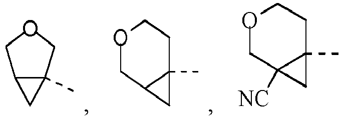 and

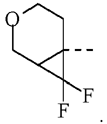

8. The compound or the pharmaceutically acceptable salt thereof according to claim 1, wherein, the moiety 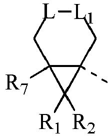 is selected from

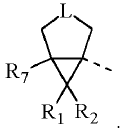

9. The compound or the pharmaceutically acceptable salt thereof according to claim 8, wherein, the moiety 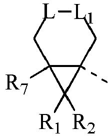 is selected from

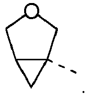

10. The compound or the pharmaceutically acceptable salt thereof according to claim 1 or 2, wherein, the moiety 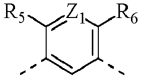 is selected from

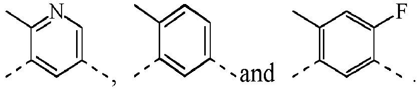

11. The compound or the pharmaceutically acceptable salt thereof according to claim 1 or 2, wherein, the moiety 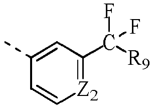 is selected from 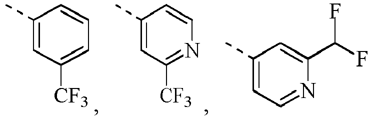 and

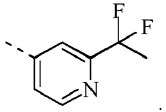

12. The compound or the pharmaceutically acceptable salt thereof according to claim 1 or 2, wherein, moiety 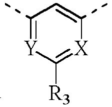 is selected from

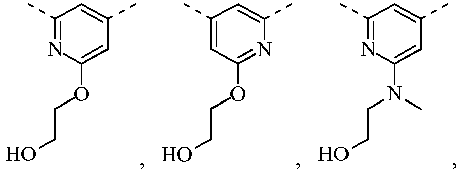

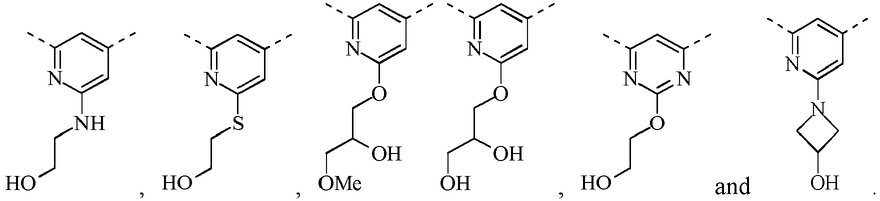

13. The compound or the pharmaceutically acceptable salt thereof according to any one of claims 1 and 3-6, wherein, the compound is selected from 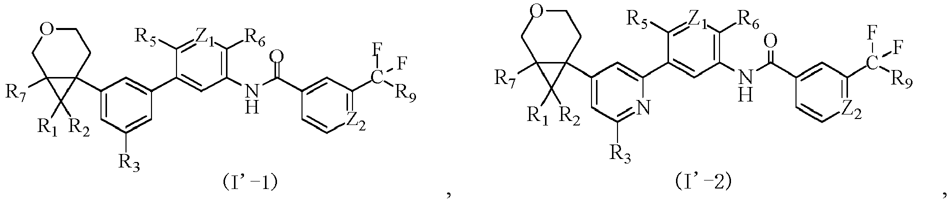 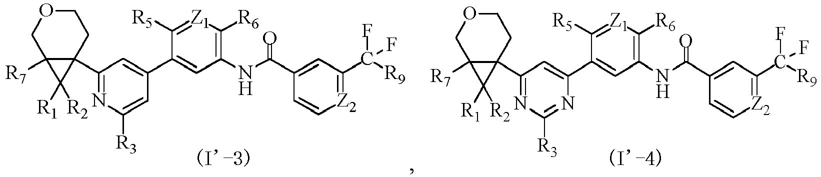 and 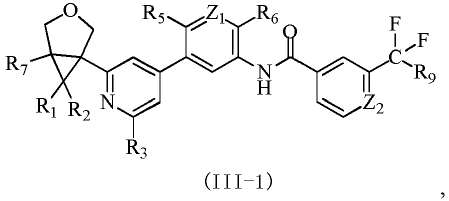 wherein, R₁, R₂, R₃, R₅, R₆, R₇, R₉, Z₁ and Z₂ are as defined in any one of claims 1-5.

14. The compound or the pharmaceutically acceptable salt thereof according to claim 13, wherein, the compound is selected from 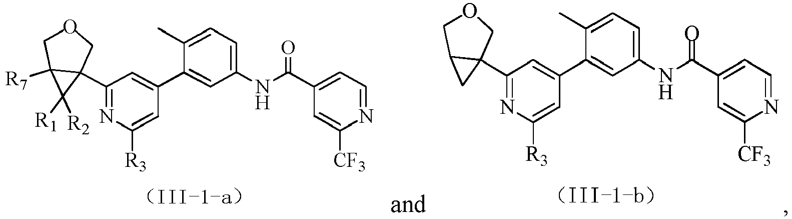 wherein, R₁, R₂, R₃ and R₇ are as defined in claim 13.

15. A compound or a pharmaceutically acceptable salt thereof, wherein, the compound is selected from

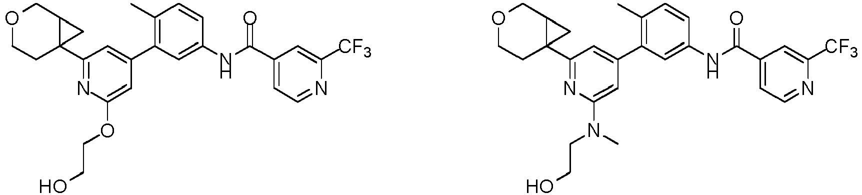

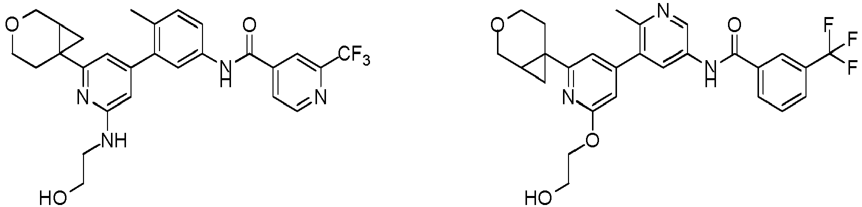

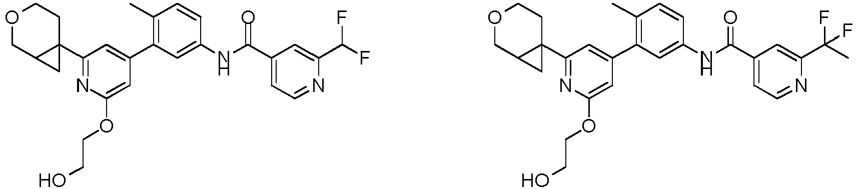

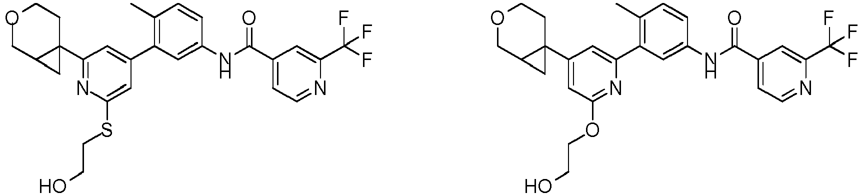

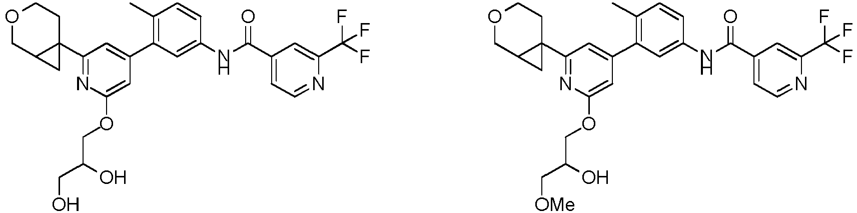

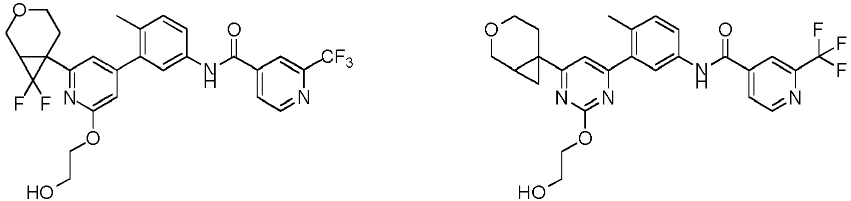

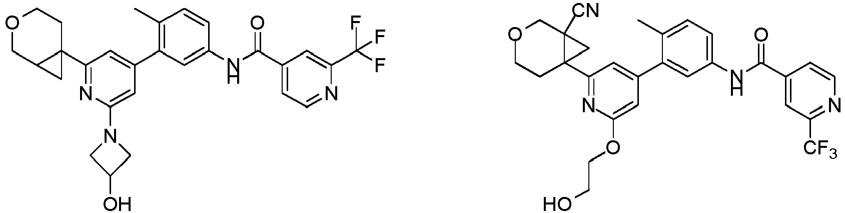

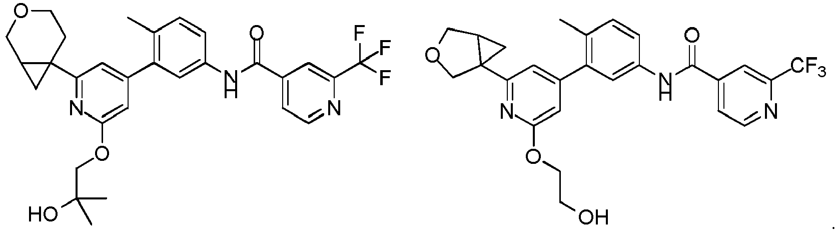

16. The compound or the pharmaceutically acceptable salt thereof according to claim 15, wherein, the compound is selected from

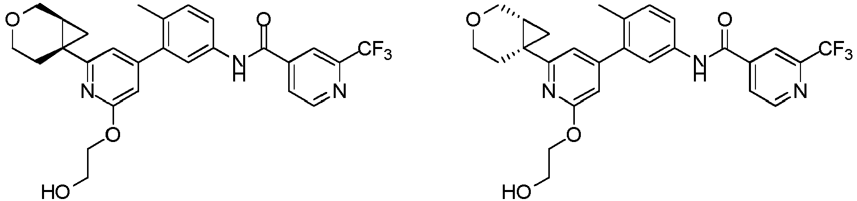

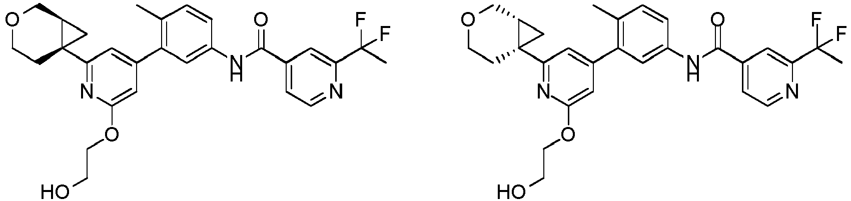

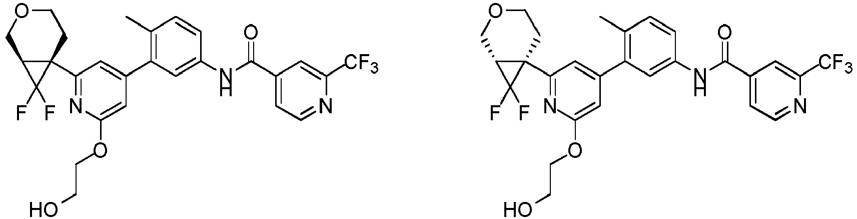

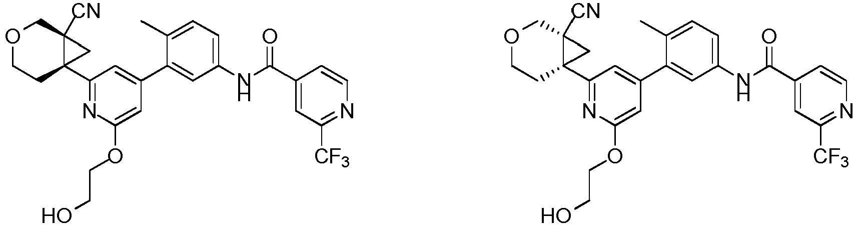

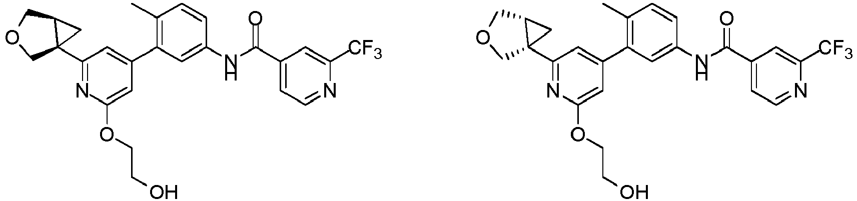

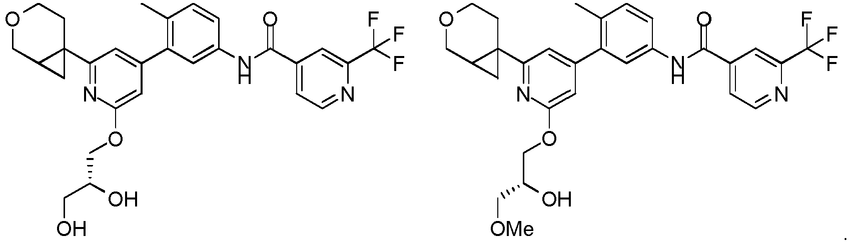

17. A compound as shown in formula (IV-1), (IV-2), (IV-3) or (IV-4), or a pharmaceutically acceptable salt thereof, 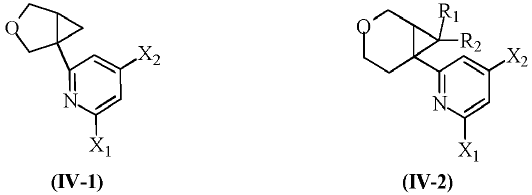 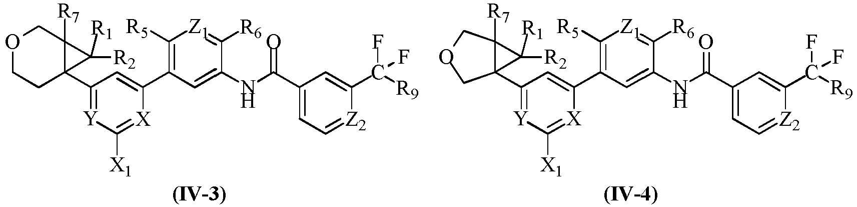 wherein,
X₁ is selected from halogen, -SO₂Me, -OMs, OTf, OTs, 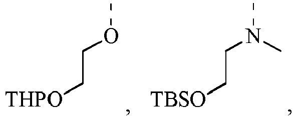 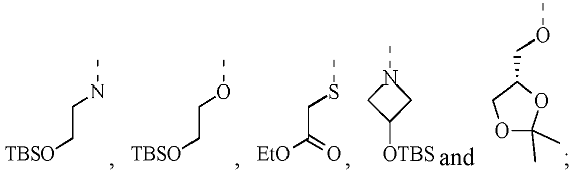
X₂ is selected from halogen, OH, -SO₂Me, -OMs, OTf, OTs and H;
R₁, R₂, R₅, R₆, R₇, R₉, X, Y, Z₁ and Z₂ are as defined in claim 1.

18. A compound as shown in formula (V) or a pharmaceutically acceptable salt thereof, 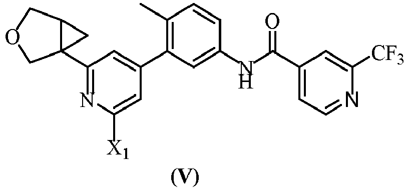 wherein,
X₁ is selected from halogen, -SO₂Me, -OMs, OTf, OTs,

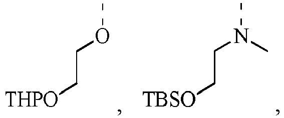

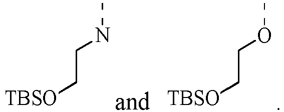

19. Use of the compound or the pharmaceutically acceptable salt thereof according to any one of claims 1-18 in the preparation of an RAF kinase inhibitor.

20. Use of the compound or the pharmaceutically acceptable salt thereof according to any one of claims 1-18 in the preparation of a medicament for treating cancers.
